# EUROPÄISCHE PATENTSCHRIFT

(11) **EP 1 628 952 B1**
(45) Veröffentlichungstag und Bekanntmachung des Hinweises auf die Patenterteilung: **03.09.2014**
(21) Anmeldenummer: 04739427.5
(22) Anmeldetag: 28.05.2004
(51) Int. Cl.: C07C 275/70, C07C 323/25, C07F 9/28, C07F 9/30

(54) **IONISCHE FLÜSSIGKEITEN MIT URONIUM- ODER THIOURONIUM-KATIONEN**
IONIC LIQUID COMPRISING URONIUM CATIONS OR THIOURONIUM CATIONS
LIQUIDES IONIQUES CONTENANT DES CATIONS D'URONIUM OU DE THIOURONIUM

(30) Priorität: 02.06.2003 DE 10324891; 02.06.2003 DE 10325050; 17.11.2003 DE 10353758
(43) Veröffentlichungstag der Anmeldung: 01.03.2006
(73) Patentinhaber: Merck Patent GmbH, 64293 Darmstadt (DE)
(72) Erfinder: IGNATYEV, Nikolai (Mykola), 47058 Duisburg (DE); WELZ-BIERMANN, Urs, 64646 Heppenheim (DE); BISSKY, German, 42109 Wuppertal (DE); WILLNER, Helge, 45481 Mühlheim/Ruhr (DE); KUCHERYNA, Andriy, 42117 Wuppertal (DE)
(86) Internationale Anmeldenummer: PCT/EP2004/005772
(87) Internationale Veröffentlichungsnummer: WO 2004/106287

(56) Entgegenhaltungen:
- DE-A- 19 648 125
- DATABASE CA [Online] CHEMICAL ABSTRACTS SERVICE, COLUMBUS, OHIO, US; ECHNER, HARTMUT ET AL ECHNER, HARTMUT ET AL: "Rapid thioester couplings mediated by new uronium salts Rapid thioester couplings mediated by new uronium salts" XP002302549 gefunden im STN Database accession no. 1999:396527 & PEPTIDES: FRONTIERS OF PEPTIDE SCIENCE, PROCEEDINGS OF THE AMERICAN PEPTIDE SYMPOSIUM, 15TH, NASHVILLE, JUNE 14-19, 1997 , MEETING DATE 1997, 283-284. EDITOR(S): TAM, JAMES P.; KAUMAYA, PRAVIN T. P. PUBLISHER: KLUWER, DORDRECHT, NETH. CODEN: 67UCAR, 1999,
- DATABASE CA [Online] CHEMICAL ABSTRACTS SERVICE, COLUMBUS, OHIO, US; FEITH, BERNHARD ET AL: "Dication ethers. 11. Ambidoselective reactions of .beta.-keto enolates with dication ethers" XP002302550 gefunden im STN Database accession no. 1987:83983 & LIEBIGS ANNALEN DER CHEMIE , (12), 2123-41 CODEN: LACHDL; ISSN: 0170-2041, 1986,
- DATABASE CA [Online] CHEMICAL ABSTRACTS SERVICE, COLUMBUS, OHIO, US; FEITH, BERNHARD ET AL: "Ring-opening of N-(tetraalkylamidinio)pyridinium salts by anions of CH-acidic methylene compounds" XP002302551 gefunden im STN Database accession no. 1987:32793 & CHEMISCHE BERICHTE , 119(11), 3276-96 CODEN: CHBEAM; ISSN: 0009-2940, 1986,
- KANTLEHNER W ET AL: "HERSTELLUNG VON 1,1,2,3,3-PENTASUBSTITUIERTEN UND 1,1,2,2,3,3-HEXASUBSTITUIERTEN GUANIDINIUMSALZEN SOWIE VON 1,1,2,3,3-PENTAALKYLGUANIDINEN PREPARATION OF 1,1,2,3,3-PENTASUBSTITUTED AND 1,1,2,2,3,3-HEXASUBSTITUTED GUANIDINIUM SALTS AND 1,1,2,3,3-PENTAAL" LIEBIGS ANNALEN DER CHEMIE, VERLAG CHEMIE GMBH. WEINHEIM, DE, Bd. 108, 1984, Seiten 108-126, XP001194522 ISSN: 0170-2041
- NEIBECKER, D. ET AL NEIBECKER, D. ET AL: "Cationic .eta.3-allylic complexes. 4. Cationic .eta.3-allylic complexes of nickel from S-allyl-1,1,3,3-tetramethylthiouronium salts Cationic .eta.3-allylic complexes. 4. Cationic .eta.3-allylic complexes of nickel from S-allyl-1,1,3,3-tetramethylthiouronium salts" INORGANIC CHEMISTRY , 19(12), 3725-9 CODEN: INOCAJ; ISSN: 0020-1669 INORGANIC CHEMISTRY , 19(12), 3725-9 CODEN: INOCAJ; ISSN: 0020-1669, 1980, XP002302546
- NEIBECKER, D. ET AL: "Cationic .eta.3 allylic complexes. III. The reaction of S-allyl iso tetramethyl thiouronium salts with nickel tetracarbonyl" TETRAHEDRON LETTERS , (27), 2351-2 CODEN: TELEAY; ISSN: 0040-4039, 1977, XP002302547
- DATABASE BEILSTEIN BEILSTEIN INSTITUTE FOR ORGANIC CHEMISTRY, FRANKFURT-MAIN, DE; XP002302552 gefunden im XFIRE Database accession no. 6029233 & CAN. J. CHEM., Bd. 61, 1983, Seiten 235-243,
- DATABASE BEILSTEIN BEILSTEIN INSTITUTE FOR ORGANIC CHEMISTRY, FRANKFURT-MAIN, DE; XP002302553 gefunden im XFIRE Database accession no. 7243216, 7242991, 7240790 & J. ORG. CHEM., Bd. 60, Nr. 8, 1995, Seiten 2330-2343,
- DATABASE BEILSTEIN BEILSTEIN INSTITUTE FOR ORGANIC CHEMISTRY, FRANKFURT-MAIN, DE; XP002302554 gefunden im XFIRE Database accession no. 7160121 & TETRAHEDRON, Bd. 51, Nr. 3, 1995, Seiten 935-940,
- DATABASE BEILSTEIN BEILSTEIN INSTITUTE FOR ORGANIC CHEMISTRY, FRANKFURT-MAIN, DE; XP002302555 gefunden im XFIRE Database accession no. 6883816 & J. GEN. CHEM. USSR, Bd. 58, Nr. 9, 1988, Seiten 1930-1931,
- DATABASE CA [Online] CHEMICAL ABSTRACTS SERVICE, COLUMBUS, OHIO, US; HABERMANN, JOERG ET AL HABERMANN, JOERG ET AL: "Glycopeptide synthesis using O-pentafluorophenyluronium salts as novel condensing reagents Glycopeptide synthesis using O-pentafluorophenyluronium salts as novel condensing reagents" XP002302556 gefunden im STN Database accession no. 1998:239887 & JOURNAL FUER PRAKTISCHE CHEMIE/CHEMIKER-ZEITUNG , 340(3), 233-239 CODEN: JPCCEM; ISSN: 0941-1216 JOURNAL FUER PRAKTISCHE CHEMIE/CHEMIKER-ZEITUNG , 340(3), 233-239 CODEN: JPCCEM; ISSN: 0941-1216, 1998,
- ORGANIC LETTERS, Bd. 5, Nr. 10, 2003, Seiten 1633-1635, XP002302548

## Beschreibung

Die vorliegende Erfindung betrifft Salze enthaltend Uronium- oder Thiouronium-Kationen und verschiedene Anionen, Verfahren zur Herstellung dieser Salze sowie ihre Verwendung als ionische Flüssigkeiten.

Ionische Flüssigkeiten oder flüssige Salze sind ionische Spezies, die aus einem organischen Kation und einem in der Regel anorganischen Anion bestehen. Sie enthalten keine neutralen Moleküle und weisen meistens Schmelzpunkte kleiner 373 K auf. Im Stand der Technik sind eine Vielzahl von Verbindungen bekannt, die als ionische Flüssigkeiten Verwendung finden. So wurden lösungsmittelfreie ionische Flüssigkeiten erstmals von Hurley und Wier in einer Reihe von US-Patenten (US 2,446,331, US 2,446,339 und US 2,446,350) offenbart. Diese "bei Raumtemperatur geschmolzenen Salze" basierten auf AlCl₃ und einer Vielzahl von n-Alkylpyridinium-Halogeniden.

In den letzten Jahren wurden einige Übersichtsartikel zu diesem Thema veröffentlicht (R. Sheldon "Catalytic reactions in ionic liquids", Chem. Commun., 2001, 2399-2407; M.J. Earle, K.R. Seddon "Ionic liquids. Green solvent for the future", Pure Appl. Chem., 72 (2000), 1391-1398; P. Wasserscheid, W. Keim "Ionische Flüssigkeiten - neue Lösungen für die Übergangsmetallkatalyse", Angew. Chem., 112 (2000), 3926-3945; T. Welton "Room temperature ionic liquids. Solvents for synthesis and catalysis", Chem. Rev., 92 (1999), 2071-2083; R. Hagiwara, Ya. Ito "Room temperature ionic liquids of alkylimidazolium cations and fluoroanions", Journal of Fluorine Chem., 105 (2000), 221-227).

Die Eigenschaften ionischer Flüssigkeiten, z.B. Schmelzpunkt, thermische und elektrochemische Stabilität, Viskosität, werden stark von der Natur des Anions beeinflusst. Demgegenüber können die Polarität und die Hydrophilie bzw. Lipophilie durch die geeignete Wahl des Kation/Anion-Paares variiert werden. Daher besteht Bedarf an neuen ionischen Flüssigkeiten mit variierten Eigenschaften, die zusätzliche Möglichkeiten hinsichtlich ihrer Verwendung ermöglichen.

Aufgabe der vorliegenden Erfindung ist es, neue stabile salzartige Verbindungen mit wertvollen Eigenschaften, die als ionische Flüssigkeiten verwendet werden können, sowie ein Verfahren zu ihrer Herstellung zur Verfügung zu stellen. Insbesondere ist es Aufgabe der vorliegenden Erfindung, ionische Verbindungen mit sehr stabilen Kationen bereit zu stellen.

Gelöst wird diese Aufgabe durch das Bereitstellen von Salzen mit Uronium- oder Thiouronium-Kationen, bei denen die positive Ladung über mehrere Heteroatome delokalisiert ist, gemäß der allgemeinen Formel (1), 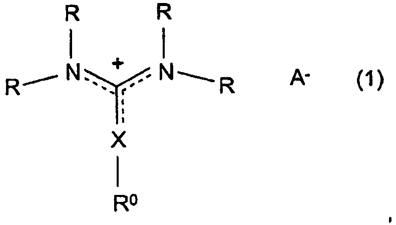 worin
X O oder S bedeutet, gemäß der Ansprüche 5 und 6 und der Verwendung von Verbindungen der allgemeinen Formel (1) worin X O oder S bedeutet gemäß der Ansprüche 1 bis 4 und
worin die Substituenten R und R⁸ jeweils unabhängig voneinander die Bedeutung von
geradkettiges oder verzweigtes Alkyl mit 1-20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen
gesättigtes, teilweise oder vollständig ungesättigtes Cycloalkyl mit 3-7 C-Atomen, welches mit Alkylgruppen mit 1-6 C-Atomen substituiert sein kann, haben,
und R auch Wasserstoff bedeuten kann,
wobei ein oder mehrere Substituenten R oder R⁰ teilweise oder vollständig durch Halogen oder teilweise durch CN oder NO₂ substituiert sein können, wobei jedoch die Halogenierung in α-Stellung in R⁰ ausgeschlossen ist, und
Halogen F, Cl, Br oder I bedeutet,
wobei die Substituenten R und R⁰ paarweise durch Einfach- oder Doppelbindung miteinander verbunden sein können
und wobei ein Kohlenstoffatom oder zwei nicht benachbarte Kohlenstoffatome eines oder mehrerer Substituenten R oder R⁰, die nicht direkt dem Heteroatom benachbart sind, durch Atome und/oder Atomgruppierungen, ausgewählt aus der Gruppe -O-, -C(O)-, -C(O)O-, -S-, -S(O)-, -SO₂-, -SO₃-, -N=, -N=N-, -NH-, -NR'-, -PR'-, -P(O)R'-, -P(O)R'-O-, - 0-P(0)R'-0-, und -P(R')₂=N- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus ist,
und
A- ausgewählt wird aus der Gruppe bestehend aus:
[R¹SO₃]⁻, [R^{F'}SO₃]⁻, [(R^{F}SO₂)₂N]⁻, [(R^{F}SO₂)₃C]⁻, [(FSO₂)₃C]⁻, [R¹CH₂OSO₃]⁻, [R¹C(O)O]⁻, [R^{F'}C(O)O]⁻, [CCl₃C(O)O]⁻, [(CN)₃C]⁻, [(CN)₂CR¹]⁻, [(R¹O(O)C)₂CR¹]⁻, [P(CₙF₂ₙ₊₁₋ₘHₘ)_{y}F_{6-y}]⁻, [P(C₆F₅)yF_{6-y}]⁻, [R¹₂P(O)O]⁻, [R¹P(O)O₂]²⁻, [(R¹O)₂P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4-z}]⁻, [B(CN)₄]⁻, [B(C₆F₅)₄]⁻, [B(OR¹)₄]⁻, [N(CF₃)₂]⁻, [N(CN)₂]⁻, [AlCl₄]⁻ oder [SiF₆]²⁻,
wobei die Substituenten R^{F} und R^{F'} jeweils unabhängig voneinander die Bedeutung von
perfluoriertes und geradkettiges oder verzweigtes Alkyl mit 1-20 C-Atomen, wobei für R^{F'} die Trifluormethylgruppe ausgeschlossen ist, perfluoriertes und geradkettiges oder verzweigtes Alkenyl mit 2-20 C-Atomen und einer oder mehreren Doppelbindungen, perfluoriertes Phenyl und gesättigtes, teilweise oder vollständig ungesättigtes Cycloalkyl mit 3-7 C-Atomen, das mit Perfluoralkylgruppen substituiert sein kann,
wobei die Substituenten R^{F} oder R^{F'} paarweise durch Einfach- oder Doppelbindung miteinander verbunden sein können und wobei ein Kohlenstoffatom oder zwei nicht benachbarte Kohlenstoffatome des Substituenten R^{F} oder R^{F'}, die nicht α-ständig zu dem Heteroatom stehen, durch Atome und/oder Atomgruppierungen, ausgewählt aus der Gruppe -O-, -C(O)-, -S-, -S(O)-, -SO₂-, -N=, -N=N-, -NR'-, -PR'- und -P(O)R'- ersetzt sein können oder eine Endgruppe R'-O-SO₂- oder R'-O-C(O)- besitzen können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und
wobei die Substituenten R¹ jeweils unabhängig voneinander die Bedeutung von
Wasserstoff, für den Fall dass A⁻ = [(CN)₂CR¹]⁻ oder [(R¹O(O)C)₂CR¹]⁻ ist und X = O oder S, oder
Wasserstoff für den Fall dass A⁻ = [R¹CH₂OSO₃]⁻ ist, X = S oder O und die Substitutenten R und R⁰ = Alkylgruppen mit 1 bis 20 C-Atomen sind, geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das durch Alkylgruppen mit 1-6 C-Atomen substituiert sein kann, wobei die Substituenten R¹ teilweise durch CN, NO₂ oder Halogen substituiert sein können und
Halogen F, Cl, Br oder I bedeutet,
wobei die Substituenten R¹ paarweise durch Einfach- oder Doppelbindung miteinander verbunden sein können und
wobei ein Kohlenstoffatom oder zwei nicht benachbarte Kohlenstoffatome des Substituenten R¹, die nicht α-ständig zum Heteroatom stehen, durch Atome und/oder Atomgruppierungen, ausgewählt aus der Gruppe -O-, -C(O)-, -C(O)O-, -S-, -S(O)-, -SO₂-, -SO₃-, -N=, -N=N-, -NH-, -NR'-, -PR'-und -P(O)R'-, (P(O)R'O-, OP(O)R'O-, -PR'₂=N-, -C(O)NH-, -C(O)NR'-, -SO₂NH- oder -SO₂NR'- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und die Variablen
n 1 bis 20,
m 0, 1, 2 oder 3,
y 0, 1, 2, 3, oder 4,
z 0, 1, 2 oder 3 bedeuten,
mit der Maßgabe, dass
für X = S und R und R⁰ = Alkylgruppen mit 1 bis 20 C-Atomen, die Anionen A⁻ = [BF₄]⁻, CF₃COO⁻, [B(C₆H₅)₄]⁻ oder [CH₃C₆H₄SO₃]⁻ zuzulassen sind
und
für X = O die Anionen A⁻ = = CF₃COO⁻ und [B(C₆H₅)₄]⁻ zuzulassen sind und für X = O und R⁰ = Ethyl das Anion A⁻ = CH₃CH₂OSO₃⁻ auszuschliessen ist.

Unter vollständig ungesättigten Substituenten werden im Sinne der vorliegenden Erfindung auch aromatische Substituenten verstanden.

Die erfindungsgemäßen Verbindungen zeichnen sich insbesondere durch sehr stabile Kationen aus.

Bei den erfindungsgemäßen Verbindungen handelt es sich demnach um Salze, die gegebenenfalls substituierte Thiouronium- oder Uronium-Kationen besitzen.

Als Substituenten R kommen erfindungsgemäß dabei neben Wasserstoff in Frage: C₁- bis C₂₀-, insbesondere C₁- bis C₁₂-Alkylgruppen, C₂- bis C₂₀-, insbesondere C₂- bis C₁₂-, Alkenyl- oder Alkinylgruppen und gesättigte oder ungesättigte, d.h. auch aromatische, C₃- bis C₇-Cycloalkylgruppen, die mit C₁- bis C₆-Alkylgruppen substituiert sein können, insbesondere Phenyl.

Die vier Substituenten R der erfindungsgemäßen Salze können gleich oder verschieden sein.

Als Substituent R⁰ kommen erfindungsgemäß dabei Frage: C₁- bis C₂₀-, insbesondere C₁- bis C₁₂-Alkylgruppen, C₂- bis C₂₀-, insbesondere C₂- bis C₁₂-, Alkenyl- oder Alkinylgruppen und gesättigte oder ungesättigte, d.h. auch aromatische, C₃- bis C₇-Cycloalkylgruppen, die mit C₁- bis C₆-Alkylgruppen substituiert sein können, insbesondere Phenyl.

Die C₁-C₁₂-Alkylgruppe ist beispielsweise Methyl, Ethyl, Isopropyl, Propyl, Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, Heptyl, Octyl, Nonyl, Decyl, Undecyl oder Dodecyl. Gegebenenfalls auch teilweise oder vollständig durch F subsitituiert, beispielsweise Difluormethyl, Trifluormethyl, Trifluorethyl, Pentafluorethyl, Pentafluorpropyl, Heptafluorpropyl, Heptafluorbutyl, Nonafluorbutyl oder Nonafluorhexyl.
Bevorzugte Alkylgruppen, wie zuvor beschrieben, haben 1 bis 6 C-Atome.

Unsubstituierte gesättigte oder teilweise oder vollständig ungesättigte Cycloalkylgruppen mit 3-7 C-Atomen sind daher Cyclopropyl, Cyclobutyl, Cyclopentyl, Cyclohexyl, Cycloheptyl, Cyclopentenyl, Cyclopenta-1,3-dienyl, Cyclohexenyl, Cyclohexa-1,3-dienyl, Cyclohexa-1,4-dienyl, Phenyl, Cycloheptenyl, Cyclohepta-1,3-dienyl, Cyclohepta-1,4-dienyl oder Cyclohepta-1,5-dienyl, welche mit C₁- bis C₆-Alkylgruppen substituiert sein können, wobei wiederum die Cycloalkylgruppe oder die mit C₁- bis C₆-Alkylgruppen substituierte Cycloalkylgruppe auch mit Halogenatomen wie F, Cl, Br oder I, insbesondere F oder Cl, CN oder NO₂ substituiert sein kann.

Die Substituenten R und R⁰ können teilweise oder vollständig mit Halogenatomen, insbesondere mit F und/oder Cl, oder teilweise mit CN oder NO₂ substituiert sein, wobei die Halogenierung der α-CH₂-Gruppe von R⁰ ausgeschlossen ist. Ferner können die Substituenten R oder R⁰ ein oder zwei, einander nicht benachbarte Heteroatome oder Atomgruppierungen, ausgewählt aus der Gruppe -O-, -C(O)-, -C(O)O-, -S-, -S(O)-, -SO₂-, -SO₂O-, -N=, -N=N-, -NH-, -NR'-, -PR'-, -P(O)R'-, -P(O)R'-O-, -O-P(O)R'-O-, -P(O)(NR'₂)-NR'- und -PR'₂=N- enthalten, wobei R' ein nicht, teilweise oder perfluoriertes C₁- bis C₆-Alkyl, C₃- bis C₇-Cycloalkyl, unsubstituiertes oder substituiertes Phenyl oder unsubstituierter oder substituierter Heterocyclus, sein kann.

In R' ist C₃- bis C₇-Cycloalkyl beispielweise Cyclopropyl, Cyclobutyl, Cyclopentyl, Cyclohexyl oder Cycloheptyl.

In R' bedeutet substituiertes Phenyl, durch C₁- bis C₆-Alkyl, C₁- bis C₆-Alkenyl, NO₂, F, Cl, Br, I, OH, C₁-C₆-Alkoxy, CN, SCN, SCF₃, SO₂CF₃, C(O)O-C₁-C₆-Alkyl, NH₂, C₁-C₆-Alkylamino oder C₁-C₆-Dialkylamino, COOH, C(O)NR"₂, SO₂OR", SO₂X', SO₂NR"₂, SO₃H oder NHC(O)R" substituiertes Phenyl, wobei X' F, Cl oder Br und R" ein nicht, teilweise oder perfluoriertes C₁- bis C₆-Alkyl oder C₃- bis C₇-Cycloalkyl wie für R' definiert bedeutet, beispielsweise, o-, m- oder p-Methylphenyl, o-, m- oder p-Ethylphenyl, o-, m- oder p-Propylphenyl, o-, m- oder p-Isopropylphenyl, o-, m- oder p-tert.-Butylphenyl, o-, m- oder p-Aminophenyl, o-, m- oder p-(N,N-Dimethylamino)phenyl, o-, m- oder p-Nitrophenyl, o-, m- oder p-Hydroxyphenyl, o-, m- oder p-Methoxyphenyl, o-, m- oder p-Ethoxyphenyl, o-, m-, p-(Trifluormethyl)phenyl, o-, m-, p-(Trifluormethoxy)phenyl, o-, m-, p-(Trifluormethylsulfonyl)phenyl, o-, m- oder p-Fluorphenyl, o-, m- oder p-Chlorphenyl, o-, m- oder p-Bromphenyl, o-, m- oder p-Iodphenyl, weiter bevorzugt 2,3-, 2,4-, 2,5-, 2,6-, 3,4- oder 3,5-Dimethylphenyl, 2,3-, 2,4-, 2,5-, 2,6-, 3,4- oder 3,5-Dihydroxyphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphehyl, 2,3-, 2,4-, 2,5-, 2,6-, 3,4- oder 3,5-Dimethoxyphenyl, 5-Fluor-2-methylphenyl, 3,4,5-Trimethoxyphenyl oder 2,4,5-Trimethylphenyl.

In R' wird als Heterocyclus ein gesättigter oder ungesättigter mono- oder bicyclischer heterocyclischer Rest mit 5 bis 13 Ringgliedern verstanden, wobei 1, 2 oder 3 N- und/oder 1 oder 2 S- oder O-Atome vorliegen können und der heterocyclische Rest ein- oder mehrfach durch C₁- bis C₆-Alkyl, C₁-bis C₆-Alkenyl, NO₂, F, Cl, Br, I, OH, C₁-C₆-Alkoxy, CN, SCN, SCF₃, SO₂CF₃, C(O)O-C₁-C₆-Alkyl, NH₂, C₁-C₆₋Alkylamino oder C₁-C₆-Dialkylamino, COOH, C(O)NR"₂, SO₂OR", SO₂X', SO₂NR"₂, SO₃H oder NHC(O)R" substituiert sein kann, wobei X' und R" eine zuvor angegebene Bedeutung haben. -
Der heterocyclische Rest ist vorzugsweise substituiertes oder unsubstituiertes 2- oder 3-Furyl, 2- oder 3-Thienyl, 1-, 2- oder 3-Pyrrolyl, 1-, 2-, 4- oder 5-Imidazolyl, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3-oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -4- oder -5-yl, 1- oder 5-Tetrazolyl, 1,2,3-Oxadiazol-4- oder -5-yl 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-1 H-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 1-, 2-, 3-, 4-, 5-, 6-, 7- oder 8-Chinolinyl, 1-, 3-, 4-, 5-, 6-, 7- oder 8-Isochinolinyl, 1-, 2-, 3-, 4-oder 9-Carbazolyl, 1-, 2-, 3-, 4-, 5-, 6-, 7-, 8- oder 9-Acridinyl, 3-, 4-, 5-, 6-, 7- oder 8-Cinnolinyl, 2-, 4-, 5-, 6-, 7- oder 8-Chinazolinyl oder 1-, 2- oder 3-Pyrrolidinyl.

Ohne Einschränkung der Allgemeinheit sind Beispiele für Substituenten R und R⁰ des Thiouronium- oder Uronium-Kations:
- CH₃, -C₂H₅, -C₃H₇, -CH(CH₃)₂, -C₄H₉, -C(CH₃)₃, -C₅H₁₁, -C₆H₁₃, -C₇H₁₅, -C₈H₁₇, -C₉H₁₉, -C₁₀H₂₁, -C₁₂H₂₅, -C₂₀H₄₁, -OCH₃, -OCH(CH₃)₂, -CH₂OCH₃, -C₂H₄OH(CH₃)₂, -C₂H₄SC₂H₅, -C₂H₄SCH(CH₃)₂, -S(O)CH₃, -SO₂CH₃, -SO₂C₆H₅, -SO₂C₃H₇, -SO₂CH(CH₃) 2, -SO₂CH₂CF₃, -CH₂SO₂CH₃, -CH₂N(H)C₂H₅, -C₂H₄N(H)C₂H₅, -CH₂N(CH₃)CH₃, -CN, -C₂H₄N(CH₃)CH₃, -CF₃, -C₂F₅, -C₃F₇, -C₄F₉, -C(CF₃)₃, -CF₂SO₂CF₃, -C₂F₄N(C₂F₅)C₂F₅, -CHF₂, -CH₂CF₃, -C₂F₂H₃, -C₃FH₆, -CH₂C₃F₇, -C(CFH₂)₃, -CH₂C(O)OH, -CH₂C₆H₅, -CH₂C(O)CH₃, -CH₂C(O)C₂H₅, -CH₂C(O)OCH₃, CH₂C(O)OC₂H₅, -C(O)CH₃, -C(O)C₆H₅, -C(O)OCH₃, -C(O)OC₂H₅, P(O)(C₂H₅)₂, P(O)[N(C₂H₅)₂]₂,

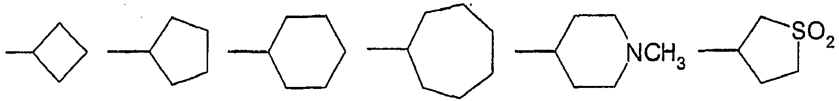

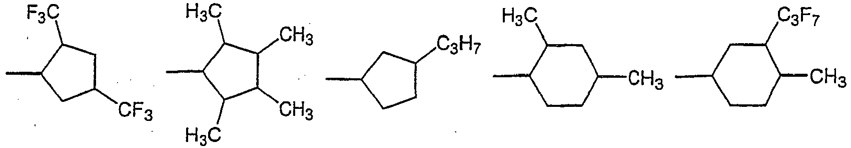

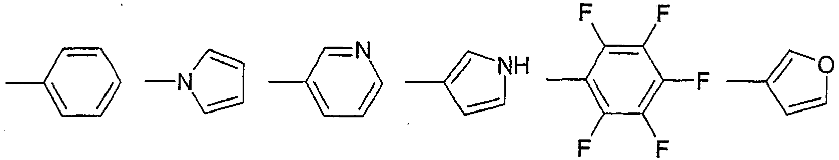

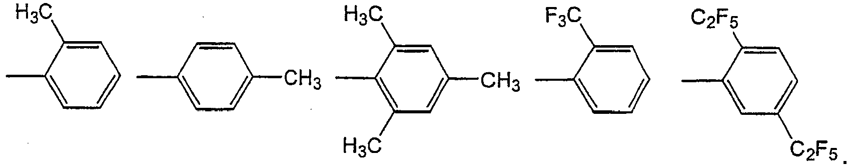

Bis zu vier Substituenten R können auch paarweise derart verbunden sein, dass mono-, bi- oder polycyclische Kationen entstehen.
Ohne Einschränkung der Allgemeinheit sind Beispiele für solche Kationen: 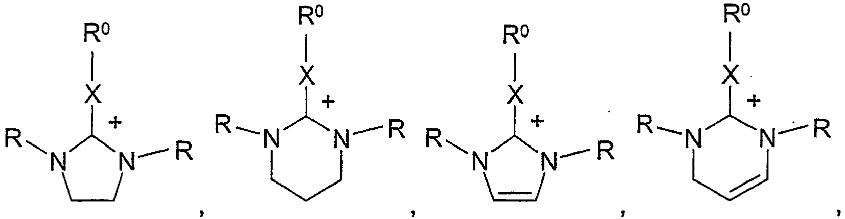 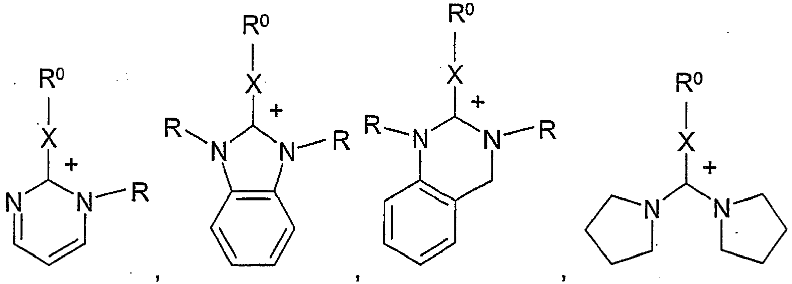 oder 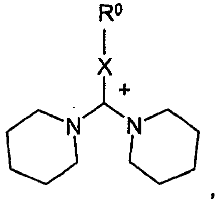 wobei die Substituenten R und R⁰ eine zuvor angegebene oder besonders bevorzugte Bedeutung haben können. Gegebenenfalls können die Carbocyclen oder Heterocyclen der zuvor angegebenen Kationen noch durch C₁- bis C₆-Alkyl, C₁- bis C₆-Alkeny), NO₂, F, Cl, Br, I, OH, C₁-C₆-Alkoxy, CN, SCN, SCF₃, SO₂CF₃, C(O)O-C₁-C₆-Alkyl, NH₂, C₁-C₆-Alkylamino oder C₁-C₆-Dialkylamlno, COOH, C(O)NR"₂, SO₂OR", SO₂NR"₂, SO₂X', SO₃H oder NHC(O)R" oder substituiertes oder unsubstituiertes Phenyl oder unsubstituierter oder substituierter Heterocyclus substituiert sein, wobei X' und R" eine zuvor angegebene Bedeutung haben.

Das Anion A⁻ der erfindungsgemäßen Salze nach Anspruch 5 ist ausgewählt aus [R¹SO₃]⁻, [R^{F'}SO₃)⁻, [(R^{F}SO₂)₂N]⁻, [(R^{F}SO₂)₃C]⁻, [(FSO₂)₃C]⁻, [R¹CH₂C)SO₃]⁻, [R¹C(O)O]⁻, [R^{F'}C(O)O]⁻, [CCl₃C(O)O]⁻, [(CN)₃C]⁻, [(CN)₂CR¹]⁻. [(R¹O(O)C)₂CR¹]⁻, [P(CnF₂ₙ₊₁₋ₘHₘ)_{y}F_{6-y}]⁻, [P(C₆F₆)_{y}F_{6-y}]⁻, [R¹₂P(O)O]⁻, [R¹P(O)O₂]²⁻, [(R¹O)₂P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [B^{F}_{z}R^{F}_{4-z}]⁻, [BF_{z}(CN)_{4-z}]⁻, [B(CN)₄]⁻, [B(C₈F₅)₄]⁻, [B(OR¹)₄]⁻, [N(CF₃)₂]⁻, [N(CN)₂]⁻, [AlCl₄]⁻, [SiF₆]²⁻, CF₃ COO⁻ oder [B(C₆H₅)₄]⁻,
wobei die Substituenten R^{F} und R^{F} jeweils unabhängig voneinander die Bedeutung von
perfluoriertes und geradkettiges oder verzweigtes Alkyl mit 1-20 C-Atomen, wobei für R^{F'} die Trifluormethylgruppe ausgeschlossen ist,
perfluoriertes und geradkettiges oder verzweigtes Alkenyl mit 2-20 C-Atomen und einer oder mehreren Doppelbindungen,
perfluoriertes Phenyl und gesättigtes, teilweise oder vollständig ungesättigtes Cycloalkyl mit 3-7 C-Atömen, das mit Perfluoralkylgruppen substituiert sein kann,
wobei die Substituenten R^{F} oder R^{F'} paarweise durch Einfach- oder Doppelbindung miteinander verbunden sein können und
wobei ein Kohlenstoffatom oder zwei nicht benachbarte Kohlenstoffatome des Substituenten R^{F} oder R^{F'}, die nicht α-ständig zu dem Heteroatom stehen, durch Atome und/oder Atomgruppierungen, ausgewählt aus der Gruppe -O-, -C(O)-, -S-, -S(O)-, -SO₂-, -N=, -N=N-, -NR'-, -PR'- und -P(O)R'- ersetzt sein können oder eine Endgruppe R'-O-SO₂- oder R'-O-C(O)- besitzen können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und
wobei die Substituenten R¹ jeweils unabhängig voneinander die Bedeutung von
Wasserstoff, für den Fall dass A⁻ = [(CN)₂CR¹]⁻ oder [(R¹O(O)C)₂CR¹]⁻ ist und X = O oder S, oder
Wasserstoff für den Fall dass A⁻ = [R¹CH₂OSO₃]- ist, X = S oder O und die Substitutenten R und R⁰ = Alkylgruppen mit 1 bis 20 C-Atomen sind, geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das durch Alkylgruppen mit 1-6 C-Atomen substituiert sein kann, wobei die Substituenten R¹ teilweise durch CN, NO₂ oder Halogen substituiert sein können und
Halogen F, Cl, Br oder I bedeutet,
wobei die Substituenten R¹ paarweise durch Einfach- oder Doppelbindung miteinander verbunden sein können und
wobei ein Kohlenstoffatom oder zwei nicht benachbarte Kohlenstoffatome des Substituenten R¹, die nicht α-ständig zum Heteroatom stehen, durch Atome und/oder Atomgruppierungen, ausgewählt aus der Gruppe -O-, -C(O)-, -C(O)O-, -S-, -S(O)-, -SO₂, -SO₃-, -N=, -N=N-, -NH-, -NR'-, -PR'- und -P(O)R'-, (P(O)R'O-, OP(O)R'O-, -PR'₂=N-, -C(O)NH-, -C(O)NR'-, -SO₂NH- oder -SO₂NR'- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und die Variablen
n 1 bis 20,
m 0, 1, 2 oder 3,
y 1, 2, 3, oder 4,
z 0, 1, 2 oder 3 bedeuten,
mit der Maßgabe, dass
für X = O und R⁰ = Ethyl das Anion A⁻ = CH₃CH₂OSO₃⁻ auszuschliessen ist.

Das Anion A⁻ der erfindungsgemäßen Salze nach Anspruch 6 ist ausgewählt aus
[R¹SO₃]⁻, [R^{F'}SO₃]⁻, [(R^{F}SO₂)₂N]⁻, [(R^{F}SO₂)₃C]⁻, [(FSO₂)₃C]⁻, [R¹CH₂OSO₃]⁻, [R¹C(O)O]⁻, [R^{F'}C(O)O]⁻, [CCl₃C(O)O]⁻, [(CN)₃C]⁻, [(CN)₂CR¹]⁻, [(R¹O(O)C)₂CR¹]⁻, [P(CₙF₂ₙ₊₁₋ₘHₘ)_{y}F_{6-y}]⁻, [P(C₆F₅)_{y}F_{6-y}]⁻, [R¹₂P(O)O]⁻ [R¹P(O)O₂]²⁻, [(R¹O)₂P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4-z}]⁻, [B(CN)₄]⁻, [B(C₆F₅)4]⁻, [B(OR¹)₄]⁻, [N(CF₃)₂]⁻, [N(CN)₂]⁻, [AlCl₄]⁻ oder [SiF₆]²⁻,
wobei die Substituenten R^{F} und R^{F'} jeweils unabhängig voneinander die Bedeutung von
perfluoriertes und geradkettiges oder verzweigtes Alkyl mit 1-20 C-Atomen, wobei für R^{F'} die Trifluormethylgruppe ausgeschlossen ist,
perfluoriertes und geradkettiges oder verzweigtes Alkenyl mit 2-20 C-Atomen und einer oder mehreren Doppelbindungen,
perfluoriertes Phenyl und gesättigtes, teilweise oder vollständig ungesättigtes Cycloalkyl mit 3-7 C-Atomen, das mit Perfluoralkylgruppen substituiert sein kann,
wobei die Substituenten R^{F} oder R^{F'} paarweise durch Einfach- oder Doppelbindung miteinander verbunden sein können und
wobei ein Kohlenstoffatom oder zwei nicht benachbarte Kohlenstoffatome des Substituenten R^{F} oder R^{F'}, die nicht α-ständig zu dem Heteroatom stehen, durch Atome und/oder Atomgruppierungen, ausgewählt aus der Gruppe -O-, -C(O)-, -S-, -S(O)-, -SO₂-, -N=, -N=N-, -NR'-, -PR'- und -P(O)R'- ersetzt sein können oder eine Endgruppe R'-O-SO₂- oder R'-O-C(O)- besitzen können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und
wobei die Substituenten R¹ jeweils unabhängig voneinander die Bedeutung von
Wasserstoff, für den Fall dass A⁻ = [(CN)₂CR¹]⁻ oder [(R¹O(O)C)₂CR¹]⁻ ist und X = O oder S, oder
Wasserstoff für den Fall dass A⁻ = [R¹CH₂OSO₃]⁻ ist, X = S oder O und die Substitutenten R und R⁰ = Alkylgruppen mit 1 bis 20 C-Atomen sind, geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das durch Alkylgruppen mit 1-6 C-Atomen substituiert sein kann, wobei die Substituenten R¹ teilweise durch CN, NO₂ oder Halogen substituiert sein können und
Halogen F, Cl, Br oder I bedeutet,
wobei die Substituenten R¹ paarweise durch Einfach- oder Doppelbindung miteinander verbunden sein können und
wobei ein Kohlenstoffatom oder zwei nicht benachbarte Kohlenstoffatome des Substituenten R¹, die nicht α-ständig zum Heteroatom stehen, durch Atome und/oder Atomgruppierungen, ausgewählt aus der Gruppe -O-, -C(O)-, -C(O)O-, -S-, -S(O)-, -SO₂-, -SO₃-, -N=, -N=N-, -NH-, -NR'-, -PR'-und -P(O)R'-, (P(O)R'O-, OP(O)R'O-, -PR'₂=N-, -C(O)NH-, -C(O)NR'-, -SO₂NH- oder -SO₂NR'- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und die Variablen
n 1 bis 20,
m 0, 1, 2 oder 3,
y 1, 2, 3, oder 4,
z 0, 1, 2 oder 3 bedeuten,
mit der Maßgabe, dass
für X = S und R und R⁰ = Alkylgruppen mit 1 bis 20 C-Atomen, die Anionen A⁻ = [BF₄]⁻, CF₃COO⁻, [B(C₆H₅)₄]⁻ oder [CH₃C₆H₄SO₃]⁻ zuzulassen sind.

Ein geradkettiges oder verzweigtes Alkenyl mit 2 bis 20 C-Atomen, wobei auch mehrere Doppelbindungen vorhanden sein können, ist beispielsweise Allyl, 2- oder 3- Butenyl, Isobytenyl, sek.-Butenyl, ferner 4-Pentenyl, Isopentenyl, Hexenyl, Heptenyl, Octenyl, C₉H₁₇, C₁₀H₁₉, C₂₀H₃₉, vorzugsweise Allyl, 2- oder 3-Butenyl, Isobutenyl, sek.-Butenyl, ferner bevorzugt ist 4-Pentenyl, iso-Pentenyl oder Hexenyl.

Ein geradkettiges oder verzweigtes Alkinyl mit 2 bis 20 C-Atomen, wobei auch mehrere Dreifachbindungen vorhanden sein können, ist beispielsweise Ethinyl, 1- oder 2-Propinyl, 2- oder 3-Butinyl, ferner 4-Pentinyl, 3-Pentinyl, Hexinyl, Heptinyl, Octinyl, -C₉H₁₅, -C₁₀H₁₇ bis -C₂₀H₃₇, vorzugsweise Ethinyl, 1- oder 2-Propinyl, 2- oder 3-Butinyl, 4-Pentinyl, 3-Pentinyl oder Hexinyl.

Für den Fall, dass mehrere R^{F} bzw. R^{F'} in einem Anion vorhanden sind, können diese auch paarweise derart durch Einfach- oder Doppelbindungen verbunden sein, dass bi- oder polycyclische Anionen entstehen.

Ferner können die Substituenten R^{F} oder R^{F'} ein oder zwei, einander nicht benachbarte und nicht zum Heteroatom *α*-ständige Atome oder Atomgruppierungen, ausgewählt aus der Gruppe -O-, -SO₂- und -NR'- oder die Endgruppe -SO₂X' enthalten, wobei R' = nicht, teilweise oder perfluoriertes C₁- bis C₆-Alkyl, C₃- bis C₇-Cycloalkyl, unsubstituiertes oder substituiertes Phenyl, inklusive -C₆F₅, oder unsubstituierter oder substituierter Heterocyclus und X' = F, Cl oder Br, sein kann.

Ohne Einschränkung der Allgemeinheit sind Beispiele für Substituenten R^{F} bzw. R^{F'} des Anions:
- CF₃, -C₂F₅, -C₃F₇, -C₄F₉, -C(CF₃)₃, -CF₂N(CF₃)CF₃, -CF₂OCF₃, - CF₂S(O)CF₃, -CF₂SO₂CF₃, -C₂F₄N(C₂F₅)C₂F₅, CF=CF₂, -C(CF₃)=CFCF₃,-CF₂CF=CFCF₃, -CF=CFN(CF₃)CF₃ oder -CF₂SO₂F, -C(CF₃)=CFCF₃, -CF₂CF=CFCF₃ oder -CF=CFN(CF₃)CF₃.

Bevorzugt ist R^{F'} Pentafluorethyl, Heptafluorpropyl oder Nonafluorbutyl. Bevorzugt ist R^{F} Trifluormethyl, Pentafluorethyl, Heptafluorpropyl oder Nonafluorbutyl.

Mit der Einschränkung der Disclaimer in den Ansprüchen 1 bis 6 sind im folgenden einige Beispiele für erfindungsgemäße Anionen angegeben:
[CF₃CF₂SO₃]⁻, [CH₃CH₂SO₃]⁻, [(CF₃SO₂)₂N]⁻, [(C₂F₅SO₂)₂N]⁻, [(CF₃SO₂)₃C]⁻ [(C₂F₅SO₂)₃C]⁻, [CH₃CH₂OSO₃]⁻, [(FSO₂)₃C]⁻, [CF₃C(O)O]⁻, [CF₃CF₂C(O)O]⁻, [CH₃CH₂C(O)O]⁻, [CH₃C(O)O]⁻, [P(C₂F₅)₃F₃]⁻ [P(CF₃)₃F₃]⁻ [P(C₂F₄H)(CF₃)₂F₃]⁻, [P(C₂F₃H₂)₃F₃]⁻, [P(C₂F₅)(CF₃)₂F₃]⁻, [P(C₆F₅)₃F₃]⁻, [P(C₃F₇)₃F₃]⁻, [P(C₄F₉)₃F₃]⁻, [P(C₂F₅)₂F₄]⁻, [(C₂F₅)₂P(O)O]⁻, [(C₂F₅)P(O)O₂]²⁻, [P(C6F₅)₂F₄]⁻, [(CF₃)₂P(O)O]⁻, [(CH₃)₂P(O)O]⁻, [(C₄F₉)₂P(O)O]⁻, [CF₃P(O)O₂]²⁻, [CH₃P(O)O₂]²⁻, [(CH₃O)₂P(O)O]⁻, [BF₃(CF₃)]⁻, [BF₂(C₂F₅)₂]⁻, [BF₃(C₂F₅)]⁻, [BF₂(CF₃)₂]⁻, [B(C₂F₅)₄]⁻, [BF₃(CN)]⁻, [BF₂(CN)₂]⁻, [B(CN)₄]⁻, [B(CF₃)₄]⁻, [B(OCH₃)₄]⁻, [B(OCH₃)₂(OC₂H₅)]⁻, [B(O₂C₂H₄)₂]⁻, [B(O₂C₂H₂)₂]⁻, [B(O₂CH₄)₂]⁻, [N(CF₃)₂]⁻, [N(CN₂)₂]⁻, [C(CN)₃]⁻, [AlCl₄]⁻ oder [SiF₆]²⁻.
Besonders bevorzugte Anionen dieser Gruppe unter Beachtung der Disclaimer und gemaß der Ansprüche 5, 6 und 11 sind [R^{F}SO₃]⁻, [P(CₙF₂ₙ₊₁₋ₘHₘ)_{y}F_{6-y}]⁻, [R^{F}₂P(O)O]⁻, [BF_{z}R^{F}_{4-z}]⁻ oder [BF_{z}(CN)_{4-z}]⁻, wobei n, m, y und z eine der vorgenannten Bedeutungen haben, beispielsweise [B(CN)₄]⁻, [(C₂F₅)₃PF₃]⁻, [(C₂F₅)₂PF₄]⁻ , [(C₄F₉)₃PF₃]⁻, [(C₃F₇)₃PF₃]⁻, [B(C₂F₅)F₃]⁻,[(CF₃)₂P(O)O]⁻, [(C₂F₅)₂P(O)O]⁻ oder [B(CF₃)₄]⁻. Ganz besonders bevorzugte Anionen dieser Gruppe unter Beachtung der Disclaimer und gemaß der Ansprüche 5, 6 und 11 sind [BF₄]⁻, [(C₂F₅)₃PF₃]⁻, [(C₂F₅)₂P(O)O]⁻ oder [CF₃SO₃]⁻.

Unter den besonders bevorzugten Anionen haben die Phosphate der Formel [P(CₙF₂ₙ₊₁₋ₘHₘ)_{y}F_{6-y}]⁻ eine besondere Bedeutung, da die Auswahl dieses Anions zu besonders niedrig viskosen Salzen der Formel (1) führt.

Erfindungsgemäß ist eine Gruppe von Salzen der Formel (1) bevorzugt, wobei die Substituenten R des Kations Wässerstoff oder eine geradkettige oder verzweigte Alkylgruppe mit 1-12 C-Atomen, insbesondere mit 1-6 C-Atomen bedeuten und A⁻ eine bei Formel (1) oder eine bevorzugt oder besonders bevorzugt angegebene Bedeutung hat.

Erfindungsgemäß ist eine Gruppe von Salzen der Formel (1) bevorzugt, wobei die Substituenten R des Kations aus der Gruppe Methyl, Ethyl, n-Propyl, Isopropyl, n-Butyl, tert.-Butyl, sek.-Butyl, Phenyl und Cyclohexyl ausgewählt werden und A⁻ = eine bei Formel (1) oder eine bevorzugt oder besonders bevorzugt angegebene Bedeutung hat.

Erfindungsgemäß ist eine Gruppe von Salzen der Formel (1) bevorzugt, wobei X Schwefel bedeutet und A⁻ eine bei Formel (1) oder eine bevorzugt oder besonders bevorzugt angegebene Bedeutung hat.

Erfindungsgemäß ist eine Gruppe von Salzen der Formel (1) bevorzugt, wobei X Sauerstoff bedeutet und A⁻ eine bei Formel (1) oder eine bevorzugt oder besonders bevorzugt angegebene Bedeutung hat.

Erfindungsgemäß ist eine Gruppe von Salzen der Formel (1) bevorzugt, wobei R⁰ eine geradkettige oder verzweigte Alkylgruppe mit 1-20 C-Atomen bedeutet, die teilweise oder vollständig fluoriert sein kann, wobei jedoch eine Fluorierung der α-CH₂-Gruppe von R⁰ ausgeschlossen ist, und X Schwefel bedeutet oder wobei R⁰ eine geradkettige oder verzweigte Alkylgruppe mit 1-20 C-Atomen bedeutet und X Sauerstoff bedeutet, und A⁻ eine bei Formel (1) oder eine bevorzugt oder besonders bevorzugt angegebene Bedeutung hat.

Erfindungsgemäß ist eine Gruppe von Salzen der Formel (1) bevorzugt, wobei R⁰ eine geradkettige oder verzweigte Alkylgruppe mit 1-20 C-Atomen bedeutet, die teilweise oder vollständig fluoriert sein kann, X = S ist und die Substituenten R jeweils unabhänig voneinander eine geradkettige oder verzweigte Alkylgruppe mit 1-20 C-Atomen bedeuten und A⁻ eine bei Formel (1) oder eine bevorzugt oder besonders bevorzugt angegebene Bedeutung hat.

Erfindungsgemäß ist eine Gruppe von Salzen der Formel (1) bevorzugt, wobei R° eine geradkettige oder verzweigte Alkylgruppe mit 1-20 C-Atomen bedeutet, X = O ist und die Substituenten R jeweils unabhänig voneinander eine geradkettige oder verzweigte Alkylgruppe mit 1-20 C-Atomen bedeuten und A⁻ = eine bei Formel (1) oder eine bevorzugt oder besonders bevorzugt angegebene Bedeutung hat.

Ein zweiter Gegenstand der vorliegenden Erfindung ist ein Verfahren zur Herstellung der erfindungsgemäßen Salze mit Thiouronium- oder Uronium-Kationen gemäß der allgemeinen Formel (1) gemäß Anspruch 12. Hierzu wird ein Thioharnstoff C(S)(NR₂)₂ oder ein Harnstoff C(O)(NR₂)₂ mit einem Ester AR⁰ oder mit einem Oxoniumsalz (R⁰)₃O⁺ A⁻ alkyliert.

Die Gruppen bzw. Substituenten R⁰ und R sind dabei definiert wie diejenigen der allgemeinen Formel (1) oder besitzen eine besonders angegebene Bedeutung. A kann im Fall der Umsetzung mit dem Ester aus der Gruppe [R¹CH₂OSO₃], [R¹SO₃], [R^{F}SO₃], [R^{F}C(O)O] und [CCl₃C(O)O] und A⁻ kann im Fall der Umsetzung mit dem Oxoniumsalz aus der Gruppe [(FSO₂)₃C]⁻ und [BF₄]⁻ ausgewählt sein.

Vorzugsweise wird die Umsetzung bei einer Temperatur durchgeführt wird, bei der mindestens eine der Komponenten flüssig ist. Besonders bevorzugt wird die Umsetzung in einem Temperaturbereich durchgeführt, bei der die Reaktionsmischung flüssig ist.

Die Reaktion des Thioharnstoffs oder Harnstoffs mit einem Ester oder einem Oxoniumsalz kann in polaren organischen Lösungsmitteln, beispielsweise 1,2-Dichlorethan oder Dichlormethan, in unpolaren organischen Lösungsmitteln, beispielsweise Hexan oder Pentan, oder ohne Lösungsmittel, z.B. in der Salzschmelze, durchgeführt werden. Erfindungsgemäß können an Stelle reiner Lösungsmittel auch Lösungsmittelgemische verwendet werden.

Erfindungsgemäß können die Reagentien mit einem Mengenverhältnis bis zum fünffachen Überschuss eines der Reaktanden umgesetzt werden. Bevorzugt werden die Reaktanden jedoch in äquimolarer Menge eingesetzt.

Die erfindungsgemäßen Salze können mit sehr guten Ausbeuten, in der Regel über 80%, vorzugsweise über 90%, isoliert werden.

Ein weiterer Gegenstand der vorliegenden Erfindung ist ein alternatives Verfahren zur Herstellung eines erfindungsgemäßen Salzes gemäß Anspruch 14 mittels einer Umsalzung. Hierbei wird ein Salz mit einem Thiouronium- oder Uronium-Kation gemäß der allgemeinen Formel (1) und einem Anion A⁻, ausgewählt aus der Gruppe [BF₄]⁻, Br⁻, Cl⁻, I⁻ oder [ClO₄]⁻, mit einem Salz Kt⁺ A- oder mit einer Säure AH, wobei Kt ein Alkali- oder Erdalkalimetall und A gemäß der allgemeinen Formel (1) definiert ist, umgesetzt.

Vorzugsweise wird die Umsetzung bei einer Temperatur durchgeführt wird, bei der mindestens eine der Komponenten flüssig ist. Besonders bevorzugt wird die Umsetzung in einem Temperaturbereich durchgeführt, bei der die Reaktionsmischung flüssig ist.

Die Umsalzung des Thiouronium- oder Uronium-Salzes kann in polaren Lösungsmitteln, beispielsweise Wasser, Acetonitril, Dimethoxyethan, Dimethylformamid, Methanol oder Propionitril, in unpolaren organischen Lösungsmitteln, beispielsweise Dichlormethan, oder ohne Lösungsmittel, z.B. in der Salzschmelze, durchgeführt werden. Erfindungsgemäß können an Stelle reiner Lösungsmittel auch Lösungsmittelgemische verwendet werden.

Erfindungsgemäß können die Reagentien mit einem Mengenverhältnis bis zu 10% Überschuss von einem der Reaktanden umgesetzt werden. Bevorzugt werden die Reaktanden jedoch in äquimolarer Menge eingesetzt.

Die erfindungsgemäßen Salze der Formel (1), wobei das Anion A⁻ = [R^{F}₂P(O)O]⁻ bedeutet, können alternativ auch durch die Umsetzung von einem Tris(perfluoralkyl)phosphinoxid mit einem Alkohol und einem Thioharnstoff C(S)(NR₂)₂ oder einem Harnstoff C(O)(NR₂)₂, wobei die Reste R wie zuvor definiert sind und die stärker basisch sind als der Alkohol, hergestellt werden.

Der geeignete Alkohol wird so ausgewählt, dass nach der Alkylierung der verwendeten Base das gewünschte Kation entsteht.

Die Herstellung der verwendeten Tris(perfluoralkyl)-phosphinoxide kann nach üblichen, dem Fachmann bekannten Methoden erfolgen. Vorzugsweise werden diese Verbindungen durch Umsetzung mit Hexamethyldisiloxan (V.Ya Sememii et al, J. Gen. Chem. USSR (Engl. Trans.) 55, Nr. 12 (1985), 2415-2417) hergestellt.

Alle erfindungsgemäßen Verbindungen besitzen salzartigen Charakter, relativ niedrige Schmelzpunkte (meist unter 100°C) und könnerr als ionische Flüssigkeiten verwendet werden.

Die Salze der Formel (1) können als Lösungsmittel für viele synthetische oder katalytische Reaktionen eingesetzt werden, z.B. Friedel-Crafts-Acylierung und -Alkylierung, Diels-Alder-Cycloadditionen, Hydrogenierungs- und Oxidationsreaktionen, Heck-Reaktionen. Weiterhin können zum Beispiel fluorierte Lösungsmittel für sekundäre und primäre Batterien synthetisiert werden.

Auch der Einsatz der Verbindungen der Formel (1) als nichtwässriger Elektrolyt gegebenenfalls in Kombination mit anderen, dem Fachmann bekannten Elektrolyten, ist möglich.

Daneben können die Salze der Formel (1) als nichtwässerige polare Substanzen in geeigneten Reaktionen als Phasentransferkatalysator, als Sufactant (surface active agent = grenzflächenaktive Stoffe) oder als Medium zur Heterogenisierung von homogenen Katalysatoren verwendet werden.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Die NMR-Spektren wurden an Lösungen in deuterierten Lösungsmitteln bei 20°C an einem Bruker Avance DRX Spektrometer mit einem 5 mm Breitbandkopf ¹H/BB mit Deuterium Lock gemessen. Die Messfrequenzen der verschiedenen Kerne sind: ¹H: 300,13 MHz, ¹¹B: 96,92 MHz, ¹⁹F: 282,41 MHz und ³¹P: 121,49 MHz. Die Methode der Referenzierung wird bei jedem Spektrum bzw. bei jedem Datensatz separat angegeben.

### Beispiel 1: N,N,N',N'-Tetramethyl-S-ethylisothiouronium Triflat

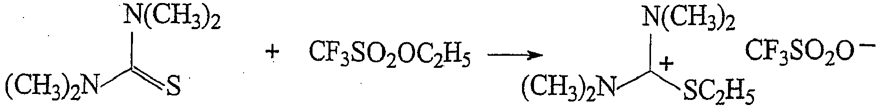

15.0 g (113.4 mmol) N,N,N',N'-Tetramethylthioharnstoff werden in 50 cm³ Dichloromethan gelöst und unter Kühlen mit einem Eisbad und intensivem Rühren der Reaktionsmischung mit einem Magnetrührer werden 20.6 g (115.6 mmol) Ethyltriflat, CF₃SO₂OC₂H₅,langsam (Tropfen für Tropfen)innerhalb von 30 min zugegeben. Die Reaktionsmischung wird weitere 10 min bei Raumtemperatur gerührt. Das Lösungsmittel wird im Vakuum entfernt. Der Rückstand wird dreimal mit 30 cm³ Pentan gewaschen und eine Stunde im Vakuum von 7.0 Pa bei 40-50°C getrocknet. 35.0 g eines leicht schmelzenden kristallinen Materials werden erhalten. Die Ausbeute an N,N,N',N'-Tetramethyl-N"-ethylisothiouronium Triflat ist 99.5 % bezogen auf N,N,N',N'-Tetramethylthioharnstoff.
¹⁹F NMR (Referenz: CCl₃F - interner Standard; Lösungsmittel: CD₃CN) :-77.84 s (CF₃).
¹H NMR (Referenz: TMS ; Lösungsmittel: CD₃CN) : 1.30 t (CH₃); 3.02 q (CH₂); 3.25 s (4CH₃); ³J_{H,H} = 7.4 Hz.

| Elementaranalyse | | | |
|---|---|---|---|
| Gefunden, %: | C 30.82 | H 5.49 | N 9.07 |
| Berechnet für C₈H₁₇F₃N₂O₃S₂, %: | C 30.96 | H 5.52 | N 9.03 |

### Beispiel 2: N,N,N',N'-Tetramethyl-S-ethylisothiouronium Tris(pentafluorethyl)trifluorphosphat

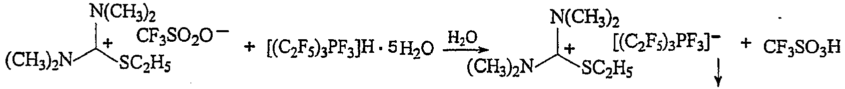

17.1 g (55.1 mmol) N,N,N',N'-Tetramethyl-S-ethylisothiouronium Triflat werden in 70 cm³ Wasser gelöst und unter intensivem Rühren der Reaktionsmischung mit einem Magnetrührer werden 31.0 g (57.8 mmol) Tris(pentafluorethyl)trifluorphosphorsäure-Pentahydrat bei Raumtemperatur zugegeben. Die untere flüssige Phase wird separiert und viermal mit 30 cm³ Wasser gewaschen. Der Rückstand wird drei Stunden im Vakuum von 7.0 Pa bei 60°C in einem Ölbad getrocknet. 30.0 g eines flüssigen Materials werden erhalten. Die Ausbeute an N,N,N',N'-Tetramethyl-S-ethylisothiouronium Tris(pentafluorethyl)trifluorphosphat ist 89.8 % bezogen auf N,N,N',N'-Tetramethyl-S-ethylisothiouronium Triflat.
¹⁹F NMR (Referenz: CCl₃F - interner Standard; Lösungsmittel: CD₃CN) :-43.56 dm (PF); -79.58 m (CF₃); -81.27 m (2CF₃); -86.92 dm (PF₂); -114.93 m (CF₂); -115.52 m (2CF₂); ¹J_{P,F} = 890 Hz ; ¹J_{P,F} = 904 Hz ; ²J_{P,F} = 86 Hz ; ²J_{P,F} = 97 Hz.
¹H NMR (Referenz: TMS ; Lösungsmittel: CD₃CN) : 1.34 t (CH₃); 3.03 q (CH₂); 3.25 s (4CH₃); ³J_{H,H} = 7.4 Hz.
³¹P NMR (Referenz: 85% H₃PO₄ ; Lösungsmittel: CD₃CN): -148.55 dtm ; ¹J_{P,F} = 890 Hz ; ¹J_{P,F} = 902 Hz.

| Elementaranalyse | | | |
|---|---|---|---|
| Gefunden, %: | C 25.63 | H 2.87 | N 4.59 |
| Berechnet für C₁₃H₁₇F₁₈N₂PS, %: | C 25.75 | H 2.83 | N 4.62 |

### Beispiel 3: N,N,N',N'-Tetramethyl-S-(2,2,2-trifluorethyl)isothiouronium Triflat

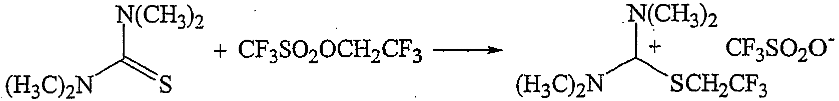

1.0 g (7.56 mmol) N,N,N',N'-Tetramethylthioharnstoff werden in 20 cm³ Hexan gelöst und unter intensivem Rühren der Reaktionsmischung mit einem Magnetrührer werden 1.84 g (7.93 mmol) 2,2,2-Trifluorethyltriflat, CF₃SO₂OCH₂CF₃, langsam (Tropfen für Tropfen) bei Raumtemperatur zugegeben. Die Reaktionsmischung wird weitere 6 Stunden bei Raumtemperatur gerührt. Die untere Phase wird vom Hexan separiert und zweimal mit 10 cm³ Hexan gewaschen. Der Rückstand wird zwei Stunden im Vakuum von 1.3 Pa bei 50°C getrocknet. 2.74 g eines öligen Materials werden erhalten (Smp. 69-70°C). Die Ausbeute an N,N,N',N'-Tetramethyl-S-(2,2,2-trifluorethyl)isothiouronium Triflat ist 99.4 % bezogen auf N,N,N',N'-Tetramethylthioharnstoff.
¹⁹F NMR (Referenz: CCl₃F - interner Standard; Lösungsmittel: CD₃CN):-65.98 t (CF₃); -77.92 t (CF₃); ³J_{H,F} = 9,6 Hz.
¹H NMR (Referenz: TMS ; Lösungsmittel: CD₃CN) : 3.30 s (4CH₃); 3.75 q (CH₂); ³J_{H,F} = 9.6 Hz.

### Beispiel 4: N,N,N',N'-Tetramethyl-S-ethylisothiouronium Tetrafluoroborat

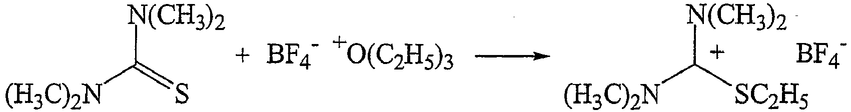

Zu 4.6 g (34.8 mmol) N,N,N',N'-Tetramethylthioharnstoff werden unter intensivem Rühren der Reaktionsmischung mit einem Magnetrührer und Eisbadkühlung 70 cm³ einer 1 M Lösung von Triethyloxoniumtetrafluorborat (69.6 mmol) in Dichlormethan zugegeben. Die Reaktionsmischung wird 6 Stunden bei Raumtemperatur gerührt und alle flüchtigen Komponenten werden im Vakuum entfernt.. Der Rückstand wird dreimal mit 40 cm³ heißem (60°C) Hexan gewaschen und eine Stunde im Vakuum von 1.3 Pa bei Raumtemperatur getrocknet. 6.8 g eines festen Materials werden erhalten. Die Ausbeute an N,N,N',N'- Tetramethyl-S-ethylisothiouronium Tetrafluorborat ist 78.8 % bezogen auf N,N,N',N'-Tetramethylthioharnstoff.
¹⁹F NMR (Referenz: CCl₃F - interner Standard; Lösungsmittel: CD₃CN) : -150.34 s; 150.40 s (BF₄⁻).
¹H NMR (Referenz: TMS ; Lösungsmittel: CD₃CN) : 1.29 t (CH₃); 2.99 q (CH₂); 3.22 s (4CH₃); ³J_{H,H} = 7.4 Hz.

### Beispiel 5: N,N,N',N'-Tetramethyl-O-methylisouronium Triflat

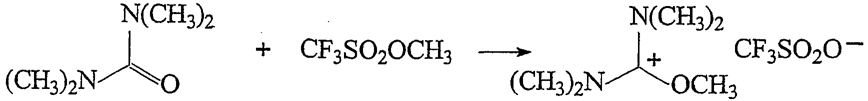

30.0 g (258.3 mmol) N,N,N',N'-Tetramethylharnstoff werden in 150 cm³ Pentan gelöst und unter Kühlung der Reaktionsmischung mit einem Eisbad und intensivem Rühren mit einem Magnetrührer werden langsam (Tropfen für Tropfen) 43.2 g (263.3 mmol) Methyltriflat, CF₃SO₂OCH₃, innerhalb von 30 Minuten zugegeben. Die Reaktionsmischung wird weitere 10 min bei Raumtemperatur gerührt. Die untere flüssige Phase wird separiert und zweimal mit 50 cm³ Pentan gewaschen. Der Rückstand wird eine Stunde im Vakuum von 7.0 Pa bei 60°C getrocknet. 71.0 g eines flüssigen Materials werden erhalten. Die Ausbeute an N,N,N',N'-Tetramethyl-O-methylisouronium Triflat ist 98.1 % bezogen auf N,N,N',N'-Tetramethylharnstoff.
¹⁹F NMR (Referenz: CCl₃F - interner Standard; Lösungsmittel: CD₃CN) :-77.88 s (CF₃).
¹H NMR (Referenz: TMS ; Lösungsmittel: CD₃CN) : 3.05 s (4CH₃); 4.04 s (CH₃).

### Beispiel 6: N,N,N',N'-Tetramethyl-O-methylisouronium Tris(pentafluorethyl)trifluorphosphat

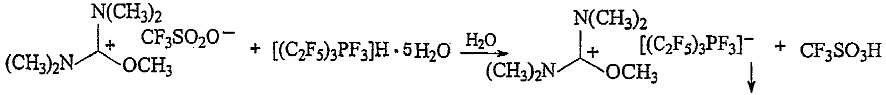

31.5 g (112.4 mmol) N,N,N',N'-Tetramethyl-O-methylisouronium Triflat werden in 300 cm³ Wasser gelöst und unter intensivem Rühren der Reaktionsmischung mit einem Magnetrührer werden 63.3 g (118.7 mmol) Tris(pentafluorethyl)trifluorphosphorsäure-Pentahydrat bei Raumtemperatur zugegeben. Der Niederschlag wird abfiltriert und dreimal mit 30 cm³ Wasser gewaschen. Der Rückstand wird drei Stunden im Vakuum von 7.0 Pa bei 50-60°C in einem Ölbad getrocknet. 62.7 g eines festen weißen Materials werden erhalten (Smp. 69-70°C). Die Ausbeute an N,N,N',N'-Tetramethyl-O-methylisouronium Tris(pentafluorethyl)trifluorphosphat ist 96.8 % bezogen auf N,N,N',N'-Tetramethyl-O-methylisouronium Triflat.
¹⁹F NMR (Referenz: CCl₃F - interner Standard; Lösungsmittel: CD₃CN) :-43.51 dm (PF); -79.54 m (CF₃); -81.23 m (2CF₃); -86.90 dm (PF₂); -114.90 m (CF₂); -115.48 m (2CF₂); ¹J_{P,F} = 889 Hz ; ¹J_{P,F} = 901 Hz ; ²J_{P,F} = 86 Hz ; ²J_{P,F} = 98 Hz.
¹H NMR (Referenz: TMS ; Lösungsmittel: CD₃CN) : 3.05 s (4CH₃); 4.04 s (CH₃).
³¹P NMR (Referenz: 85% H₃PO₄ ; Lösungsmittel: CD₃CN): -148.57 dtm ; ¹J_{P,F} = 890 Hz ¹J_{P,F} = 902 Hz.

| Elementaranalyse | | | |
|---|---|---|---|
| Gefunden, %: | C 24.94 | H 2.64 | N 4.89 |
| Berechnet für C₁₂H₁₅F₁₈N₂OP, %: | C 25.01 | H 2.62 | N 4.86 |

### Beispiel 7: N,N,N',N'-Tetramethyl-O-ethylisouronium Triflat

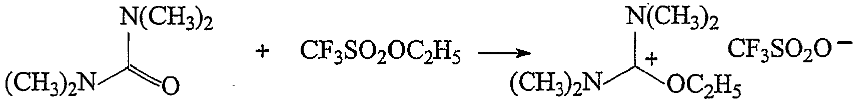

15.0 g (129.1 mmol) N,N,N',N'-Tetramethylharnstoff werden in 70 cm³ of Pentan gelöst und unter Kühlung der Reaktionsmischung mit einem Eisbad und intensivem Rühren mit einem Magnetrührer werden langsam (Tropfen für Tropfen) 23.5 g (131.9 mmol) Ethyltriflat, CF₃SO₂OC₂H₅, innerhalb von 30 Minuten zugegeben. Die Reaktionsmischung wird weitere 10 min bei Raumtemperatur gerührt. Die untere flüssige Phase wird separiert und dreimal mit 30 cm³ Pentan gewaschen. Der Rückstand wird eine Stunde im Vakuum von 7.0 Pa bei 50°C getrocknet. 37.9 g eines flüssigen Materials werden erhalten. Die Ausbeute an N,N,N',N'-Tetramethyl-O-ethylisouronium Triflat ist 99.8 % bezogen auf N,N,N',N'-Tetramethylarnstoff.
¹⁹F NMR (Referenz: CCl₃F - interner Standard; Lösungsmittel: CD₃CN): - 77.86 s (CF₃).
¹H NMR (Referenz: TMS ; Lösungsmittel: CD₃CN) : 1.40 t (CH₃); 3.05 s (4CH₃); 4.38 q (CH₂); ³J_{H,H} = 7.1 Hz.

### Beispiel 8: N,N,N',N'-tetramethyl-O-ethylisouronium Tris(pentafluorethyl)trifluorphosphat

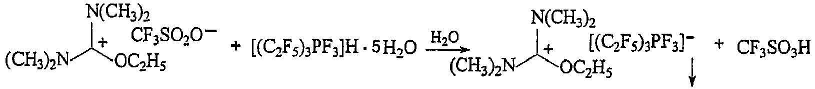

18.8 g (63.9 mmol) N,N,N',N'-Tetramethyl-O-ethylisouronium Triflat werden in 70 cm³ Wasser gelöst und unter intensivem Rühren der Reaktionsmischung mit einem Magnetrührer werden 36.0 g (67.2 mmol) Tris(pentafluorethyl)trifluorphosphorsäure-Pentahydrat bei Raumtemperatur zugegeben. Der Niederschlag wird abfiltriert und viermal mit 30 cm³ Wasser gewaschen. Der Rückstand wird drei Stunden im Vakuum von 7.0 Pa bei 60°C in einem Ölbad getrocknet. 36.7 g eines festen weißen Materials werden erhalten (Smp. 32-34°C). Die Ausbeute an N,N,N',N'-Tetramethyl-O-ethylisouronium Tris(pentafluorethyl)trifluorphosphat ist 97.3 % bezogen auf N,N,N',N'-Tetramethyl-O-ethylisouronium Triflat.
¹⁹F NMR (Referenz: CCl₃F - interner Standard; Lösungsmittel: CD₃CN) : -43.50 dm (PF); -79.52 m (CF₃); -81.21 m (2CF₃); -86.88 dm (PF₂); -114.90 m (CF₂); -115.48 m (2CF₂); ¹J_{P,F} = 889 Hz ; ¹J_{P,F} = 900 Hz ; ²J_{P,F} = 84 Hz ; ²J_{P,F} = 98 Hz.
¹H NMR (Referenz: TMS ; Lösungsmittel: CD₃CN) : 1.42 t (CH₃); 3.05 s (4CH₃); 4.37 q (CH₂); ³J_{H,H} = 7.0 Hz.
³¹P NMR (Referenz: 85% H₃PO₄ ; Lösungsmittel: CD₃CN): -148.60 dtm ;
¹J_{P,F} = 888 Hz ; ¹J_{P,F} = 902 Hz.

| Elementaranalyse | | | |
|---|---|---|---|
| Gefunden, %: | C 26.50 | H 2.95 | N 4.78 |
| Berechnet für C₁₃H₁₇F₁₈N₂OP, %: | C 26.45 | H 2.90 | N 4.75 |

### Beispiel 9: 2-Methyl-1,1,3,3-tetramethylisouronium-Bis(pentafluorethyl)phosphinat

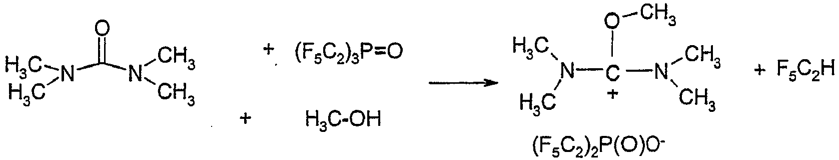

6.72 g (16.6 mmol) Tris(pentafluorethyl)phosphinoxid, (C₂F₅)₃P=O, werden in einem 25 ml Kolben, der mit einem Rückflusskühler ausgestattet ist, mit 15 cm³ Dimethoxyethan und 1.93 g (16.6 mmol) Tetramethylharnstoff vermischt. Zu dieser Mischung werden 0.532 g (16.6 mmol) Methanol unter Rühren der Reaktionsmischung mit einem Magnetrührer zugegeben. Die Reaktionsmischung wird 5 Stunden gekocht und alle flüchtigen Produkte werden im Hochvakuum (1.4 Pa) bei 50° C entfernt. 6.59 g einer viskosen Flüssigkeit werden erhalten. Die Ausbeute an 2-Methyl-1,1,3,3-tetramethylisouronium-Bis(pentafluorethyl)phosphinat ist 91.9 %.
¹⁹F NMR (Referenz: CCl₃F - interner Standard; Lösungsmittel: CD₃CN) :-80.21 m (2CF₃); -124.91 dm (2CF₂); ²J_{P,F} = 67 Hz.
¹H NMR (Referenz: TMS ; Lösungsmittel: CD₃CN) : 3.05 s (4CH₃); 4.05 s (OCH₃).
³¹P NMR (Referenz: 85 % H₃PO₄; Lösungsmittel: CD₃CN) : -2.12 quin. ; ²J_{P,F} = 67 Hz.

## Patentansprüche

1. Verwendung eines Salzes der Formel (1), 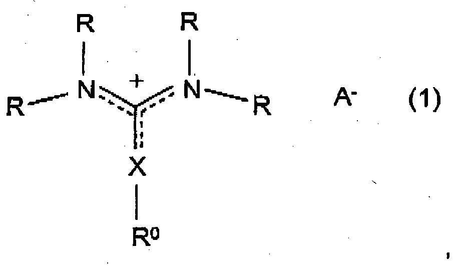 worin
X O oder S bedeutet,
worin die Substituenten R und R⁰ jeweils unabhängig voneinander die Bedeutung von
geradkettiges oder verzweigtes Alkyl mit 1-20 C-Atomen,
geradkettiges oder verzweigtes Alkenyl mit 2-20 C-Atomen und
einer oder mehreren Doppelbindungen,
geradkettiges oder verzweigtes Alkinyl mit 2-20 C-Atomen und
einer oder mehreren Dreifachbindungen
gesättigtes, teilweise oder vollständig ungesättigtes Cycloalkyl mit 3-7 C-Atomen, welches mit Alkylgruppen mit 1-6 C-Atomen substituiert sein kann, haben,
und R auch Wasserstoff bedeuten kann,
wobei ein oder mehrere Substituenten R oder R⁰ teilweise oder
vollständig durch Halogen oder teilweise durch CN oder NO₂ substituiert sein können, wobei jedoch die Halogenierung in α-Stellung in R⁰ ausgeschlossen ist, und
Halogen F, Cl, Br oder I bedeutet,
wobei die Substituenten R und R⁰ paarweise durch Einfach- oder Doppelbindung miteinander verbunden sein können,
und wobei ein Kohlenstoffatom oder zwei nicht benachbarte Kohlenstoffatome eines oder mehrerer Substituenten R oder R⁰, die nicht direkt dem Heteroatom benachbart sind, durch Atome und/oder Atomgruppierungen, ausgewählt aus der Gruppe -O-, -C(O)-, -C(O)O-,-S-, -S(O)-, -SO₂-, -SO₃-, -N=, -N=N-, -NH-, -NR'-, -PR'-, -P(O)R'-,-P(0)R'-0-, -O-P(O)R'-O-, und -P(R')₂=N- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus ist,
und
A⁻ ausgewählt wird aus der Gruppe bestehend aus:
[R¹SO₃]⁻, [R^{F'}SO₃]⁻, [(R^{F}SO₂)₂N]⁻, [(R^{F}SO₂)₃C]⁻, [(FSO₂)₃C]⁻, [R¹CH₂OSO₃]⁻, [R¹C(O)O]⁻, [R^{F'}C(O)O]⁻, [CCl₃C(O)O]⁻, [(CN)₃C]⁻, [(CN)₂CR¹]⁻. [(R¹O(O)C)₂CR¹]⁻, [P(CₙF₂ₙ₊₁₋ₘHₘ)_{y}F_{6-y}]⁻, [P(C₆F₅)_{y}F_{6-y}]⁻, [R¹₂P(O)O]⁻, [R¹P(O)O₂]²⁻, [(R¹O)₂P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4-z}]⁻ , [B(CN)₄]⁻, [B(C₆F₅)₄]⁻, [B(OR¹)₄]⁻, [N(CF₃)₂]⁻, [N(CN)₂]⁻, [AlCl₄]⁻ oder [SiF₆]²⁻,
wobei die Substituenten R^{F} und R^{F'} jeweils unabhängig voneinander die Bedeutung von
perfluoriertes und geradkettiges oder verzweigtes Alkyl mit 1-20 C-Atomen,
wobei für R^{F'} die Trifluormethylgruppe ausgeschlossen ist,
perfluoriertes und geradkettiges oder verzweigtes Alkenyl mit 2-20 C-Atomen und einer oder mehreren Doppelbindungen,
perfluoriertes Phenyl und gesättigtes, teilweise oder vollständig ungesättigtes Cycloalkyl mit 3-7 C-Atomen, das mit Perfluoralkylgruppen substituiert sein kann,
wobei die Substituenten R^{F} oder R^{F'} paarweise durch Einfach- oder
Doppelbindung miteinander verbunden sein können und
wobei ein Kohlenstoffatom oder zwei nicht benachbarte Kohlenstoffatome des Substituenten R^{F} oder R^{F}, die nicht α-ständig zu dem Heteroatom stehen, durch Atome und/oder Atomgruppierungen,
ausgewählt aus der Gruppe -O-, -C(O)-. -S-, -S(O)-, -SO₂-, -N=, -N=N-,-NR'-, -PR'- und -P(O)R'- ersetzt sein können oder eine Endgruppe R'-O-SO₂- oder R'-O-C(O)- besitzen können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und
wobei die Substituenten R¹ jeweils unabhängig voneinander die Bedeutung von
Wasserstoff, für den Fall dass A⁻ = [(CN)₂CR¹]⁻ oder [(R¹O(O)C)₂CR¹]⁻ ist und X = O oder S, oder
Wasserstoff für den Fall dass A⁻ = [R¹CH₂OSO₃]⁻ ist, X = S oder O und
die Substitutenten R und R⁰ = Alkylgruppen mit 1 bis 20 C-Atomen sind,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das durch Alkylgruppen mit 1-6 C-Atomen substituiert sein kann,
wobei die Substituenten R¹ teilweise durch CN, NO₂ oder Halogen substituiert sein können und
Halogen F, Cl, Br oder I bedeutet,
wobei die Substituenten R¹ paarweise durch Einfach- oder Doppelbindung miteinander verbunden sein können und wobei ein Kohlenstoffatom oder zwei nicht benachbarte Kohlenstoffatome des Substituenten R¹, die nicht α-ständig zum Heteroatom stehen, durch Atome und/oder Atomgruppierungen, ausgewählt aus der Gruppe -O-, -C(O)-, -C(O)O-, -S-, -S(O)-, -SO₂-, -SO₃-, -N=, -N=N-, -NH-, -NR'-, -PR'- und -P(O)R'-, (P(O)R'O-, OP(O)R'O-, -PR'₂=N-, -C(O)NH-, -C(O)NR'-, -SO₂NH- oder -SO₂NR'-ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und die Variablen
n 1 bis 20,
m 0, 1, 2 oder 3,
y 0, 1, 2, 3, oder 4,
z 0, 1, 2 oder 3 bedeuten,
mit der Maßgabe, dass
für X = S und R und R⁰ = Alkylgruppen mit 1 bis 20 C-Atomen, die Anionen A⁻ = [BF₄]⁻, CF₃COO⁻ oder [B(C₆H₅)₄]⁻ zuzulassen sind
und
für X = O die Anionen A⁻ = CF₃COO⁻ und [B(C₆H₅)₄]⁻ zuzulassen sind und
für X = O und R° = Ethyl das Anion A⁻ = CH₃CH₂OSO₃⁻ auszuschliessen ist,
als ionische Flüssigkeit.

2. Verwendung eines Salzes der Formel (1), 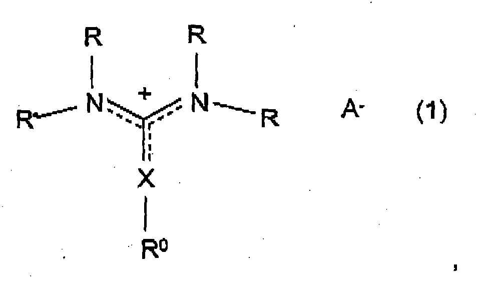 worin
X O oder S bedeutet,
worin die Substituenten R und R⁰ jeweils unabhängig voneinander die Bedeutung von
geradkettiges oder verzweigtes Alkyl mit 1-20 C-Atomen,
geradkettiges oder verzweigtes Alkenyl mit 2-20 C-Atomen und
einer oder mehreren Doppelbindungen,
geradkettiges oder verzweigtes Alkinyl mit 2-20 C-Atomen und
einer oder mehreren Dreifachbindungen
gesättigtes, teilweise oder vollständig ungesättigtes Cycloalkyl mit 3-7 C-Atomen, welches mit Alkylgruppen mit 1-6 C-Atomen substituiert sein kann, haben,
und R auch Wasserstoff bedeuten kann,
wobei ein oder mehrere Substituenten R oder R⁰ teilweise oder vollständig durch Halogen oder teilweise durch CN oder NO₂ substituiert sein können, wobei jedoch die Halogenierung in α-Stellung in R⁰ ausgeschlossen ist, und
Halogen F, Cl. Br oder I bedeutet,
wobei die Substituenten R und R⁰ paarweise durch Einfach- oder Doppelbindung miteinander verbunden sein können
und wobei ein Kohlenstoffatom oder zwei nicht benachbarte Kohlenstoffatome eines oder mehrerer Substituenten R oder R⁰, die nicht direkt dem Heteroatom benachbart sind, durch Atome und/oder Atomgruppierungen, ausgewählt aus der Gruppe -O-, -C(O)-, -C(O)O-,-S-, -S(O)-, -SO₂-, -SO₃-, -N=, -N=N-, -NH-, -NR'- , -PR'-, -P(0)R'-,-P(O)R'-O-, -O-P(O)R'-O-, und -P(R')₂=N- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus ist,
und
A⁻ ausgewählt wird aus der Gruppe bestehend aus:
[R¹SO₃]⁻, [R^{F'}SO₃]⁻, [(R^{F}SO₂)₂N]⁻, [(R^{F}SO₂)₃C]⁻, [(FSO₂)₃C]⁻, [R¹CH₂OSO₃]⁻, [R¹C(O)O]⁻, [R^{F'}C(O)O]⁻, [CCl₃C(O)O]⁻, [(CN)₃C]⁻, [(CN)₂CR¹]⁻, [(R¹O(O)C)₂CR¹]⁻, [P(CₙF₂ₙ₊₁₋ₘHₘ)_{y}F_{6-y}]⁻, [P(C₆F₅)_{y}F_{6-y}]⁻, [R¹₂P(O)O]⁻, [R¹P(O)O₂]²⁻, [(R¹O)₂P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4-z}]⁻ , [B(CN)₄]⁻, [B(C₆F₅)₄]⁻, [B(OR¹)₄]⁻, [N(CF₃)₂]⁻, [N(CN)₂]⁻, [AlCl₄]⁻ oder [SiF₆]2⁻,
wobei die Substituenten R^{F} und R^{F'} jeweils unabhängig voneinander die Bedeutung von
perfluoriertes und geradkettiges oder verzweigtes Alkyl mit 1-20 C-Atomen,
wobei für R^{F'} die Trifluormethylgruppe ausgeschlossen ist,
perfluoriertes und geradkettiges oder verzweigtes Alkenyl mit 2-20 C-Atomen und einer oder mehreren Doppelbindungen,
perfluoriertes Phenyl und gesättigtes, teilweise oder vollständig ungesättigtes Cycloalkyl mit 3-7 C-Atomen, das mit Perfluoralkylgruppen substituiert sein kann,
wobei die Substituenten R^{F} oder R^{F'} paarweise durch Einfach- oder Doppelbindung miteinander verbunden sein können und
wobei ein Kohlenstoffatom oder zwei nicht benachbarte Kohlenstoffatome des Substituenten R^{F} oder R^{F'}, die nicht α-ständig zu dem Heteroatom stehen, durch Atome und/oder Atomgruppierungen, ausgewählt aus der Gruppe -O-, -C(O)-, -S-, -S(O)-, -SO₂-, -N=, -N=N-,-NR'-, -PR'- und -P(0)R'- ersetzt sein können oder eine Endgruppe R'-O-SO₂- oder R'-O-C(O)- besitzen können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und
wobei die Substituenten R¹ jeweils unabhängig voneinander die Bedeutung von
Wasserstoff, für den Fall dass A⁻ = [(CN)₂CR¹]⁻ oder [(R¹O(O)C)₂CR¹]⁻ ist und X = O oder S, oder
Wasserstoff für den Fall dass A⁻ = [R¹CH₂OSO₃]⁻ ist, X = S oder O und die Substitutenten R und R⁰ = Alkylgruppen mit 1 bis 20 C-Atomen sind, geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das durch Alkylgruppen mit 1-6 C-Atomen substituiert sein kann,
wobei die Substituenten R¹ teilweise durch CN, NO₂ oder Halogen substituiert sein können und
Halogen F, Cl, Br oder I bedeutet,
wobei die Substituenten R¹ paarweise durch Einfach- oder
Doppelbindung miteinander verbunden sein können und
wobei ein Kohlenstoffatom oder zwei nicht benachbarte Kohlenstoffatome des Substituenten R¹, die nicht α-ständig zum Heteroatom stehen, durch Atome und/oder Atomgruppierungen, ausgewählt aus der Gruppe -O-, -C(O)-, -C(O)O-, -S-, -S(O), -SO₂-, -SO₃-, -N=, -N=N-, -NH-, -NR'-, -PR'- und -P(O)R'-, (P(O)R'O-, OP(O)R'O-, -PR'₂=N-, -C(O)NH-, -C(O)NR'-, -SO₂NH- oder-SO₂NR'-ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und die Variablen
n 1 bis 20,
m 0, 1, 2 oder 3,
y 0, 1, 2, 3, oder 4,
z 0, 1, 2 oder 3 bedeuten,
mit der Maßgabe, dass
für X = S und R und R⁰ = Alkylgruppen mit 1 bis 20 C-Atomen, die Anionen A⁻ = [BF₄)⁻, CF₃COO⁻ oder [B(C₆H₅)₄]⁻ zuzulassen sind
und
für X = O die Anionen A⁻ = CF₃COO⁻ und [B(C₆H₅)₄]⁻ zuzulassen sind und
für X = O und R⁰ = Ethyl das Anion A⁻ = CH₃CH₂OSO₃⁻ auszuschliessen ist,
als nicht-wässriger Elektrolyt.

3. Verwendung eines Salzes der Formel (1), 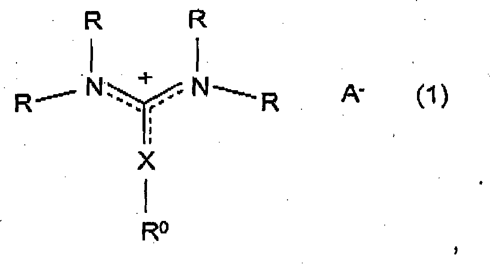 worin
X O oder bedeutet,
worin die Substituenten R und R⁰ jeweils unabhängig voneinander die Bedeutung von
geradkettiges oder verzweigtes Alkyl mit 1-20 C-Atomen,
geradkettiges oder verzweigtes Alkenyl mit 2-20 C-Atomen und
einer oder mehreren Doppelbindungen,
geradkettiges oder verzweigtes Alkinyl mit 2-20 C-Atomen und
einer oder mehreren Dreifachbindungen
gesättigtes, teilweise oder vollständig ungesättigtes Cycloalkyl mit 3-7 C-Atomen, welches mit Alkylgruppen mit 1-6 C-Atomen substituiert sein kann, haben,
und R auch Wasserstoff bedeuten kann,
wobei ein oder mehrere Substituenten R oder R⁰ teilweise oder vollständig durch Halogen oder teilweise durch CN oder NO₂ substituiert sein können, wobei jedoch die Halogenierung in α-Stellung in R⁰ ausgeschlossen ist, und
Halogen F, Cl, Br oder I bedeutet,
wobei die Substituenten R und R⁰ paarweise durch Einfach- oder Doppelbindung miteinander verbunden sein können
und wobei ein Kohlenstoffatom oder zwei nicht benachbarte Kohlenstoffatome eines oder mehrerer Substituenten R oder R⁰, die nicht direkt dem Heteroatom benachbart sind, durch Atome und/oder Atomgruppierungen, ausgewählt aus der Gruppe -O-, -C(O)-, -C(0)0-, - S-, -S(O)-, -SO₂-, -SO₃-, -N=, -N=N-, -NH-, -NR'-, -PR'-, -P(O)R'-,-P(0)R'-0-, -O-P(O)R'-O-, und -P(R')₂=N- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus ist,
und
A⁻ = ausgewählt wird aus der Gruppe bestehend aus:
[R¹SO₃]⁻, [R^{F'}SO₃]⁻, [(R^{F}SO₂)₂N]⁻, [(R^{F}SO₂)₃C]⁻, [(FSO₂)₃C]⁻. [R¹CH₂OSO₃]⁻, [R¹C(O)O]⁻, [R^{F'}C(O)O]⁻, [CCl₃C(O)O]⁻, [(CN)₃C]⁻ [(CN)₂CR¹]⁻, [(R¹O(O)C)₂CR¹]⁻, [P(CₙF₂ₙ₊₁₋ₘHₘ)_{y}F_{6-y}]⁻, [P(C₆F₅)_{y}F_{6-y}]⁻, [R¹₂P(O)O]⁻, [R¹P(O)O₂]²⁻, [(R¹O)₂P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4-z}]⁻ [B(CN)₄]⁻, [B(C₆F₅)₄]⁻, [B(OR¹)₄]⁻. [N(CF₃)₂]⁻, [N(CN)₂]⁻, [AlCl₄]⁻ oder [SiF₆]²⁻,
wobei die Substituenten R^{F} und R^{F} jeweils unabhängig voneinander die Bedeutung von
perfluoriertes und geradkettiges oder verzweigtes Alkyl mit 1-20 C-Atomen,
wobei für R^{F'} die Trifluormethylgruppe ausgeschlossen ist,
perfluoriertes und geradkettiges oder verzweigtes Alkenyl mit 2-20 C-Atomen und einer oder mehreren Doppelbindungen,
perfluoriertes Phenyl und gesättigtes, teilweise oder vollständig ungesättigtes Cycloalkyl mit 3-7 C-Atomen, das mit Perfluoralkylgruppen substituiert sein kann,
wobei die Substituenten R^{F} oder R^{F'} paarweise durch Einfach- oder Doppelbindung miteinander verbunden sein können und
wobei ein Kohlenstoffatom oder zwei nicht benachbarte Kohlenstoffatome des Substituenten R^{F} oder R^{F'}, die nicht α-ständig zu dem Heteroatom stehen, durch Atome und/oder Atomgruppierungen, ausgewählt aus der Gruppe -O-, -C(O)-, -S-, -S(O)-, -SO₂-, -N=, -N=N-,-NR'-, -PR'- und -P(O)R'- ersetzt sein können oder eine Endgruppe R'-O-SO₂- oder R'-O-C(O)- besitzen können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und
wobei die Substituenten R¹ jeweils unabhängig voneinander die Bedeutung von
Wasserstoff, für den Fall dass A⁻ = [(CN)₂CR¹]⁻ oder [(R¹O(O)C)₂CR¹]⁻ ist und X = O oder S, oder
Wasserstoff für den Fall dass A⁻ = [R¹CH₂OSO₃]⁻ ist, X = S oder O und die Substitutenten R und R⁰ = Alkylgruppen mit 1 bis 20 C-Atomen sind, geradkettiges oder verzweigtes Alkyl mit 1-20 C-Atomen, geradkettiges oder verzweigtes Alkenyl mit 2-20 C-Atomen und einer oder mehreren Doppelbindurigen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das durch Alkylgruppen mit 1-6 C-Atomen substituiert sein kann,
wobei die Substituenten R¹ teilweise durch CN, NO₂ oder Halogen substituiert sein können und
Halogen F, Cl, Br oder I bedeutet,
wobei die Substituenten R¹ paarweise durch Einfach- oder Doppelbindung miteinander verbunden sein können und
wobei ein Kohlenstoffatom oder zwei nicht benachbarte Kohlenstoffatome des Substituenten R¹, die nicht α-ständig zum Heteroatom stehen, durch Atome und/oder Atomgruppierungen, ausgewählt aus der Gruppe -O-, -C(O)-, -C(O)O-, -S-, -S(O)-, -SO₂-, -SO₃-, -N=, -N=N-, -NH-, -NR'-, -PR'- und -P(O)R'-, (P(O)R'O-, OP(O)R'O-, -PR'₂=N-, -C(O)NH-, -C(O)NR'-, -SO₂NH- oder -SO₂NR'-ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und die Variablen
n 1 bis 20,
m 0, 1, 2 oder 3,
y 0, 1, 2, 3, oder 4,
z 0, 1, 2 oder 3 bedeuten,
mit der Maßgabe, dass
für X = S und R und R⁰ = Alkylgruppen mit 1 bis 20 C-Atomen, die Anionen A⁻ = [BF₄]⁻, CF₃COO⁻ oder [B(C₆H₅)₄]⁻ zuzulassen sind
und
für X = O die Anionen A⁻ = CF₃COO⁻ und [B(C₆H₅)₄]⁻ zuzulassen sind und
für X = O und R⁰ = Ethyl das Anion A⁻ = = CH₃CH₂OSO₃⁻ auszuschliessen ist,
als Phasentransferkatalysator.

4. Verwendung eines Salzes der Formel (1), 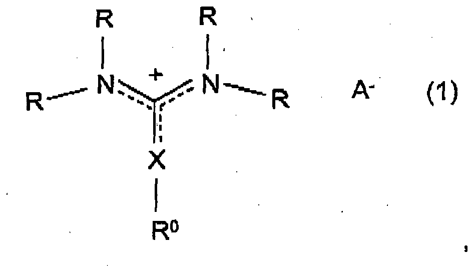 worin
X O oder S bedeutet,
worin die Substituenten R und R⁰ jeweils unabhängig voneinander die Bedeutung von
geradkettiges oder verzweigtes Alkyl mit 1-20 C-Atomen, geradkettiges oder verzweigtes Alkenyl mit 2-20 C-Atomen und
einer oder mehreren Doppelbindungen,
geradkettiges oder verzweigtes Alkinyl mit 2-20 C-Atomen und
einer oder mehreren Dreifachbindungen
gesättigtes, teilweise oder vollständig ungesättigtes Cycloalkyl mit 3-7 C-Atomen, welches mit Alkylgruppen mit 1-6 C-Atomen substituiert sein kann, haben,
und R auch Wasserstoff bedeuten kann,
wobei ein oder mehrere Substituenten R oder R⁰ teilweise oder vollständig durch Halogen oder teilweise durch CN oder NO₂ substituiert sein können, wobei jedoch die Halogenierung in α-Stellung in R⁰ ausgeschlossen ist, und
Halogen F, Cl, Br oder I bedeutet,
wobei die Substituenten R und R⁰ paarweise durch Einfach- oder Doppelbindung miteinander verbunden sein können
und wobei ein Kohlenstoffatom oder zwei nicht benachbarte Kohlenstoffatome eines oder mehrerer Substituenten R oder R⁰, die nicht direkt dem Heteroatom benachbart sind, durch Atome und/oder Atomgruppierungen, ausgewählt aus der Gruppe -O-, -C(O)-, -C(0)0-,-S-, -S(O)-. -SO₂-, -SO₃-, -N=, -N=N-, -NH-, -NR'-, -PR'-, -P(0)R'-, - P(O)R'-O-, -O-P(O)R'-O-, und -P(R')₂=N- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus ist,
und
A' ausgewählt wird aus der Gruppe bestehend aus:
[R¹SO₃]⁻, [R^{F'}SO₃]⁻, [(R^{F}SO₂)₂N]⁻, [(R^{F}SO₂)₃C]⁻, [(FSO₂)₃C]⁻, [R¹CH₂OSO₃]⁻, [R¹C(O)O]⁻, [R^{F'}C(O)O]⁻, [CCl₃C(O)O]⁻, [(CN)₃C]⁻, [(CN)₂CR¹]⁻, [(R¹O(O)C)₂CR¹]⁻, [P(CₙF₂ₙ₊₁₋ₘHₘ)_{y}F_{6-y}]⁻, [P(C₆F₅)_{y}F_{6-y}]⁻, [R¹₂P(O)O]⁻, [R¹P(O)O₂]²⁻, [(R¹O)₂P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4-z}]⁻ , [B(CN)₄]⁻, [B(C₆F₅)₄]⁻, [B(OR¹)₄]⁻, [N(CF₃)₂]⁻, [N(CN)₂]⁻, [AlCl₄]⁻ oder [SiF₆]²⁻,
wobei die Substituenten R^{F} und R^{F'} jeweils unabhängig voneinander die Bedeutung von
perfluoriertes und geradkettiges oder verzweigtes Alkyl mit 1-20 C-Atomen,
wobei für R^{F'} die Trifluormethylgruppe ausgeschlossen ist, perfluoriertes und geradkettiges oder verzweigtes Alkenyl mit 2-20 C-Atomen und einer oder mehreren Doppelbindungen,
perfluoriertes Phenyl und gesättigtes, teilweise oder vollständig ungesättigtes Cycloalkyl mit 3-7 C-Atomen, das mit Perfluoralkylgruppen substituiert sein kann,
wobei die Substituenten R^{F} oder R^{F'} paarweise durch Einfach- oder Doppelbindung miteinander verbunden sein können und
wobei ein Kohlenstoffatom oder zwei nicht benachbarte Kohlenstoffatome des Substituenten R^{F} oder R^{F'}, die nicht α-ständig zu dem Heteroatom stehen, durch Atome und/oder Atomgruppierungen, ausgewählt aus der Gruppe -O-, -C(O)-, -S-, -S(O)-, -SO₂-, -N=, -N=N-,-NR'-, -PR'- und -P(O)R'- ersetzt sein können oder eine Endgruppe R'-O-SO₂- oder R'-O-C(O)- besitzen können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und
wobei die Substituenten R¹ jeweils unabhängig voneinander die Bedeutung von
Wasserstoff, für den Fall dass A⁻ = [(CN)₂CR¹]⁻ oder [(R¹O(O)C)₂CR¹]⁻ ist und X = O oder S, oder
Wasserstoff für den Fall dass A⁻ = [R¹CH₂OSO₃]⁻ ist, X = S oder O und die Substitutenten R und R⁰ = Alkylgruppen mit 1 bis 20 C-Atomen sind,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das durch Alkylgruppen mit 1-6 C-Atomen substituiert sein kann,
wobei die Substituenten R¹ teilweise durch CN, NO₂ oder Halogen substituiert sein können und
Halogen F, Cl, Br oder I bedeutet,
wobei die Substituenten R¹ paarweise durch Einfach- oder Doppelbindung miteinander verbunden sein können und
wobei ein Kohlenstoffatom oder zwei nicht benachbarte Kohlenstoffatome des Substituenten R¹, die nicht α-ständig zum Heteroatom stehen, durch Atome und/oder Atomgruppierungen, ausgewählt aus der Gruppe -O-, -C(O)-, -C(O)O-, -S-, -S(O)-, -SO)₂-, -SO₃-, -N=, -N=N-, -NH-, -NR'-, -PR'- und -P(O)R'-, (P(O)R'O-, OP(O)R'O-, -PR'₂=N-, -C(O)NH-, -C(O)NR-, -SO₂NH- oder -SO₂NR'-ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und die Variablen
n 1 bis 20,
m 0, 1, 2 oder 3,
y 0, 1, 2, 3, oder 4,
z 0, 1, 2 oder 3 bedeuten,
mit der Maßgabe, dass
für X = S und R und R⁰ = Alkylgruppen mit 1 bis 20 C-Atomen, die Anionen A⁻ = [BF₄]⁻, CF₃COO⁻ oder [B(C₆H₅)₄]⁻ zuzulassen sind
und
für X = O die Anionen A⁻ = CF₃COO⁻ und [B(C₆H₅)₄]⁻ zuzulassen sind und
für X = O und R⁰ = Ethyl das Anion A⁻ = CH₃CH₂OSO₃⁻ auszuschliessen ist,
als grenzflächenaktiver Stoff.

5. Uronium-Salze der Formel (1), 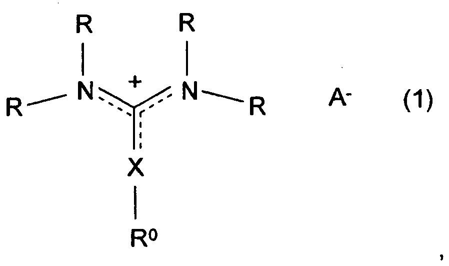 worin
X O bedeutet,
worin die Substituenten R und R⁰ jeweils unabhängig voneinander die Bedeutung von
geradkettiges oder verzweigtes Alkyl mit 1-20 C-Atomen,
geradkettiges oder verzweigtes Alkenyl mit 2-20 C-Atomen und
einer oder mehreren Doppelbindungen,
geradkettiges oder verzweigtes Alkinyl mit 2-20 C-Atomen und
einer oder mehreren Dreifachbindungen
gesättigtes, teilweise oder vollständig ungesättigtes Cycloalkyl mit 3-7 C-Atomen, welches mit Alkylgruppen mit 1-6 C-Atomen substituiert sein kann, haben,
und R auch Wasserstoff bedeuten kann,
wobei ein oder mehrere Substituenten R oder R⁰ teilweise oder vollständig durch Halogen oder teilweise durch CN oder NO₂ substituiert sein können, wobei jedoch die Halogenierung in α-Stellung in R⁰ ausgeschlossen ist, und
Halogen F, Cl, Br oder I bedeutet,
wobei die Substituenten R und R⁰ paarweise durch Einfach- oder Doppelbindung miteinander verbunden sein können und wobei ein Kohlenstoffatom oder zwei nicht benachbarte Kohlenstoffatome eines oder mehrerer Substituenten R oder R⁰, die nicht direkt dem Heteroatom benachbart sind, durch Atome und/oder Atomgruppierungen, ausgewählt aus der Gruppe -O-, -C(O)-, -C(0)0-, - S-, -S(O)-, -SO₂-, -SO₃-, -N=, -N=N-, -NH-, -NR'-, -PR'-, -P(0)R'-, - P(0)R'-0-, -O-P(O)R'-O-, und -P(R')₂=N- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus ist,
und
A⁻ ausgewählt wird aus der Gruppe bestehend aus:
[R¹SO₃]⁻, [R^{F'}SO₃]⁻, [(R^{F}SO₂)₂N]⁻, [(R^{F}SO₂)₃C]⁻, [(FSO₂)₃C]⁻, [R¹CH₂OSO₃]⁻, [R¹C(O)O]⁻, [R^{F'}C(O)O]⁻, [CCl₃C(O)O]⁻, [(CN)₃C]⁻, [(CN)₂CR¹]⁻, [(R¹O(O)C)₂CR¹]⁻, [P(CₙF₂ₙ₊₁-ₘHₘ)_{y}F_{6-y}]⁻, [P(C₆F₅)_{y}F_{6-y}]⁻, [R¹₂P(O)O]⁻, [R¹P(O)O₂]²⁻, [(R¹O)₂P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4-z}]⁻ , [B(CN)₄]⁻, [B(C₆F₅)₄]⁻, [B(OR¹)₄]⁻, [N(CF₃)₂]⁻, [N(CN)₂]⁻, [AlCl₄]⁻[SiF₆]²⁻ , CF₃COO⁻ oder [B(C₆H₅)₄]⁻
wobei die Substituenten R^{F} und R^{F'} jeweils unabhängig voneinander die Bedeutung von perfluoriertes und geradkettiges oder verzweigtes Alkyl mit 1-20 C-Atomen,
wobei für R^{F'} die Trifluormethylgruppe ausgeschlossen ist,
perfluoriertes und geradkettiges oder verzweigtes Alkenyl mit 2-20 C-Atomen und einer oder mehreren Doppelbindungen,
perfluoriertes Phenyl und gesättigtes, teilweise oder vollständig ungesättigtes Cycloalkyl mit 3-7 C-Atomen, das mit Perfluoralkylgruppen substituiert sein kann,
wobei die Substituenten R^{F} oder R^{F'} paarweise durch Einfach- oder
Doppelbindung miteinander verbunden sein können und
wobei ein Kohlenstoffatom oder zwei nicht benachbarte Kohlenstoffatome des Substituenten R^{F} oder R^{F'}, die nicht α-ständig zu dem Heteroatom stehen, durch Atome und/oder Atomgruppierungen, ausgewählt aus der Gruppe -O-, -C(O)-, -S-, -S(O)-, -SO₂-, -N=, -N=N-, - NR'-, -PR'- und -P(O)R'- ersetzt sein können oder eine Endgruppe R'-O-SO₂- oder R'-O-C(O)- besitzen können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und
wobei die Substituenten R¹ jeweils unabhängig voneinander die Bedeutung von
Wasserstoff, für den Fall dass A⁻ = [(CN)₂CR¹]⁻ oder [(R¹O(O)C)₂CR¹]⁻ ist und X = O, oder
Wasserstoff für den Fall dass A⁻ = [R¹CH₂OSO₃]⁻ ist, X = 0 und die Substitutenten R und R⁰ = Alkylgruppen mit 1 bis 20 C-Atomen sind,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das durch Alkylgruppen mit 1-6 C-Atomen substituiert sein kann,
wobei die Substituenten R¹ teilweise durch CN, NO₂ oder Halogen substituiert sein können und
Halogen F, Cl, Br oder I bedeutet,
wobei die Substituenten R¹ paarweise durch Einfach- oder
Doppelbindung miteinander verbunden sein können und
wobei ein Kohlenstoffatom oder zwei nicht benachbarte Kohlenstoffatome des Substituenten R¹, die nicht α-ständig zum Heteroatom stehen, durch Atome und/oder Atomgruppierungen, ausgewählt aus der Gruppe -O-, -C(O)-, -C(O)O-, -S-, -S(O)-, -SO₂-, -SO₃-, -N=, -N=N-, -NH-, -NR'-, -PR'- und -P(O)R'-, (P(O)R'O-, OP(O)R'O-, -PR'₂=N-, -C(O)NH-, -C(O)NR'-, -SO₂NH- oder -SO₂NR'-ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und die Variablen
n 1 bis 20,
m 0, 1, 2 oder 3,
y 1, 2, 3, oder 4,
z 0, 1, 2 oder 3 bedeuten,
mit der Maßgabe, dass
für R⁰ = Ethyl das Anion A⁻ = CH₃CH₂OSO₃⁻ auszuschliessen ist und die Verbindung N,N,N,N-tetramethyldiaminomethoxycarbonium methylsulfat ausgeschlossen ist.

6. Thiouroniumsalze der Formel (1), 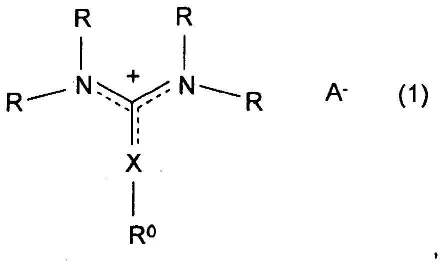 worin
X S bedeutet, worin die Substituenten R und R⁰ jeweils unabhängig voneinander die Bedeutung von
geradkettiges oder verzweigtes Alkyl mit 1-20 C-Atomen,
geradkettiges oder verzweigtes Alkenyl mit 2-20 C-Atomen und
einer oder mehreren Doppelbindungen,
geradkettiges oder verzweigtes Alkinyl mit 2-20 C-Atomen und
einer oder mehreren Dreifachbindungen
gesättigtes, teilweise oder vollständig ungesättigtes Cycloalkyl mit 3-7 C-Atomen, welches mit Alkylgruppen mit 1-6 C-Atomen substituiert sein kann, haben,
und R auch Wasserstoff bedeuten kann,
wobei ein oder mehrere Substituenten R oder R⁰ teilweise oder vollständig durch Halogen oder teilweise durch CN oder NO₂ substituiert sein können, wobei jedoch die Halogenierung in α-Stellung in R⁰ ausgeschlossen ist, und
Halogen F, Cl, Br oder I bedeutet,
wobei die Substituenten R und R⁰ paarweise durch Einfach- oder Doppelbindung miteinander verbunden sein können
und wobei ein Kohlenstoffatom oder zwei nicht benachbarte Kohlenstoffatome eines oder mehrerer Substituenten R oder R⁰, die nicht direkt dem Heteroatom benachbart sind, durch Atome und/oder Atomgruppierungen, ausgewählt aus der Gruppe -O-, -C(O)-, -C(0)0-, - S-, -S(O)-, -SO₂-, -SO₃-, -N=, -N=N-, -NH-, -NR'-, -PR'-, -P(0)R'-, - P(0)R'-0-, -O-P(O)R'-O-, und -P(R')₂=N- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus ist,
und
A⁻ = ausgewählt wird aus der Gruppe bestehend aus:
[R¹SO₃]⁻. [R^{F'}SO₃]⁻, [(R^{F}SO₂)₂N]⁻, [(R^{F}SO₂)₃C]⁻, [(FSO₂)₃C]⁻, [R¹CH₂OSO₃]⁻, [R¹C(O)O]⁻, [R^{F'}C(O)O]⁻, [CCl₃C(O)O]⁻, [(CN)₃C]⁻, [(CN)₂CR¹]⁻, [(R¹O(O)C)₂CR¹]⁻, [P(CₙF₂ₙ₊₁₋ₘHₘ)_{y}F_{6-y}]⁻, [P(C₆F₅)_{y}F_{6-y}]⁻, [R¹₂P(O)O]⁻, [R¹P(O)O₂]²⁻, [(R¹O)₂P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4-z}]⁻ , [B(CN)₄]⁻, [B(C₆F₅)₄]⁻, [B(OR¹)₄]⁻, [N(CF₃)₂]⁻, [N(CN)₂]⁻, [AlCl₄]⁻ oder [SiF₆]²⁻,
wobei die Substituenten R^{F} und R^{F'} jeweils unabhängig voneinander die Bedeutung von
perfluoriertes und geradkettiges oder verzweigtes Alkyl mit 1-20 C-Atomen,
wobei für R^{F'} die Trifluormethylgruppe ausgeschlossen ist,
perfluoriertes und geradkettiges oder verzweigtes Alkenyl mit 2-20 C-Atomen und einer oder mehreren Doppelbindungen,
perfluoriertes Phenyl und gesättigtes, teilweise oder vollständig ungesättigtes Cycloalkyl mit 3-7 C-Atomen, das mit Perfluoralkylgruppen substituiert sein kann,
wobei die Substituenten R^{F} oder R^{F'} paarweise durch Einfach- oder
Doppelbindung miteinander verbunden sein können und
wobei ein Kohlenstoffatom oder zwei nicht benachbarte Kohlenstoffatome des Substituenten R^{F} oder R^{F'}, die nicht α-ständig zu dem Heteroatom stehen, durch Atome und/oder Atomgruppierungen,
ausgewählt aus der Gruppe -O-, -C(O)-, -S-, -S(O)-, -SO₂-, -N=, -N=N-, - NR'-, -PR'- und -P(O)R'- ersetzt sein können oder eine Endgruppe R'-O-SO₂- oder R'-O-C(O)- besitzen können, wobei R' nicht fluoriertes,
teilweise oder perfluoriertes Alkyl mit 1-6 C-Atomen, gesättigtes oder
teilweise ungesättigtes Cycloalkyl mit 3-7 C-Atomen, unsubstituiertes oder substituiertes Phenyl oder unsubstituierter oder substituierter Heterocyclus bedeutet
und
wobei die Substituenten R¹ jeweils unabhängig voneinander die Bedeutung von
Wasserstoff, für den Fall dass A⁻ = [(CN)₂CR¹]⁻ oder [(R¹O(O)C)₂CR¹]⁻ ist oder
Wasserstoff für den Fall dass A⁻ = [R¹CH₂OSO₃]⁻ ist, und die Substitutenten R und R⁰ = Alkylgruppen mit 1 bis 20 C-Atomen sind,
geradkettiges oder verzweigtes Alkyl mit 1-20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das durch Alkylgruppen mit 1-6 C-Atomen substituiert sein kann,
wobei die Substituenten R¹ teilweise durch CN, NO₂ oder Halogen substituiert sein können und
Halogen F, Cl, Br oder I bedeutet,
wobei die Substituenten R¹ paarweise durch Einfach- oder
Doppelbindung miteinander verbunden sein können und
wobei ein Kohlenstoffatom oder zwei nicht benachbarte Kohlenstoffatome des Substituenten R¹, die nicht α-ständig zum Heteroatom stehen, durch Atome und/oder Atomgruppierungen,
ausgewählt aus der Gruppe -O-, -C(O)-, -C(O)O-, -S-, -S(O)-, -SO₂-, -SO₃-, -N=, -N=N-, -NH-, -NR'-, -PR'- und -P(O)R'-, (P(O)R'O-, OP(O)R'O-, -PR'₂=N-, -C(O)NH-, -C(O)NR'-, -SO₂NH- oder -SO₂NR'-ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und die Variablen
n 1 bis 20,
m 0, 1, 2 oder 3,
y 1, 2, 3, oder 4,
z 0, 1, 2 oder 3 bedeuten,
mit der Maßgabe, dass
für R und R⁰ = Alkylgruppen mit 1 bis 20 C-Atomen, die Anionen A⁻ = [BF₄]⁻, CF₃COO⁻ oder [B(C₆H₅)₄]⁻ zuzulassen sind und die Verbindungen
[n-Butyl-SC(NH₂)₂]⁺ EthylOSO₃⁻, 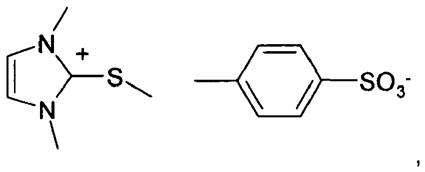 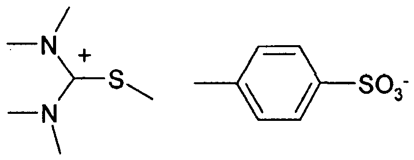 und
2-((1S,2S,5R)-2-isopropyl-5-methylcylohexyl)thiouronium p-toluolsulfonat und
[[(CH₃)₂N]₂CSCH₃]⁺CH₃SO₄⁻
ausgenommen sind.

7. Salz nach Anspruch 5 oder 6, **dadurch gekennzeichnet, dass** ⁷R Wasserstoff und/oder ein gerad kettiges oder verzweigtes Alkyl mit 1 bis 12 C-Atomen ist.

8. Salz nach Anspruch 5 oder 6, **dadurch gekennzeichnet, dass** R ausgewählt ist aus der Gruppe Methyl, Ethyl, n-Propyl, Isopropyl, n-Butyl, tert.-Butyl, sek.-Butyl, Phenyl und Cyclohexyl.

9. Salz nach einem der Ansprüche 6 bis 8, **dadurch gekennzeichnet, dass** R⁰ ein geradkettiges oder verzweigtes Alkyl mit 1 bis 12 C-Atomen bedeutet, das teilweise oder vollständig durch F substituiert sein kann, wobei jedoch eine Fluorierung der α-CH₂-Gruppe von R⁰ ausgeschlossen ist und X = S ist.

10. Salz nach einem der Ansprüche 5 oder 7 bis 8, **dadurch gekennzeichnet, dass** R⁰ ein geradkettiges oder verzweigtes Alkyl mit 1 bis 12 C-Atomen bedeutet und X = O ist.

11. Salz nach einem der Ansprüche 5 oder 6, **dadurch gekennzeichnet, dass** das Anion ausgewählt ist aus der Gruppe [CF₃CF₂SO₃]⁻, [CH₃CH₂SO₃]⁻, [(CF₃SO₂)₂N]⁻, [(C₂F₅SO₂)₂N]⁻, [(CF₃SO₂)₃C]⁻, [(C₂F₅SO₂)₃C]⁻, [CH₃CH₂OSO₃]⁻, [(FSO₂)₃C]⁻, [CF₃C(O)O]⁻, [CF₃CF₂C(O)O]⁻, [CH₃CH₂C(O)O]⁻, [CH₃C(O)O]⁻, [P(C₂F₅)₃F₃]⁻, [P(CF₃)₃F₃]⁻, [P(C₂F₄H)(CF₃)₂F₃]⁻, [P(C₂F₃H₂)₃F₃]⁻, [P(C₂F₅)(CF₃)₂F₃]⁻, [P(C₆F₅)₃F₃]⁻, [P(C₃F₇)₃F₃]⁻, [P(C₄F₉)₃F₃]⁻, [P(C₂F₅)₂F₄]⁻, [(C₂F₅)₂P(O)O]⁻, [(C₂F₅)P(O)O₂]²⁻, [P(C₆F₅)₂F₄]⁻, [(CF₃)₂P(O)O]⁻, [(CH₃)₂P(O)O]⁻, [(C₄F₉)₂P(O)O]⁻, [CF₃P(O)O₂]²⁻, [CH₃P(O)O₂]²⁻, [(CH₃O)₂P(O)O]⁻, [BF₃(CF₃)]⁻, [BF₂(C₂F₅)₂]⁻, [BF₃(C₂F₅)] , [BF₂(CF₃)₂]⁻, [B(C₂F₅)₄]⁻, [BF₃(CN)]⁻, [BF₂(CN)₂]⁻, [B(CN)₄]⁻, [B(CF₃)₄]⁻, [B(OCH₃)₄]⁻, [B(OCH₃)₂(OC₂H₅)]⁻, [B(O₂C₂H₄)₂]⁻, [B(O₂C₂H₂)₂]⁻ , [B(O₂CH₄)₂]⁻, [N(CF₃)₂]⁻, [N(CN₂)₂]⁻, [C(CN)₃]⁻, [AlCl₄]⁻ oder [SiF₆]²⁻, mit der Maßgabe, dass
für X = O die Anionen CF₃COO⁻ oder [B(C₆H₅)₄]⁻ zuzlassen sind,
für X = O und R⁰ = Ethyl das Anion A⁻ = CH₃CH₂OSO₃⁻ auszuschliessen ist und
für X = S und R und R⁰ = Alkylgruppen mit 1 bis 20 C-Atomen, die Anionen A' = [BF₄]⁻, CF₃COO⁻ oder [B(C₆H₅)₄]⁻ zuzulassen sind und die Verbindungen
[n-Butyl-SC(NH₂)₂]⁺ EthylOSO₃⁻, 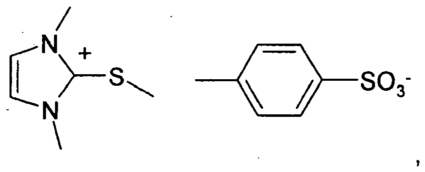 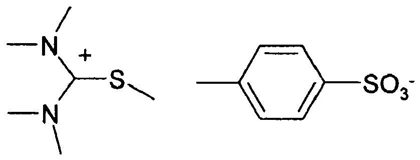 und
2-((1S,2S,5R)-2-isopropyl-5-methylcylohexyl)thiouronium p-toluolsulfonat ausgenommen sind.

12. Verfahren zur Herstellung eines Salzes nach einem der Ansprüche 5 bis 11, **dadurch gekennzeichnet, dass** ein Thioharnstoff C(S)(NR₂)₂ oder ein Harnstoff C(O)(NR₂)₂, wobei die Reste R wie in einem der Ansprüche 5 bis 11 definiert sind, mit einem Ester AR⁰, wobei die Reste R⁰ wie in einem der Ansprüche 5 bis 11 definiert sind und A ausgewählt ist aus der Gruppe [R¹CH₂OSO₃], [R¹SO₃], [R^{F}SO₃], [R^{F}C(O)O] und [CCl₃C(O)O], oder mit einem Oxoniumsalz (R⁰)₃O⁺ A⁻, wobei die Reste R¹ oder R^{F} wie in einem der Ansprüche 5 bis 11 definiert sind und A⁻ ausgewählt ist aus der Gruppe [(FSO₂)₃C]⁻ und [BF₄]⁻, alkyliert wird, unter Beachtung der Maßgabe, dass
für X = O die Anionen CF₃COO⁻ oder [B(C₆H₅)₄]⁻ zuzlassen sind,
für X = O und R⁰ = Ethyl das Anion A- = CH₃CH₂OSO₃⁻ auszuschliessen ist und
für X = S und R und R⁰ = Alkylgruppen mit 1 bis 20 C-Atomen, die Anionen A⁻ = [BF₄]⁻, CF₃COO⁻ oder [B(C₆H₅)₄]⁻ zuzulassen sind und die Verbindungen
[n-Butyl-SC(NH₂)₂]⁺ EthylOSO₃⁻, 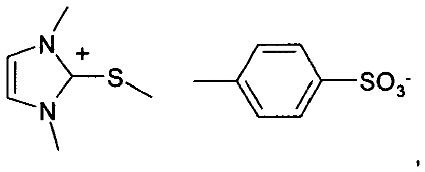 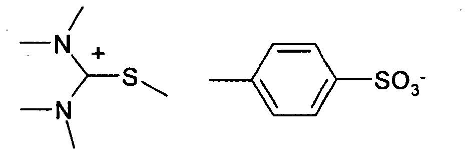 und
2-((1S,2S,5R)-2-isopropyl-5-methylcylohexyl)thiouronium p-toluolsulfonat,
N,N,N,N-tetramethyldiaminomethoxycarbonium methylsulfat, [[(CH₃)₂N]₂CSCH₃]⁺CH₃SO₄⁻
ausgenommen sind

13. Verfahren nach Anspruch 12, **dadurch gekennzeichnet, dass** die Umsetzung bei einer Temperatur durchgeführt wird, bei der mindestens eine der Komponenten flüssig ist.

14. Verfahren zur Herstellung eines Salzes der Formel (1) nach einem der Ansprüche 5 bis 11, **dadurch gekennzeichnet, dass** ein Salz mit einem Kation der allgemeinen Formel (1) nach einem der Ansprüche 5 bis 11 und einem Anion A⁻ ausgewählt aus der Gruppe [BF₄]⁻, Br⁻, Cl⁻, I⁻ oder [ClO₄]⁻, mit einem Salz Kt⁺ A⁻ oder mit einer Säure AH, wobei Kt ein Alkali- oder Erdalkalimetall und A wie in einem der Ansprüche 5 bis 11 definiert ist, umgesetzt wird.

## Claims

1. Use of a salt of the formula (1), 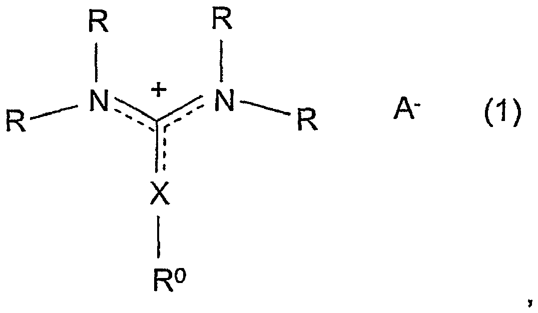 in which
X denotes O or S,
in which the substituents R and R⁰ each, independently of one another, have the meaning of
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
and R may also denote hydrogen,
where one or more substituents R or R⁰ may be partially or fully substituted by halogen or partially by CN or NO₂, but where halogenation in the α-position in R⁰ is excluded, and
halogen denotes F, Cl, Br or I,
where the substituents R and R⁰ may be connected to one another in pairs by a single or double bond,
and where one carbon atom or two non-adjacent carbon atoms of one or more substituents R or R⁰ which are not directly adjacent to the heteroatom may be replaced by atoms and/or atom groups selected from the group -0-, -C(O)-, -C(O)O-, -S-, -S(O)-, -SO₂-, -SO₃-, -N=, -N=N-, -NH-, -NR'-, -PR'-, -P(O)R'-, -P(O)R'-O-, -O-P(O)R'-O- and -P(R')₂=N-, where R' is unfluorinated, partially or perfluorinated alkyl having 1-6 C atoms, saturated or partially unsaturated cycloalkyl having 3-7 C atoms, unsubstituted or substituted phenyl or an unsubstituted or substituted heterocycle,
and
A- is selected from the group consisting of:
[R¹SO₃]⁻, [R^{F'}SO₃]⁻, [(R^{F}SO₂)₂N]⁻, [(R^{F}SO₂)₃C]⁻, [(FSO₂)₃C]⁻, [R¹CH₂OSO₃]⁻, [R¹C(O)O]⁻, [R^{F'}C(O)O]⁻, [CCl₃C(O)O]⁻, [(CN)₃C]⁻, [(CN)₂CR¹]⁻, [(R¹O(O)C)₂CR¹]⁻, [P(CₙF₂ₙ₊₁₋ₘHₘ)_{y}F_{6-y}]⁻, [P(C₆F₅)_{y}F_{6-y}]⁻, [R¹₂P(O)O]⁻, [R¹P(O)O₂]²⁻, [(R¹O)₂P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4-z}]⁻, [B(CN)₄]⁻, [B(C₆F₅)₄]⁻, [B(OR¹)₄]⁻, [N(CF₃)₂]⁻, [N(CN)₂]⁻, [AlCl₄]⁻ or [SiF₆]²⁻,
where the substituents R^{F} and R^{F'} each, independently of one another, have the meaning of
perfluorinated and straight-chain or branched alkyl having 1-20 C atoms,
where the trifluoromethyl group is excluded for R^{F'},
perfluorinated and straight-chain or branched alkenyl having 2-20 C atoms and one or more double bonds,
perfluorinated phenyl and saturated, partially or fully unsaturated cycloalkyl having 3-7 C atoms, which may be substituted by perfluoroalkyl groups,
where the substituents R^{F} or R^{F'} may be connected to one another in pairs by a single or double bond and
where one carbon atom or two non-adjacent carbon atoms of the substituent R^{F} or R^{F'} which are not in the α-position to the heteroatom may be replaced by atoms and/or atom groups selected from the group -O-, -C(O)-, -S-, -S(O)-, -SO₂-, -N=, -N=N-, -NR'-, -PR'- and -P(O)R'- or may have an end group R'-O-SO₂- or R'-O-C(O)-, where R' denotes unfluorinated, partially or perfluorinated alkyl having 1-6 C atoms, saturated or partially unsaturated cycloalkyl having 3-7 C atoms, unsubstituted or substituted phenyl or an unsubstituted or substituted heterocycle and
where the substituents R¹ each, independently of one another, have the meaning of
hydrogen in the case where A⁻ = [(CN)₂CR¹]⁻ or [(R¹O(O)C)₂CR¹]⁻ and X = O or S, or
hydrogen in the case where A⁻ = [R¹CH₂OSO₃]⁻, X = S or O and the substituents R and R⁰ = alkyl groups having 1 to 20 C atoms,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms,
which may be substituted by alkyl groups having 1-6 C atoms,
where the substituents R¹ may be partially substituted by CN, NO₂ or halogen and
halogen denotes F, Cl, Br or I,
where the substituents R¹ may be connected to one another in pairs by a single or double bond and
where one carbon atom or two non-adjacent carbon atoms of the substituent R¹ which are not in the α-position to the heteroatom may be replaced by atoms and/or atom groups selected from the group -O-, -C(O)-, -C(O)O-, -S-, -S(O)-, -SO₂-, -SO₃-, -N=, -N=N-, -NH-, -NR'-, -PR'- and -P(O)R'-, -P(O)R'O-, -OP(O)R'O-, -PR'₂=N-, -C(O)NH-, -C(O)NR'-, -SO₂NH- or -SO₂NR'-, where R' denotes unfluorinated, partially or perfluorinated alkyl having 1-6 C atoms, saturated or partially unsaturated cycloalkyl having 3-7 C atoms, unsubstituted or substituted phenyl or an unsubstituted or substituted heterocycle and the variables
n denotes 1 to 20,
m denotes 0, 1, 2 or 3,
y denotes 0, 1, 2, 3 or 4,
z denotes 0, 1, 2 or 3,
with the proviso that
for X = S and R and R⁰ = alkyl groups having 1 to 20 C atoms, the anions A⁻ = [BF₄]⁻, CF₃COO⁻ or [B(C₆H₅)₄]⁻ are to be allowed and
for X = O, the anions A⁻ = CF₃COO⁻ and [B(C₆H₅)₄]⁻ are to be allowed and
for X = O and R⁰ = ethyl, the anion A⁻ = CH₃CH₂OSO₃⁻ is to be excluded,
as ionic liquid.

2. Use of a salt of the formula (1), 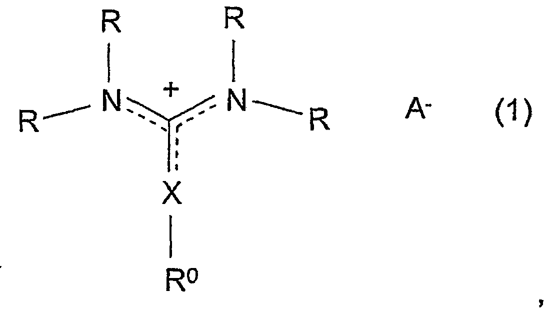 in which
X denotes O or S,
in which the substituents R and R⁰ each, independently of one another, have the meaning of
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
and R may also denote hydrogen,
where one or more substituents R or R⁰ may be partially or fully substituted by halogen or partially by CN or NO₂, but where halogenation in the α-position in R⁰ is excluded, and
halogen denotes F, Cl, Br or I,
where the substituents R and R⁰ may be connected to one another in pairs by a single or double bond,
and where one carbon atom or two non-adjacent carbon atoms of one or more substituents R or R⁰ which are not directly adjacent to the heteroatom may be replaced by atoms and/or atom groups selected from the group -0-, -C(O)-, -C(O)O-, -S-, -S(O)-, -SO₂-, -SO₃-, -N=, -N=N-, -NH-, -NR'-, -PR'-, -P(O)R'-, -P(O)R'-O-, -O-P(O)R'-O- and -P(R')₂=N-, where R' is unfluorinated, partially or perfluorinated alkyl having 1-6 C atoms, saturated or partially unsaturated cycloalkyl having 3-7 C atoms, unsubstituted or substituted phenyl or an unsubstituted or substituted heterocycle,
and
A⁻ is selected from the group consisting of:
[R¹SO₃]⁻, [R^{F'}SO₃]⁻, [(R^{F}SO₂)₂N]⁻, [(R^{F}SO₂)₃C]⁻, [(FSO₂)₃C]⁻, [R¹CH₂OSO₃]⁻, [R¹C(O)O]⁻, [R^{F'}C(O)O]⁻, [CCl₃C(O)O]⁻, [(CN)₃C]⁻, [(CN)₂CR¹]⁻, [(R¹O(O)C)₂CR¹]⁻, [P(CₙF₂ₙ₊₁₋ₘHₘ)_{y}F_{6-y}]⁻, [P(C₆F₅)_{y}F_{6-y}]⁻, [R¹₂P(O)O]⁻, [R¹P(O)O₂]²⁻, [(R¹O)₂P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4-z}]⁻, [B(CN)₄]⁻, [B(C₆F₅)₄]⁻, [B(OR¹)₄]⁻, [N(CF₃)₂]⁻, [N(CN)₂]⁻, [AlCl₄]⁻ or [SiF₆]²⁻,
where the substituents R^{F} and R^{F'} each, independently of one another, have the meaning of .
perfluorinated and straight-chain or branched alkyl having 1-20 C atoms,
where the trifluoromethyl group is excluded for R^{F'},
perfluorinated and straight-chain or branched alkenyl having 2-20 C atoms and one or more double bonds,
perfluorinated phenyl and saturated, partially or fully unsaturated cycloalkyl having 3-7 C atoms, which may be substituted by perfluoroalkyl groups,
where the substituents R^{F} or R^{F'} may be connected to one another in pairs by a single or double bond and
where one carbon atom or two non-adjacent carbon atoms of the substituent R^{F} or R^{F'} which are not in the α-position to the heteroatom may be replaced by atoms and/or atom groups selected from the group -O-, -C(O)-, -S-, -S(O)-, -SO₂-, -N=, -N=N-, -NR'-, -PR'- and -P(O)R'- or may have an end group R'-O-SO₂- or R'-O-C(O)-, where R' denotes unfluorinated, partially or perfluorinated alkyl having 1-6 C atoms, saturated or partially unsaturated cycloalkyl having 3-7 C atoms, unsubstituted or substituted phenyl or an unsubstituted or substituted heterocycle and
where the substituents R¹ each, independently of one another, have the meaning of
hydrogen in the case where A⁻ = [(CN)₂CR¹]⁻ or [(R¹O(O)C)₂CR¹]⁻ and X = O or S, or
hydrogen in the case where A⁻ = [R¹CH₂OSO₃]⁻, X = S or O and the substituents R and R⁰ = alkyl groups having 1 to 20 C atoms,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where the substituents R¹ may be partially substituted by CN, NO₂ or halogen and
halogen denotes F, Cl, Br or I,
where the substituents R¹ may be connected to one another in pairs by a single or double bond and
where one carbon atom or two non-adjacent carbon atoms of the substituent R¹ which are not in the α-position to the heteroatom may be replaced by atoms and/or atom groups selected from the group -O-, -C(O)-, -C(O)O-, -S-, -S(O)-, -SO₂-, -SO₃-, -N=, -N=N-, -NH-, -NR'-, -PR'- and -P(O)R'-, -P(O)R'O-, -OP(O)R'O-, -PR'₂=N-, -C(O)NH-, -C(O)NR'-, -SO₂NH- or -SO₂NR'-, where R' denotes unfluorinated, partially or perfluorinated alkyl having 1-6 C atoms, saturated or partially unsaturated cycloalkyl having 3-7 C atoms, unsubstituted or substituted phenyl or an unsubstituted or substituted heterocycle
and the variables
n denotes 1 to 20,
m denotes 0, 1, 2 or 3,
y denotes 0, 1, 2, 3 or 4,
z denotes 0, 1, 2 or 3,
with the proviso that
for X = S and R and R⁰ = alkyl groups having 1 to 20 C atoms, the anions A⁻ = [BF₄]⁻, CF₃COO⁻ or [B(C₆H₅)₄]⁻ are to be allowed
and
for X = O, the anions A⁻ = CF₃COO⁻ and [B(C₆H₅)₄]⁻ are to be allowed and
for X = O and R⁰ = ethyl, the anion A⁻ = CH₃CH₂OSO₃⁻ is to be excluded,
as non-aqueous electrolyte.

3. Use of a salt of the formula (1), 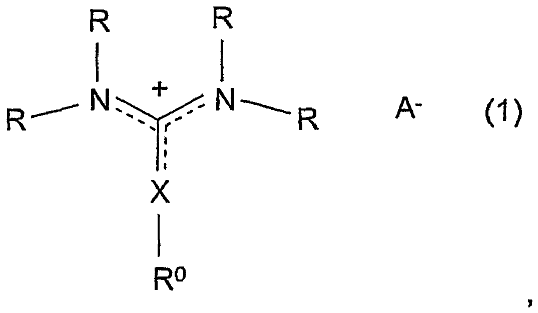 in which
X denotes O or S,
in which the substituents R and R⁰ each, independently of one another, have the meaning of
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
and R may also denote hydrogen,
where one or more substituents R or R⁰ may be partially or fully substituted by halogen or partially by CN or NO₂, but where halogenation in the α-position in R⁰ is excluded, and
halogen denotes F, Cl, Br or I,
where the substituents R and R⁰ may be connected to one another in pairs by a single or double bond,
and where one carbon atom or two non-adjacent carbon atoms of one or more substituents R or R⁰ which are not directly adjacent to the heteroatom may be replaced by atoms and/or atom groups selected from the group -O-,-C(O)-, -C(O)O-, -S-, -S(O)-, -SO₂-, -SO₃-, -N=, -N=N-, -NH-, -NR'-, -PR'-, -P(O)R'-, -P(O)R'-O-, -O-P(O)R'-O- and -P(R')₂=N-, where R' is unfluorinated, partially or perfluorinated alkyl having 1-6 C atoms, saturated or partially unsaturated cycloalkyl having 3-7 C atoms, unsubstituted or substituted phenyl or an unsubstituted or substituted heterocycle,
and
A⁻ is selected from the group consisting of:
[R¹SO₃]⁻, [R^{F'}SO₃]⁻, [(R^{F}SO₂)₂N]⁻, [(R^{F}SO₂)₃C]⁻, [(FSO₂)₃C]⁻, [R¹CH₂OSO₃]⁻, [R¹C(O)O]⁻, [R^{F'}C(O)O]⁻, [CCl₃C(O)O]⁻, [(CN)₃C]⁻, [(CN)₂CR¹]⁻, [(R¹O(O)C)₂CR¹]⁻, [P(CnF₂ₙ₊₁₋ₘHₘ)_{y}F_{6-y}]⁻, [P(C₆F₅)_{y}F_{6-y}]⁻, [R¹₂P(O)O]⁻, [R¹P(O)O₂]²⁻, [(R¹O)₂P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4**-**z}]⁻, [B(CN)₄]⁻, [B(C₆F₅)₄]⁻, [B(OR¹)₄]⁻, [N(CF₃)₂]⁻, [N(CN)₂]⁻, [AlCl₄]⁻ or [SiF₆]²⁻,
where the substituents R^{F} and R^{F'} each, independently of one another, have the meaning of
perfluorinated and straight-chain or branched alkyl having 1-20 C atoms,
where the trifluoromethyl group is excluded for R^{F'},
perfluorinated and straight-chain or branched alkenyl having 2-20 C atoms and one or more double bonds,
perfluorinated phenyl and saturated, partially or fully unsaturated cyclo-alkyl having 3-7 C atoms, which may be substituted by perfluoroalkyl groups,
where the substituents R^{F} or R^{F'} may be connected to one another in pairs by a single or double bond and
where one carbon atom or two non-adjacent carbon atoms of the substituent R^{F} or R^{F'} which are not in the α-position to the heteroatom may be replaced by atoms and/or atom groups selected from the group -0-, -C(O)-, -S-, -S(O)-, -SO₂-, -N=, -N=N-, -NR'-, -PR'- and -P(O)R'- or may have an end group R'-O-SO₂- or R'-O-C(O)-, where R' denotes unfluorinated, partially or perfluorinated alkyl having 1-6 C atoms, saturated or partially unsaturated cycloalkyl having 3-7 C atoms, unsubstituted or substituted phenyl or an unsubstituted or substituted heterocycle and
where the substituents R¹ each, independently of one another, have the meaning of
hydrogen in the case where A⁻ = [(CN)₂CR¹]⁻ or [(R¹O(O)C)₂CR¹]⁻ and X = O or S, or
hydrogen in the case where A⁻ = [R¹CH₂OSO₃]⁻, X = S or O and the substituents R and R⁰ = alkyl groups having 1 to 20 C atoms, straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where the substituents R¹ may be partially substituted by CN, NO₂ or halogen and
halogen denotes F, Cl, Br or I,
where the substituents R¹ may be connected to one another in pairs by a single or double bond and
where one carbon atom or two non-adjacent carbon atoms of the substituent R¹ which are not in the α-position to the heteroatom may be replaced by atoms and/or atom groups selected from the group -O-, -C(O)-, -C(0)0-, -S-, -S(O)-, -SO₂-, -SO₃-, -N=, -N=N-, -NH-, -NR'-, -PR'- and -P(O)R'-, -P(O)R'O-, -OP(O)R'O-, -PR'₂=N-, -C(O)NH-, -C(O)NR'-, -SO₂NH- or -SO₂NR'-, where R' denotes unfluorinated, partially or perfluorinated alkyl having 1-6 C atoms, saturated or partially unsaturated cycloalkyl having 3-7 C atoms, unsubstituted or substituted phenyl or an unsubstituted or substituted heterocycle
and the variables
n denotes 1 to 20,
m denotes 0, 1, 2 or 3,
y denotes 0, 1, 2, 3 or 4,
z denotes 0, 1, 2 or 3,
with the proviso that
for X = S and R and R⁰ = alkyl groups having 1 to 20 C atoms, the anions A⁻ = [BF₄]⁻, CF₃COO⁻ or [B(C₆H₅)₄]⁻ are to be allowed and
for X = O, the anions A⁻ = CF₃COO⁻ and [B(C₆H₅)₄]⁻ are to be allowed and
for X = O and R⁰ = ethyl, the anion A⁻ = CH₃CH₂OSO₃⁻ is to be excluded,
as phase-transfer catalyst.

4. Use of a salt of the formula (1), 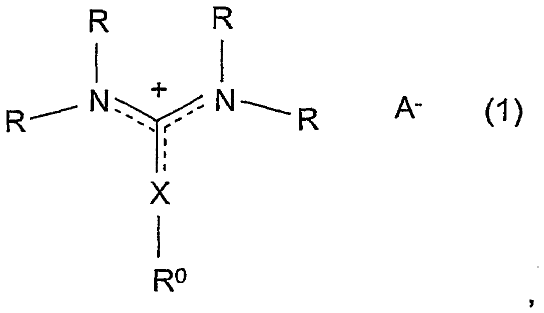 in which
X denotes O or S,
in which the substituents R and R⁰ each, independently of one another, have the meaning of
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
and R may also denote hydrogen,
where one or more substituents R or R⁰ may be partially or fully substituted by halogen or partially by CN or NO₂, but where halogenation in the α-position in R⁰ is excluded, and
halogen denotes F, Cl, Br or I,
where the substituents R and R⁰ may be connected to one another in pairs by a single or double bond,
and where one carbon atom or two non-adjacent carbon atoms of one or more substituents R or R⁰ which are not directly adjacent to the heteroatom may be replaced by atoms and/or atom groups selected from the group -0-, -C(O)-, -C(O)O-, -S-, -S(O)-, -SO₂-, -SO₃-, -N=, -N=N-, -NH-, -NR'-, -PR'- and -P(O)R'-, -P(O)R'-O-, -O-P(O)R'-O- and -P(R')₂=N-, where R' is unfluorinated, partially or perfluorinated alkyl having 1-6 C atoms, saturated or partially unsaturated cycloalkyl having 3-7 C atoms, unsubstituted or substituted phenyl or an unsubstituted or substituted heterocycle,
and
A⁻ is selected from the group consisting of:
[R¹SO₃]⁻, [R^{F'}SO₃]⁻, [(R^{F}SO₂)₂N]⁻, [(R^{F}SO₂)₃C]⁻, [(FSO₂)₃C]⁻, [R¹CH₂OSO₃]⁻, [R¹C(O)O]⁻, [R^{F'}C(O)O]⁻, [CCl₃C(O)O]⁻, [(CN)₃C]⁻, [(CN)₂CR¹]⁻, [(R¹O(O)C)₂CR¹]⁻, [P(CₙF₂ₙ₊₁₋ₘHₘ)_{y}F_{6-y}]⁻, [P(C₆F₅)_{y}F_{6-y}]⁻, [R¹₂P(O)O]⁻, [R¹P(O)O₂]²⁻, [(R¹O)₂P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4-z}]⁻, [B(CN)₄]⁻, [B(C₆F₅)₄]⁻, [B(OR¹)₄]⁻, [N(CF₃)₂]⁻, [N(CN)₂]⁻, [AlCl₄]⁻ or [SiF₆]²⁻,
where the substituents R^{F} and R^{F'} each, independently of one another, have the meaning of
perfluorinated and straight-chain or branched alkyl having 1-20 C atoms,
where the trifluoromethyl group is excluded for R^{F'},
perfluorinated and straight-chain or branched alkenyl having 2-20 C atoms and one or more double bonds,
perfluorinated phenyl and saturated, partially or fully unsaturated cycloalkyl having 3-7 C atoms, which may be substituted by perfluoroalkyl groups,
where the substituents R^{F} or R^{F'} may be connected to one another in pairs by a single or double bond and
where one carbon atom or two non-adjacent carbon atoms of the substituent R^{F} or R^{F'} which are not in the α-position to the heteroatom may be replaced by atoms and/or atom groups selected from the group -0-, -C(O)-, -S-, -S(O)-, -SO₂-, -N=, -N=N-, -NR'-, -PR'- and -P(O)R'- or may have an end group R'-O-SO₂- or R'-O-C(O)-, where R' denotes unfluorinated, partially or perfluorinated alkyl having 1-6 C atoms, saturated or partially unsaturated cycloalkyl having 3-7 C atoms, unsubstituted or substituted phenyl or an unsubstituted or substituted heterocycle
and
where the substituents R¹ each, independently of one another, have the meaning of
hydrogen in the case where A⁻ = [(CN)₂CR¹]⁻ or [(R¹O(O)C)₂CR¹]⁻ and X = O or S, or
hydrogen in the case where A⁻ = [R¹CH₂OSO₃]⁻, X = S or O and the substituents R and R⁰ = alkyl groups having 1 to 20 C atoms,
straight-chain or branched alkyl having 1-20 C atoms,
straight-chain or branched alkenyl having 2-20 C atoms and one or
more double bonds,
straight-chain or branched alkynyl having 2-20 C atoms and one or more triple bonds,
saturated, partially or fully unsaturated cycloalkyl having 3-7 C atoms,
which may be substituted by alkyl groups having 1-6 C atoms,
where the substituents R¹ may be partially substituted by CN, NO₂ or halogen and
halogen denotes F, Cl, Br or I,
where the substituents R¹ may be connected to one another in pairs by a single or double bond and
where one carbon atom or two non-adjacent carbon atoms of the substituent R¹ which are not in the α-position to the heteroatom may be replaced by atoms and/or atom groups selected from the group -O-, -C(O)-, -C(0)0-, -S-, -S(O)-, -SO₂-, -SO₃-, -N=, -N=N-, -NH-, -NR'-, -PR'- and -P(O)R'-, -P(O)R'O-, -OP(O)R'O-, -PR'₂=N-, -C(O)NH-, -C(O)NR'-, -SO₂NH- or -SO₂NR'-, where R' denotes unfluorinated, partially or perfluorinated alkyl having 1-6 C atoms, saturated or partially unsaturated cycloalkyl having 3-7 C atoms, unsubstituted or substituted phenyl or an unsubstituted or substituted heterocycle
and the variables
n denotes 1 to 20,
m denotes 0, 1, 2 or 3,
y denotes 0, 1, 2, 3 or 4,
z denotes 0, 1, 2 or 3,
with the proviso that
for X = S and R and R⁰ = alkyl groups having 1 to 20 C atoms, the anions A⁻ = [BF₄]⁻, CF₃COO⁻ or [B(C₆H₅)₄]⁻ are to be allowed and
for X = O, the anions A⁻ = CF₃COO⁻ and [B(C₆H₅)₄]⁻ are to be allowed and
for X = O and R⁰ = ethyl, the anion A⁻ = CH₃CH₂OSO₃⁻ is to be excluded,
as surface-active substance.

5. Uronium salts of the formula (1) 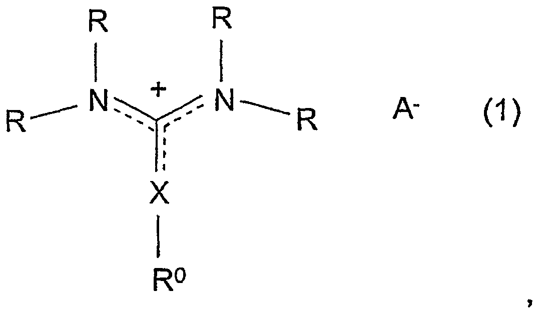 in which
X denotes O,
in which the substituents R and R⁰ each, independently of one another, have the meaning of
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
and R may also denote hydrogen,
where one or more substituents R or R⁰ may be partially or fully substituted by halogen or partially by CN or NO₂, but where halogenation in the α-position in R⁰ is excluded, and
halogen denotes F, Cl, Br or I,
where the substituents R and R⁰ may be connected to one another in pairs by a single or double bond,
and where one carbon atom or two non-adjacent carbon atoms of one or more substituents R or R⁰ which are not directly adjacent to the heteroatom may be replaced by atoms and/or atom groups selected from the group -0-, -C(O)-, -C(O)O-, -S-, -S(O)-, -SO₂-, -SO₃-, -N=, -N=N-, -NH-, -NR'-, -PR'-, -P(O)R'-, -P(O)R'-O-, -O-P(O)R'-O- and -P(R')₂=N-, where R' is unfluorinated, partially or perfluorinated alkyl having 1-6 C atoms, saturated or partially unsaturated cycloalkyl having 3-7 C atoms, unsubstituted or substituted phenyl or an unsubstituted or substituted heterocycle,
and
A⁻ is selected from the group consisting of:
[R¹SO₃]⁻, [R^{F'}SO₃]⁻, [(R^{F}SO₂)₂N]⁻, [(R^{F}SO₂)₃C]⁻, [(FSO₂)₃C]⁻, [R¹CH₂OSO₃]⁻, [R¹C(O)O]⁻, [R^{F'}C(O)O]⁻, [CCl₃C(O)O]⁻, [(CN)₃C]⁻, [(CN)₂CR¹]⁻, [(R¹O(O)C)₂CR¹]⁻, [P(CₙF₂ₙ₊₁₋ₘHₘ)_{y}F_{6-y}]⁻, [P(C₆F₅)_{y}F_{6-y}]⁻, [R¹₂P(O)O]⁻, [R¹P(O)O₂]²⁻, [(R¹O)₂P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4-z}]⁻, [B(CN)₄]⁻, B(C₆F₅)₄]⁻, [B(OR¹)₄]⁻, [N(CF₃)₂]⁻, [N(CN)₂]⁻, [AlCl₄]⁻, [SiF₆]²⁻, CF₃COO⁻ or [B(C₆H₅)₄]⁻,
where the substituents R^{F} and R^{F'} each, independently of one another, have the meaning of
perfluorinated and straight-chain or branched alkyl having 1-20 C atoms,
where the trifluoromethyl group is excluded for R^{F'},
perfluorinated and straight-chain or branched alkenyl having 2-20 C atoms and one or more double bonds,
perfluorinated phenyl and saturated, partially or fully unsaturated cycloalkyl having 3-7 C atoms, which may be substituted by perfluoroalkyl groups,
where the substituents R^{F} or R^{F'} may be connected to one another in pairs by a single or double bond and
where one carbon atom or two non-adjacent carbon atoms of the substituent R^{F} or R^{F'} which are not in the α-position to the heteroatom may be replaced by atoms and/or atom groups selected from the group -0-, -C(O)-, -S-, -S(O)-, -SO₂-, -N=, -N=N-, -NR'-, -PR'- and -P(O)R'- or may have an end group R'-O-SO₂- or R'-O-C(O)-, where R' denotes unfluorinated, partially or perfluorinated alkyl having 1-6 C atoms, saturated or partially unsaturated cycloalkyl having 3-7 C atoms, unsubstituted or substituted phenyl or an unsubstituted or substituted heterocycle
and
where the substituents R¹ each, independently of one another, have the meaning of
hydrogen in the case where A⁻ = [(CN)₂CR¹]⁻ or [(R¹O(O)C)₂CR¹]⁻ and X = O, or
hydrogen in the case where A⁻ = [R¹CH₂OSO₃]⁻, X = O and the substituents R and R⁰ = alkyl groups having 1 to 20 C atoms,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where the substituents R¹ may be partially substituted by CN, NO₂ or halogen and
halogen denotes F, Cl, Br or I,
where the substituents R¹ may be connected to one another in pairs by a single or double bond and
where one carbon atom or two non-adjacent carbon atoms of the substituent R¹ which are not in the α-position to the heteroatom may be replaced by atoms and/or atom groups selected from the group -O-, -C(O)-, -C(O)O-, -S-, -S(O)-, -SO₂-, -SO₃-, -N=, -N=N-, -NH-, -NR'-, -PR'- and -P(O)R'-, -P(O)R'O-, -OP(O)R'O-, -PR'₂=N-, -C(O)NH-, -C(O)NR'-, -SO₂NH- or -SO₂NR'-, where R' denotes unfluorinated, partially or perfluorinated alkyl having 1-6 C atoms, saturated or partially unsaturated cycloalkyl having 3-7 C atoms, unsubstituted or substituted phenyl or an unsubstituted or substituted heterocycle
and the variables
n denotes 1 to 20,
m denotes 0, 1, 2 or 3,
y denotes 1, 2, 3 or 4,
z denotes 0, 1, 2 or 3,
with the proviso that
for R⁰ = ethyl, the anion A⁻ = CH₃CH₂OSO₃⁻ is to be excluded and the compound N,N,N,N-tetramethyldiaminomethoxycarbonium methylsulfate is excluded.

6. Thiouronium salts of the formula (1), 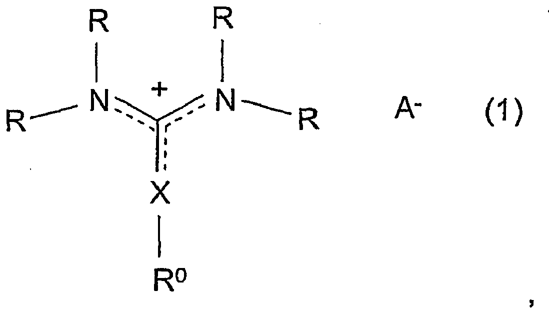 in which
X denotes S,
in which the substituents R and R⁰ each, independently of one another, have the meaning of
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
and R may also denote hydrogen,
where one or more substituents R or R⁰ may be partially or fully substituted by halogen or partially by CN or NO₂, but where halogenation in the α-position in R⁰ is excluded, and
halogen denotes F, Cl, Br or I,
where the substituents R and R⁰ may be connected to one another in pairs by a single or double bond,
and where one carbon atom or two non-adjacent carbon atoms of one or more substituents R or R⁰ which are not directly adjacent to the heteroatom may be replaced by atoms and/or atom groups selected from the group -O-,-C(O)-, -C(O)O-, -S-, -S(O)-, -SO₂-, -SO₃-, -N=, -N=N-, -NH-, -NR'-, -PR'-, -P(O)R'-, -P(O)R'-O-, -O-P(O)R'-O- and -P(R')₂=N-, where R' is unfluorinated, partially or perfluorinated alkyl having 1-6 C atoms, saturated or partially unsaturated cycloalkyl having 3-7 C atoms, unsubstituted or substituted phenyl or an unsubstituted or substituted heterocycle,
and
A⁻ is selected from the group consisting of:
[R¹SO₃]⁻, [R^{F'}SO₃]⁻, [(R^{F}SO₂)₂N]⁻, [(R^{F}SO₂)₃C]⁻, [(FSO₂)₃C]⁻, [R¹CH₂OSO₃]⁻, [R¹C(O)O]⁻, [R^{F'}C(O)O]⁻, [CCl₃C(O)O]⁻, [(CN)₃C]⁻, [(CN)₂CR¹]⁻, [(R¹O(O)C)₂CR¹]⁻, [P(CₙF₂ₙ₊₁₋ₘHₘ)_{y}F_{6-y}]⁻, [P(C₆F₅)_{y}F_{6-y}]⁻, [R¹₂P(O)O]⁻, [R¹P(O)O₂]²⁻, [(R¹O)₂P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4-z}]⁻, [B(CN)₄]⁻, B(C₆F₅)₄]⁻, [B(OR¹)₄]⁻, [N(CF₃)₂]⁻, [N(CN)₂]⁻, [AlCl₄]⁻ or [SiF₆]²⁻,
where the substituents R^{F} and R^{F'} each, independently of one another, the meaning of
perfluorinated and straight-chain or branched alkyl having 1-20 C atoms, where the trifluoromethyl group is excluded for R^{F'}, perfluorinated and straight-chain or branched alkenyl having 2-20 C atoms and one or more double bonds,
perfluorinated phenyl and saturated, partially or fully unsaturated cycloalkyl having 3-7 C atoms, which may be substituted by perfluoroalkyl groups,
where the substituents R^{F} or R^{F'} may be connected to one another in pairs by a single or double bond and
where one carbon atom or two non-adjacent carbon atoms of the substituent R^{F} or R^{F'} which are not in the α-position to the heteroatom may be replaced by atoms and/or atom groups selected from the group -O-, -C(O)-, -S-, -S(O)-, -SO₂-, -N=, -N=N-, -NR'-, -PR'- and -P(O)R'- or may have an end group R'-O-SO₂- or R'-O-C(O)-, where R' denotes unfluorinated, partially or perfluorinated alkyl having 1-6 C atoms, saturated or partially unsaturated cycloalkyl having 3-7 C atoms, unsubstituted or substituted phenyl or an unsubstituted or substituted heterocycle and
where the substituents R¹ each, independently of one another, have the meaning of
hydrogen in the case where A⁻ = [(CN)₂CR¹]⁻ or [(R¹O(O)C)₂CR¹]⁻, or hydrogen in the case where A⁻ = [R¹CH₂OSO₃]⁻ and the substituents R and R⁰ = alkyl groups having 1 to 20 C atoms,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where the substituents R¹ may be partially substituted by CN, NO₂ or halogen and
halogen denotes F, Cl, Br or I,
where the substituents R¹ may be connected to one another in pairs by a single or double bond and
where one carbon atom or two non-adjacent carbon atoms of the substituent R¹ which are not in the α-position to the heteroatom may be replaced by atoms and/or atom groups selected from the group -0-, -C(O)-, -C(0)0-, -S-, -S(O)-, -SO₂-, -SO₃-, -N=, -N=N-, -NH-, -NR'-, -PR'- and -P(O)R'-, -P(O)R'O-, -OP(O)R'O-, -PR'₂=N-, -C(O)NH-, -C(O)NR'-, -SO₂NH- or -SO₂NR'-, where R' denotes unfluorinated, partially or perfluorinated alkyl having 1-6 C atoms, saturated or partially unsaturated cycloalkyl having 3-7 C atoms, unsubstituted or substituted phenyl or an unsubstituted or substituted heterocycle
and the variables
n denotes 1 to 20,
m denotes 0, 1, 2 or 3,
y denotes 1, 2, 3 or 4,
z denotes 0, 1, 2 or 3,
with the proviso that
for R and R⁰ = alkyl groups having 1 to 20 C atoms, the anions A⁻ = [BF₄]⁻, CF₃COO⁻ or [B(C₆H₅)₄]⁻ are to be allowed
and the compounds
[n-butyl-SC(NH₂)₂]⁺ ethylOSO₃⁻, 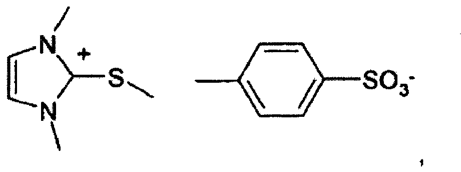 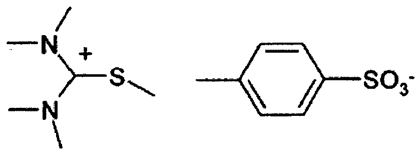 and
2-((1S,2S,5R)-2-isopropyl-5-methylcyclohexyl)thiouronium p-toluenesulfonate
and
[[(CH₃)₂N]₂CSCH₃]⁺CH₃SO₄⁻
are excluded.

7. Salt according to Claim 5 or 6, **characterised in that** R is hydrogen and/or a straight-chain or branched alkyl having 1 to 12 C atoms.

8. Salt according to Claim 5 or 6, **characterised in that** R is selected from the group methyl, ethyl, n-propyl, isopropyl, n-butyl, tert-butyl, sec-butyl, phenyl and cyclohexyl.

9. Salt according to one of Claims 6 to 8, **characterised in that** R⁰ denotes a straight-chain or branched alkyl having 1 to 12 C atoms, which may be partially or fully substituted by F, but where fluorination of the α-CH₂ group of R⁰ is excluded, and X = S.

10. Salt according to one of Claims 5 or 7 to 8, **characterised in that** R⁰ denotes a straight-chain or branched alkyl having 1 to 12 C atoms and X = O.

11. Salt according to one of Claims 5 or 6, **characterised in that** the anion is selected from the group [CF₃CF₂SO₃]⁻, [CH₃CH₂SO₃]⁻, [(CF₃SO₂)₂N]⁻, [(C₂F₅SO₂)₂N]⁻, [(CF₃SO₂)₃C]⁻, [(C₂F₅SO₂)₃C]⁻, [CH₃CH₂OSO₃]⁻, [(FSO₂)₃C]⁻, [CF₃C(O)O]⁻, [CF₃CF₂C(O)O]⁻, [CH₃CH₂C(O)O]⁻, [CH₃C(O)O]⁻, [P(C₂F₅)₃F₃]⁻, [P(CF₃)₃F₃]⁻, [P(C₂F₄H)(CF₃)₂F₃]⁻, [P(C₂F₃H₂)₃F₃]⁻, [P(C₂F₅)(CF₃)₂F₃]⁻, [P(C₆F₅)₃F₃]⁻, [P(C₃F₇)₃F₃]⁻, [P(C₄F₉)₃F₃]⁻, [P(C₂F₅)₂F₄]⁻, [(C₂F₅)₂P(O)O]⁻, [(C₂F₅)P(O)O₂]²⁻, [P(C₆F₅)₂F₄]⁻, [(CF₃)₂P(O)O]⁻, [(CH₃)₂P(O)O]⁻, [(C₄F₉)₂P(O)O]⁻, [CF₃P(O)O₂]²⁻, [CH₃P(O)O₂]²⁻, [(CH₃O)₂P(O)O]⁻, [BF₃(CF₃)]⁻, [BF₂(C₂F₅)₂]⁻ [BF₃(C₂F₅)]⁻, [BF₂(CF₃)₂]⁻, [B(C₂F₅)₄]⁻, [BF₃(CN)]⁻,
[BF₂(CN)₂]⁻, [B(CN)₄]⁻, [B(CF₃)₄]⁻, [B(OCH₃)₄]⁻, [B(OCH₃)₂(OC₂Hs)]⁻, [B(O₂C₂H₄)₂]⁻, [B(O₂C₂H₂)₂]⁻, [B(O₂CH₄)₂]⁻, [N(CF₃)₂]⁻, [N(CN₂)₂]⁻, [C(CN)₃]⁻, [AlCl₄]⁻ or [SiF₆]²⁻,
with the proviso that
for X = O, the anions CF₃COO⁻ or [B(C₆H₅)₄]⁻ are to be allowed,
for X = O and R⁰ = ethyl, the anion A⁻ = CH₃CH₂OSO₃⁻ is to be excluded and
for X = S and R and R⁰ = alkyl groups having 1 to 20 C atoms, the anions A⁻ = [BF₄]⁻, CF₃COO⁻ or [B(C₆H₅)₄]⁻ are to be allowed and the compounds
[n-butyl-SC(NH₂)₂]⁺ ethylOSO₃⁻, 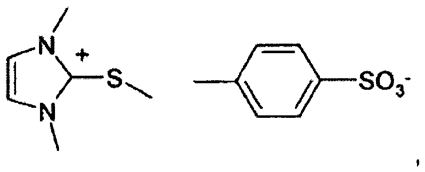 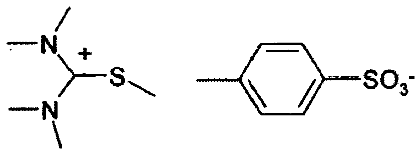 and
2-((1S,2S,5R)-2-isopropyl-5-methylcyclohexyl)thiouronium p-toluenesulfonate are excluded.

12. Process for the preparation of a salt according to one of Claims 5 to 11, **characterised in that** a thiourea C(S)(NR₂)₂ or a urea C(O)(NR₂)₂, where the radicals R are as defined in one of Claims 5 to 11, is alkylated using an ester AR⁰, where the radicals R⁰ are as defined in one of Claims 5 to 11 and A is selected from the group [R¹CH₂OSO₃], [R¹SO₃], [R^{F}SO₃], [R^{F}C(O)O] and [CCl₃C(O)O], or using an oxonium salt (R⁰)₃O⁺ A⁻, where the radicals R¹ or R^{F} are as defined in one of Claims 5 to 11 and A⁻ is selected from the group [(FSO₂)₃C]⁻ and [BF₄]⁻,
with observance of the proviso that
for X = O, the anions CF₃COO⁻ or [B(C₆H₅)₄]⁻ are to be allowed, for X = O and R⁰ = ethyl, the anion A⁻ = CH₃CH₂OSO₃⁻ is to be excluded and
for X = S and R and R⁰ = alkyl groups having 1 to 20 C atoms, the anions A⁻ = [BF₄]⁻, CF₃COO⁻ or [B(C₆H₅)₄]⁻ are to be allowed and the compounds
[n-butyl-SC(NH₂)₂]⁺ ethylOSO₃⁻, 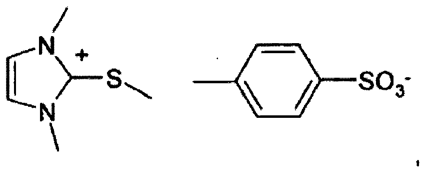 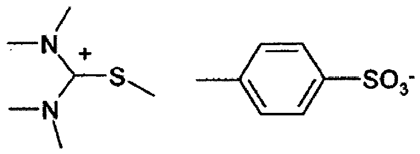 and
2-((1S,2S,5R)-2-isopropyl-5-methylcyclohexyl)thiouronium p-toluenesulfonate,
N,N,N,N-tetramethyldiaminomethoxycarbonium methylsulfate, [[(CH₃)₂N]₂CSCH₃]⁺CH₃SO₄⁻
are excluded.

13. Process according to Claim 12, **characterised in that** the reaction is carried out at a temperature at which at least one of the components is liquid.

14. Process for the preparation of a salt of the formula (1) according to one of Claims 5 to 11, **characterised in that** a salt having a cation of the general formula (1) according to one of Claims 5 to 11 and an anion A⁻ selected from the group [BF₄]⁻, Br⁻, Cl⁻, I⁻ or [ClO₄]⁻ is reacted with a salt Kt⁺ A⁻ or with an acid AH, where Kt is an alkali metal or alkaline-earth metal and A is as defined in one of Claims 5 to 11.

## Revendications

1. Utilisation d'un sel de formule (1), 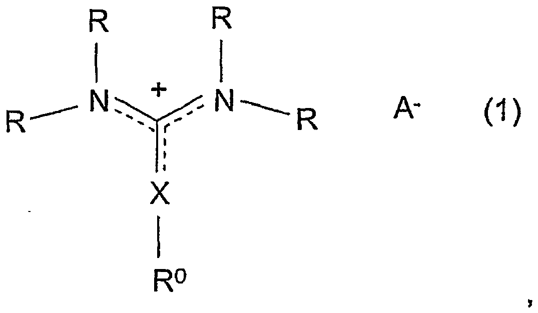 dans laquelle
X désigne O ou S,
où les substituants R et R⁰ revêtent chacun, indépendamment l'un de l'autre, la signification
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, pouvant être substitué par des groupements alkyle ayant 1-6 atomes de C,
et R peut également désigner hydrogène,
où un ou plusieurs substituants R ou R⁰ peuvent être partiellement ou totalement substitués par halogène ou partiellement par CN ou NO₂, mais où l'halogénation en position α dans R⁰ est exclue, et
halogène désigne F, CI, Br ou I,
où les substituants R et R⁰ peuvent être reliés les uns aux autres par paires par une liaison simple ou double,
et où un atome de carbone ou deux atomes de carbone non adjacents d'un ou plusieurs substituants R ou R⁰ qui ne sont pas directement adjacents à l'hétéroatome peuvent être remplacés par des atomes et/ou des groupes d'atomes choisis dans le groupe constitué par -O-, -C(O)-, -C(O)O-, -S-, -S(O)-, -SO₂-, -SO₃-, -N=, -N=N-, -NH-, -NR'-, -PR'-, -P(O)R'-, -P(O)R'-O-, -O-P(O)R'-O- et -P(R')₂=N-, où R' est alkyle non fluoré, partiellement fluoré ou perfluoré ayant 1-6 atomes de C, cycloalkyle saturé ou partiellement insaturé ayant 3-7 atomes de C, phényle non substitué ou substitué ou hétérocycle non substitué ou substitué,
et
A⁻ est choisi dans le groupe constitué par:
[R¹SO₃]⁻, [R^{F'}SO₃]⁻, [(R^{F}SO₂)₂N]⁻, [(R^{F}SO₂)₃C]⁻, [(FSO₂)₃C]⁻, [R¹CH₂OSO₃]⁻, [R¹C(O)O]⁻, [R^{F'}C(O)O]⁻, [CCl₃C(O)O]⁻, [(CN)₃C]⁻, [(CN)₂CR¹]-, [(R¹O(O)C)₂CR¹]⁻, [P(CₙF₂ₙ₊₁₋ₘHₘ)_{y}F_{6-y}]⁻, [P(C₆F₅)_{y}F_{6-y}]⁻, [R¹₂P(O)O]⁻, [R¹P(O)O₂]²⁻, [(R¹O)₂P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4-z}]⁻, [B(CN)₄]⁻, [B(C₆F₅)₄]⁻ [B(OR¹)₄]⁻, [N(CF₃)₂]⁻, [N(CN)₂]⁻, [AlCl₄]⁻ ou [SiF₆]²⁻,
où les substituants R^{F} et R^{F'} revêtent chacun, indépendamment l'un de l'autre, la signification
alkyle à chaîne linéaire ou ramifiée et perfluoré ayant 1-20 atomes de C,
où le groupement trifluorométhyle est exclu pour R^{F'},
alcényle à chaîne linéaire ou ramifiée et perfluoré ayant 2-20 atomes de C et une ou plusieurs doubles liaisons,
phényle perfluoré et cycloalkyle saturé, partiellement ou totalement insaturé ayant 3-7 atomes de C, pouvant être substitué par des groupements perfluoroalkyle,
où les substituants R^{F} ou R^{F'} peuvent être reliés les uns aux autres par paires par une liaison simple ou double et
où un atome de carbone ou deux atomes de carbone non adjacents du substituant R^{F} ou R^{F'} qui ne sont pas en position α par rapport à l'hétéroatome peuvent être remplacés par des atomes et/ou des groupes d'atomes choisis dans le groupe constitué par -O-, -C(O)-, -S-, -S(O)-, -SO₂-, -N=, -N=N-, -NR'-, -PR'- et -P(O)R'- ou peuvent posséder un groupement terminal R'-O-SO₂- ou R'-O-C(O)-, où R' désigne alkyle non fluoré, partiellement fluoré ou perfluoré ayant 1-6 atomes de C, cycloalkyle saturé ou partiellement insaturé ayant 3-7 atomes de C, phényle non substitué ou substitué ou hétérocycle non substitué ou substitué
et
où les substituants R¹ revêtent chacun, indépendamment l'un de l'autre, la signification
hydrogène dans le cas où A⁻ = [(CN)₂CR¹]⁻ ou [(R¹O(O)C)₂CR¹]⁻ et X = O ou S, ou
hydrogène dans le cas où A⁻ = [R¹CH₂OSO₃]⁻, X = S ou O et les substituants R et R⁰ = des groupements alkyle ayant de 1 à 20 atomes de C,
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, pouvant être substitué par des groupements alkyle ayant 1-6 atomes de C,
où les substituants R¹ peuvent être partiellement substitués par CN,
NO₂ ou halogène et
halogène désigne F, CI, Br ou I,
où les substituants R¹ peuvent être reliés les uns aux autres par paires par une liaison simple ou double et
où un atome de carbone ou deux atomes de carbone non adjacents du substituant R¹ qui ne sont pas en position α par rapport à l'hétéroatome peuvent être remplacés par des atomes et/ou des groupes d'atomes choisis dans le groupe constitué par -O-, -C(O)-, -C(O)O-, -S-, -S(O)-, -SO₂-, -SO₃-, -N=, -N=N-, -NH-, -NR'-, -PR'- et -P(O)R'-, -P(O)R'O-, - OP(O)R'O-, -PR'₂=N-, -C(O)NH-, -C(O)NR'-, -SO₂NH- ou -SO₂NR'-, où R' désigne alkyle non fluoré, partiellement fluoré ou perfluoré ayant 1-6 atomes de C, cycloalkyle saturé ou partiellement insaturé ayant 3-7 atomes de C, phényle non substitué ou substitué ou hétérocycle non substitué ou substitué
et les variables
n désigne 1 à 20,
m désigne 0, 1, 2 ou 3,
y désigne 0, 1, 2, 3 ou 4,
z désigne 0, 1, 2 ou 3,
à condition que
pour X = S et R et R⁰ = des groupements alkyle ayant de 1 à 20 atomes de C, les anions A⁻ = [BF₄]⁻, CF₃COO⁻ ou [B(C₆H₅)₄]⁻ doivent être permis
et
pour X = O, les anions A⁻ = CF₃COO⁻ et [B(C₆H₅)₄]⁻ doivent être permis
et
pour X = O et R⁰ = éthyle, l'anion A⁻ = CH₃CH₂OSO₃⁻ doit être exclu, comme liquide ionique.

2. Utilisation d'un sel de formule (1), 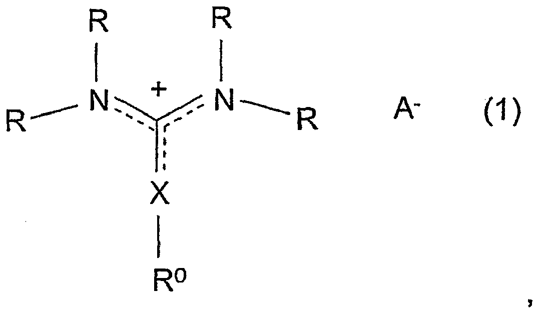 dans laquelle
X désigne O ou S,
où les substituants R et R⁰ revêtent chacun, indépendamment l'un de l'autre, la signification
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, pouvant être substitué par des groupements alkyle ayant 1-6 atomes de C,
et R peut également désigner hydrogène,
où un ou plusieurs substituants R ou R⁰ peuvent être partiellement ou totalement substitués par halogène ou partiellement par CN ou NO₂, mais où l'halogénation en position α dans R⁰ est exclue, et
halogène désigne F, CI, Br ou I,
où les substituants R et R⁰ peuvent être reliés les uns aux autres par paires par une liaison simple ou double,
et où un atome de carbone ou deux atomes de carbone non adjacents d'un ou plusieurs substituants R ou R⁰ qui ne sont pas directement adjacents à l'hétéroatome peuvent être remplacés par des atomes et/ou des groupes d'atomes choisis dans le groupe constitué par -O-, -C(O)-, -C(0)0-, -S-, -S(O)-, -SO₂-, -SO₃-, -N=, -N=N-, -NH-, -NR'-, -PR'-, -P(O)R'-, -P(O)R'-O-, -O-P(O)R'-O- et -P(R')₂=N-, où R' est alkyle non fluoré, partiellement fluoré ou perfluoré ayant 1-6 atomes de C, cycloalkyle saturé ou partiellement insaturé ayant 3-7 atomes de C, phényle non substitué ou substitué ou hétérocycle non substitué ou substitué,
et
A⁻ est choisi dans le groupe constitué par:
[R¹SO₃]⁻, [R^{F'}SO₃]⁻, [(R^{F}SO₂)₂N]⁻, [(R^{F}SO₂)₃C]⁻ [(FSO₂)₃C]⁻, [R¹CH₂OSO₃]⁻, [R¹C(O)O]⁻, [R^{F'}C(O)O]⁻, [CCl₃C(O)O]⁻, [(CN)₃C]⁻, [(CN)₂CR¹]⁻, [(R¹O(O)C)₂CR¹]⁻, [P(CₙF₂ₙ₊₁₋ₘHₘ)_{y}F_{6-y}]⁻, [P(C₆F₅)_{y}F_{6-y}]⁻, [R¹₂P(O)O]⁻, [R¹P(O)O₂]²⁻, [(R¹O)₂P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4-z}]⁻, [B(CN)₄]⁻, [B(C₆F₅)₄]⁻, [B(OR¹)₄]⁻, [N(CF₃)₂]⁻, [N(CN)₂]⁻, [AlCl₄]⁻ ou [SiF₆]²⁻,
où les substituants R^{F} et R^{F'} revêtent chacun, indépendamment l'un de l'autre, la signification
alkyle à chaîne linéaire ou ramifiée et perfluoré ayant 1-20 atomes de C,
où le groupement trifluorométhyle est exclu pour R^{F'},
alcényle à chaîne linéaire ou ramifiée et perfluoré ayant 2-20 atomes de C et une ou plusieurs doubles liaisons,
phényle perfluoré et cycloalkyle saturé, partiellement ou totalement insaturé ayant 3-7 atomes de C, pouvant être substitué par des groupements perfluoroalkyle,
où les substituants R^{F} ou R^{F'} peuvent être reliés les uns aux autres par paires par une liaison simple ou double et
où un atome de carbone ou deux atomes de carbone non adjacents du substituant R^{F} ou R^{F'} qui ne sont pas en position α par rapport à l'hétéroatome peuvent être remplacés par des atomes et/ou des groupes d'atomes choisis dans le groupe constitué par -O-, -C(O)-, -S-, -S(O)-, -SO₂-, -N=, -N=N-, -NR'-, -PR'- et -P(O)R'- ou peuvent posséder un groupement terminal R'-O-SO₂- ou R'-O-C(O)-, où R' désigne alkyle non fluoré, partiellement fluoré ou perfluoré ayant 1-6 atomes de C, cycloalkyle saturé ou partiellement insaturé ayant 3-7 atomes de C, phényle non substitué ou substitué ou hétérocycle non substitué ou substitué
et
où les substituants R¹ revêtent chacun, indépendamment l'un de l'autre, la signification
hydrogène dans le cas où A⁻ = [(CN)₂CR¹]⁻ ou [(R¹O(O)C)₂CR¹]⁻ et X = O ou S, ou
hydrogène dans le cas où A⁻ = [R¹CH₂OSO₃]⁻, X = S ou O et les substituants R et R⁰ = des groupements alkyle ayant de 1 à 20 atomes de C,
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, pouvant être substitué par des groupements alkyle ayant 1-6 atomes de C,
où les substituants R¹ peuvent être partiellement substitués par CN, NO₂ ou halogène et
halogène désigne F, CI, Br ou I,
où les substituants R¹ peuvent être reliés les uns aux autres par paires par une liaison simple ou double et
où un atome de carbone ou deux atomes de carbone non adjacents du substituant R¹ qui ne sont pas en position α par rapport à l'hétéroatome peuvent être remplacés par des atomes et/ou des groupes d'atomes choisis dans le groupe constitué par -O-, -C(O)-, -C(O)O-, -S-, -S(O)-, -SO₂-, -SO₃-, -N=, -N=N-, -NH-, -NR'-, -PR'- et -P(O)R'-, -P(O)R'O-,-OP(O)R'O-, -PR'₂=N-, -C(O)NH-, -C(O)NR'-, -SO₂NH- ou -SO₂NR'-, où R' désigne alkyle non fluoré, partiellement fluoré ou perfluoré ayant 1-6 atomes de C, cycloalkyle saturé ou partiellement insaturé ayant 3-7 atomes de C, phényle non substitué ou substitué ou hétérocycle non substitué ou substitué
et les variables
n désigne 1 à 20,
m désigne 0, 1, 2 ou 3,
y désigne 0, 1, 2, 3 ou 4,
z désigne 0, 1, 2 ou 3,
à condition que
pour X = S et R et R⁰ = des groupements alkyle ayant de 1 à 20 atomes de C, les anions A⁻ = [BF₄]⁻, CF₃COO⁻ ou [B(C₆H₅)₄]⁻ doivent être permis
et
pour X = O, les anions A⁻ = CF₃COO⁻ et [B(C₆H₅)₄]⁻ doivent être permis
et
pour X = O et R⁰ = éthyle, l'anion A⁻ = CH₃CH₂OSO₃⁻ doit être exclu, comme électrolyte non aqueux.

3. Utilisation d'un sel de formule (1), 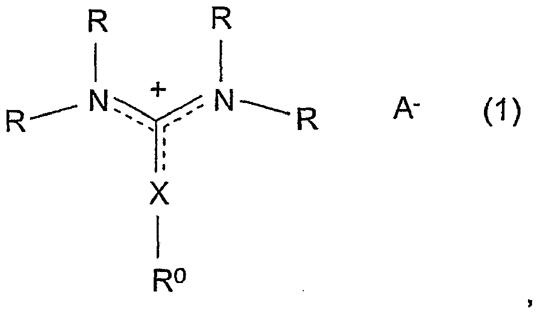 dans laquelle
X désigne O ou S,
où les substituants R et R⁰ revêtent chacun, indépendamment l'un de l'autre, la signification
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, pouvant être substitué par des groupements alkyle ayant 1-6 atomes de C,
et R peut également désigner hydrogène,
où un ou plusieurs substituants R ou R⁰ peuvent être partiellement ou totalement substitués par halogène ou partiellement par CN ou NO₂, mais où l'halogénation en position α dans R⁰ est exclue, et
halogène désigne F, Cl, Br ou I,
où les substituants R et R⁰ peuvent être reliés les uns aux autres par paires par une liaison simple ou double,
et où un atome de carbone ou deux atomes de carbone non adjacents d'un ou plusieurs substituants R ou R⁰ qui ne sont pas directement adjacents à l'hétéroatome peuvent être remplacés par des atomes et/ou des groupes d'atomes choisis dans le groupe constitué par -O-, -C(O)-, -C(0)0-, -S-, -S(O)-, -SO₂-, -SO₃-, -N=, -N=N-, -NH-, -NR'-, -PR'-, -P(O)R'-, -P(O)R'-O-, -O-P(O)R'-O- et -P(R')₂=N-, où R' est alkyle non fluoré, partiellement fluoré ou perfluoré ayant 1-6 atomes de C, cycloalkyle saturé ou partiellement insaturé ayant 3-7 atomes de C, phényle non substitué ou substitué ou hétérocycle non substitué ou substitué,
et
A⁻ est choisi dans le groupe constitué par:
[R¹SO₃]⁻, [R^{F'}SO₃]⁻, [(R^{F}SO₂)₂N]⁻, [(R^{F}SO₂)₃C]⁻, [(FSO₂)₃C]⁻, [R¹CH₂OSO₃]⁻, [R¹C(O)O]⁻, [R^{F'}C(O)O]⁻, [CCl₃C(O)O]⁻, [(CN)₃C]⁻, [(CN)₂CR¹]-, [(R¹O(O)C)₂CR¹]⁻, [P(CₙF₂ₙ₊₁₋ₘHₘ)_{y}F_{6-y}]⁻, [P(C₆F₅)_{y}F_{6-y}]⁻, [R¹₂P(O)O]⁻, [R¹P(O)O₂]²⁻, [(R¹O)₂P(O)O]⁻, [(R¹O)P(O)O₂]²-, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4-z}]⁻, [B(CN)₄]⁻, [B(C₆F₅)₄]⁻, [B(OR¹)₄]⁻, [N(CF₃)₂]⁻, [N(CN)₂]⁻, [AlCl₄]⁻ ou [SiF₆]²⁻,
où les substituants R^{F} et R^{F'} revêtent chacun, indépendamment l'un de l'autre, la signification
alkyle à chaîne linéaire ou ramifiée et perfluoré ayant 1-20 atomes de C,
où le groupement trifluorométhyle est exclu pour R^{F'},
alcényle à chaîne linéaire ou ramifiée et perfluoré ayant 2-20 atomes de C et une ou plusieurs doubles liaisons,
phényle perfluoré et cycloalkyle saturé, partiellement ou totalement insaturé ayant 3-7 atomes de C, pouvant être substitué par des groupements perfluoroalkyle,
où les substituants R^{F} ou R^{F'} peuvent être reliés les uns aux autres par paires par une liaison simple ou double et
où un atome de carbone ou deux atomes de carbone non adjacents du substituant R^{F} ou R^{F'} qui ne sont pas en position α par rapport à l'hétéroatome peuvent être remplacés par des atomes et/ou des groupes d'atomes choisis dans le groupe constitué par -O-, -C(O)-, -S-, -S(O)-, -SO₂-, -N=, -N=N-, -NR'-, -PR'- et -P(O)R'- ou peuvent posséder un groupement terminal R'-O-SO₂- ou R'-O-C(O)-, où R' désigne alkyle non fluoré, partiellement fluoré ou perfluoré ayant 1-6 atomes de C, cycloalkyle saturé ou partiellement insaturé ayant 3-7 atomes de C, phényle non substitué ou substitué ou hétérocycle non substitué ou substitué
et
où les substituants R¹ revêtent chacun, indépendamment l'un de l'autre, la signification
hydrogène dans le cas où A⁻ = [(CN)₂CR¹]⁻ ou [(R¹O(O)C)₂CR¹]⁻ et X = O ou S, ou
hydrogène dans le cas où A⁻ = [R¹CH₂OSO₃]⁻, X = S ou O et les substituants R et R⁰ = des groupements alkyle ayant de 1 à 20 atomes de C,
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, pouvant être substitué par des groupements alkyle ayant 1-6 atomes de C,
où les substituants R¹ peuvent être partiellement substitués par CN, NO₂ ou halogène et
halogène désigne F, CI, Br ou I,
où les substituants R¹ peuvent être reliés les uns aux autres par paires par une liaison simple ou double et
où un atome de carbone ou deux atomes de carbone non adjacents du substituant R¹ qui ne sont pas en position α par rapport à l'hétéroatome peuvent être remplacés par des atomes et/ou des groupes d'atomes choisis dans le groupe constitué par -O-, -C(O)-, -C(O)O-, -S-, -S(O)-, -SO₂-, -SO₃-, -N=, -N=N-, -NH-, -NR'-, -PR'- et -P(O)R'-, -P(O)R'O-, - OP(O)R'O-, -PR'₂=N-, -C(O)NH-, -C(O)NR'-, -SO₂NH- ou -SO₂NR'-, où R' désigne alkyle non fluoré, partiellement fluoré ou perfluoré ayant 1-6 atomes de C, cycloalkyle saturé ou partiellement insaturé ayant 3-7 atomes de C, phényle non substitué ou substitué ou hétérocycle non substitué ou substitué
et les variables
n désigne 1 à 20,
m désigne 0, 1, 2 ou 3,
y désigne 0, 1, 2, 3 ou 4,
z désigne 0, 1, 2 ou 3,
à condition que
pour X = S et R et R⁰ = des groupements alkyle ayant de 1 à 20 atomes de C, les anions A⁻ = [BF₄]⁻, CF₃COO⁻ ou [B(C₆H₅)₄]⁻ doivent être permis
et
pour X = O, les anions A⁻ = CF₃COO⁻ et [B(C₆H₅)₄]⁻ doivent être permis
et
pour X = O et R⁰ = éthyle, l'anion A⁻ = CH₃CH₂OSO₃⁻ doit être exclu, comme catalyseur de transfert de phase.

4. Utilisation d'un sel de formule (1), 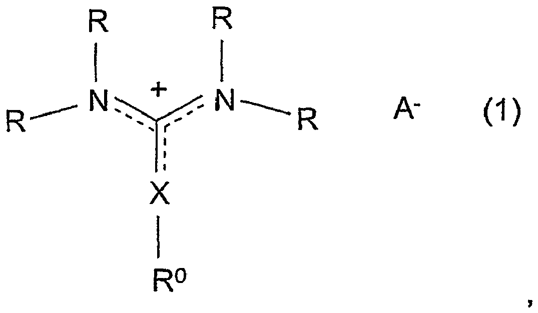 dans laquelle
X désigne O ou S,
où les substituants R et R⁰ revêtent chacun, indépendamment l'un de l'autre, la signification
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, pouvant être substitué par des groupements alkyle ayant 1-6 atomes de C,
et R peut également désigner hydrogène,
où un ou plusieurs substituants R ou R⁰ peuvent être partiellement ou totalement substitués par halogène ou partiellement par CN ou NO₂, mais où l'halogénation en position α dans R⁰ est exclue, et
halogène désigne F, CI, Br ou I,
où les substituants R et R⁰ peuvent être reliés les uns aux autres par paires par une liaison simple ou double,
et où un atome de carbone ou deux atomes de carbone non adjacents d'un ou plusieurs substituants R ou R⁰ qui ne sont pas directement adjacents à l'hétéroatome peuvent être remplacés par des atomes et/ou des groupes d'atomes choisis dans le groupe constitué par -O-, -C(O)-, -C(0)0-, -S-, -S(O)-, -SO₂-, -SO₃-, -N=, -N=N-, -NH-, -NR'-, -PR'-, -P(O)R'-, -P(O)R'-O-, -O-P(O)R'-O- et -P(R')₂=N-, où R' est alkyle non fluoré, partiellement fluoré ou perfluoré ayant 1-6 atomes de C, cycloalkyle saturé ou partiellement insaturé ayant 3-7 atomes de C, phényle non substitué ou substitué ou hétérocycle non substitué ou substitué,
et
A⁻ est choisi dans le groupe constitué par:
[R¹SO₃]⁻, [R^{F'}SO₃]⁻, [(R^{F}SO₂)₂N]⁻, [(R^{F}SO₂)₃C]⁻, [(FSO₂)₃C]⁻, [R¹CH₂OSO₃]⁻, [R¹C(O)O]⁻, [R^{F'}C(O)O]⁻, [CCl₃C(O)O]⁻, [(CN)₃C]⁻, [(CN)₂CR¹]⁻, [(R¹O(O)C)₂CR¹]⁻, [P(CₙF₂ₙ₊₁₋ₘHₘ)_{y}F_{6-y}]⁻, [P(C₆F₅)_{y}F_{6-y}]⁻, [R¹₂P(O)O]⁻, [R¹P(O)O₂]²⁻, [(R¹O)₂P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4-z}]⁻, [B(CN)₄]⁻, [B(C₆F₅)₄]⁻, [B(OR¹)₄]⁻, [N(CF₃)₂]⁻, [N(CN)₂]⁻, [AlCl₄]⁻ ou [SiF₆]²⁻,
où les substituants R^{F} et R^{F'} revêtent chacun, indépendamment l'un de l'autre, la signification
alkyle à chaîne linéaire ou ramifiée et perfluoré ayant 1-20 atomes de C,
où le groupement trifluorométhyle est exclu pour R^{F'},
alcényle à chaîne linéaire ou ramifiée et perfluoré ayant 2-20 atomes de C et une ou plusieurs doubles liaisons,
phényle perfluoré et cycloalkyle saturé, partiellement ou totalement insaturé ayant 3-7 atomes de C, pouvant être substitué par des groupements perfluoroalkyle,
où les substituants R^{F} ou R^{F'} peuvent être reliés les uns aux autres par paires par une liaison simple ou double et
où un atome de carbone ou deux atomes de carbone non adjacents du substituant R^{F} ou R^{F'} qui ne sont pas en position α par rapport à l'hétéroatome peuvent être remplacés par des atomes et/ou des groupes d'atomes choisis dans le groupe constitué par -O-, -C(O)-, -S-, -S(O)-, -SO₂-, -N=, -N=N-, -NR'-, -PR'- et -P(O)R'- ou peuvent posséder un groupement terminal R'-O-SO₂- ou R'-O-C(O)-, où R' désigne alkyle non fluoré, partiellement fluoré ou perfluoré ayant 1-6 atomes de C, cycloalkyle saturé ou partiellement insaturé ayant 3-7 atomes de C, phényle non substitué ou substitué ou hétérocycle non substitué ou substitué
et
où les substituants R¹ revêtent chacun, indépendamment l'un de l'autre, la signification
hydrogène dans le cas où A⁻ = [(CN)₂CR¹]⁻ ou [(R¹O(O)C)₂CR¹]⁻ et X = O ou S, ou
hydrogène dans le cas où A⁻ = [R¹CH₂OSO₃]⁻, X = S ou O et les substituants R et R⁰ = des groupements alkyle ayant de 1 à 20 atomes de C,
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, pouvant être substitué par des groupements alkyle ayant 1-6 atomes de C,
où les substituants R¹ peuvent être partiellement substitués par CN, NO₂ ou halogène et
halogène désigne F, CI, Br ou I,
où les substituants R¹ peuvent être reliés les uns aux autres par paires par une liaison simple ou double et
où un atome de carbone ou deux atomes de carbone non adjacents du substituant R¹ qui ne sont pas en position α par rapport à l'hétéroatome peuvent être remplacés par des atomes et/ou des groupes d'atomes choisis dans le groupe constitué par -O-, -C(O)-, -C(O)O-, -S-, -S(O)-, -SO₂-, -SO₃-, -N=, -N=N-, -NH-, -NR'-, -PR'- et -P(O)R'-, -P(O)R'O-, - OP(O)R'O-, -PR'₂=N-, -C(O)NH-, -C(O)NR'-, -SO₂NH- ou -SO₂NR'-, où R' désigne alkyle non fluoré, partiellement fluoré ou perfluoré ayant 1-6 atomes de C, cycloalkyle saturé ou partiellement insaturé ayant 3-7 atomes de C, phényle non substitué ou substitué ou hétérocycle non substitué ou substitué
et les variables
n désigne 1 à 20,
m désigne 0, 1, 2 ou 3,
y désigne 0, 1, 2, 3 ou 4,
z désigne 0, 1, 2 ou 3,
à condition que
pour X = S et R et R⁰ = des groupements alkyle ayant de 1 à 20 atomes de C, les anions A⁻ = [BF₄]⁻, CF₃COO⁻ ou [B(C₆H₅)₄]⁻ doivent être permis
et
pour X = O, les anions A⁻ = CF₃COO ⁻ et [B(C₆H₅)₄]⁻ doivent être permis
et
pour X = O et R⁰ = éthyle, l'anion A⁻ = CH₃CH₂OSO₃⁻ doit être exclu, comme substance tensioactive.

5. Sels d'uronium de formule (1) 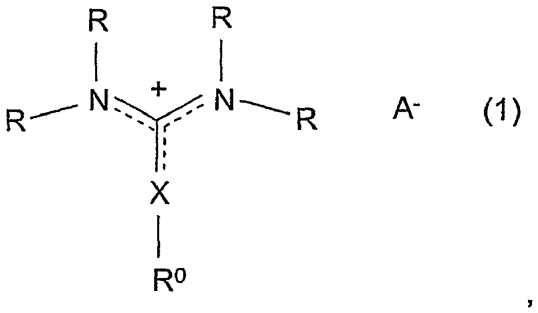 dans laquelle
X désigne O,
où les substituants R et R⁰ revêtent chacun, indépendamment l'un de l'autre, la signification
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, pouvant être substitué par des groupements alkyle ayant 1-6 atomes de C,
et R peut également désigner hydrogène,
où un ou plusieurs substituants R ou R⁰ peuvent être partiellement ou totalement substitués par halogène ou partiellement par CN ou NO₂, mais où l'halogénation en position α dans R⁰ est exclue, et
halogène désigne F, CI, Br ou I,
où les substituants R et R⁰ peuvent être reliés les uns aux autres par paires par une liaison simple ou double,
et où un atome de carbone ou deux atomes de carbone non adjacents d'un ou plusieurs substituants R ou R⁰ qui ne sont pas directement adjacents à l'hétéroatome peuvent être remplacés par des atomes et/ou des groupes d'atomes choisis dans le groupe constitué par -O-, -C(O)-, -C(O)O-, -S-, -S(O)-, -SO₂-, -SO₃-, -N=, -N=N-, -NH-, -NR'-, -PR'-, -P(O)R'-, -P(O)R'-O-, -O-P(O)R'-O- et -P(R')₂=N-, où R' est alkyle non fluoré, partiellement fluoré ou perfluoré ayant 1-6 atomes de C, cycloalkyle saturé ou partiellement insaturé ayant 3-7 atomes de C, phényle non substitué ou substitué ou hétérocycle non substitué ou substitué,
et
A⁻ est choisi dans le groupe constitué par:
[R¹SO₃]⁻, [R^{F'}SO₃]⁻ [(R^{F}SO₂)₂N]⁻, [(R^{F}SO₂)₃C]⁻, [(FSO₂)₃C]⁻, [R¹CH₂OSO₃]⁻, [R¹C(O)O]⁻, [R^{F'}C(O)O]⁻, [CCl₃C(O)O]⁻, [(CN)₃C]⁻, [(CN)₂CR¹]-, [(R¹O(O)C)₂CR¹]⁻, [P(CₙF₂ₙ₊₁₋ₘHₘ)_{y}F_{6-y}]⁻, [P(C₆F₅)_{y}F_{6-y}]⁻, [R¹₂P(O)O]⁻, [R¹P(O)O₂]²⁻, [(R¹O)2P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4-z}]⁻, [B(CN)₄]⁻, B(C₆F₅)₄]⁻, [B(OR¹)₄]⁻, [N(CF₃)₂]⁻, [N(CN)₂]⁻, [AlCl₄]⁻, [SiF₆]²⁻, CF₃COO⁻ ou [B(C₆H₅)₄]⁻,
où les substituants R^{F} et R^{F'} revêtent chacun, indépendamment l'un de l'autre, la signification
alkyle à chaîne linéaire ou ramifiée et perfluoré ayant 1-20 atomes de C,
où le groupement trifluorométhyle est exclu pour R^{F'},
alcényle à chaîne linéaire ou ramifiée et perfluoré ayant 2-20 atomes de C et une ou plusieurs doubles liaisons,
phényle perfluoré et cycloalkyle saturé, partiellement ou totalement insaturé ayant 3-7 atomes de C, pouvant être substitué par des groupements perfluoroalkyle,
où les substituants R^{F} ou R^{F'} peuvent être reliés les uns aux autres par paires par une liaison simple ou double et
où un atome de carbone ou deux atomes de carbone non adjacents du substituant R^{F} ou R^{F'} qui ne sont pas en position α par rapport à l'hétéroatome peuvent être remplacés par des atomes et/ou des groupes d'atomes choisis dans le groupe constitué par -O-, -C(O)-, -S-, -S(O)-, -SO₂-, -N=, -N=N-, -NR'-, -PR'- et -P(O)R'- ou peuvent posséder un groupement terminal R'-O-SO₂- ou R'-O-C(O)-, où R' désigne alkyle non fluoré, partiellement fluoré ou perfluoré ayant 1-6 atomes de C, cycloalkyle saturé ou partiellement insaturé ayant 3-7 atomes de C, phényle non substitué ou substitué ou hétérocycle non substitué ou substitué
et
où les substituants R¹ revêtent chacun, indépendamment l'un de l'autre, la signification
hydrogène dans le cas où A⁻ = [(CN)₂CR¹]- ou [(R¹O(O)C)₂CR¹]⁻ et X = O, ou
hydrogène dans le cas où A⁻ = [R¹CH₂OSO₃]⁻, X = O et les substituants R et R⁰ = des groupements alkyle ayant de 1 à 20 atomes de C, alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, pouvant être substitué par des groupements alkyle ayant 1-6 atomes de C,
où les substituants R¹ peuvent être partiellement substitués par CN, NO₂ ou halogène et
halogène désigne F, CI, Br ou I,
où les substituants R¹ peuvent être reliés les uns aux autres par paires par une liaison simple ou double et
où un atome de carbone ou deux atomes de carbone non adjacents du substituant R¹ qui ne sont pas en position α par rapport à l'hétéroatome peuvent être remplacés par des atomes et/ou des groupes d'atomes choisis dans le groupe constitué par -O-, -C(O)-, -C(O)O-, -S-, -S(O)-, -SO₂-, -SO₃-, -N=, -N=N-, -NH-, -NR'-, -PR'- et -P(O)R'-, -P(O)R'O-, - OP(O)R'O-, -PR'₂=N-, -C(O)NH-, -C(O)NR'-, -SO₂NH- ou -SO₂NR'-, où R' désigne alkyle non fluoré, partiellement fluoré ou perfluoré ayant 1-6 atomes de C, cycloalkyle saturé ou partiellement insaturé ayant 3-7 atomes de C, phényle non substitué ou substitué ou hétérocycle non substitué ou substitué
et les variables
n désigne 1 à 20,
m désigne 0, 1, 2 ou 3,
y désigne 1, 2, 3 ou 4,
z désigne 0, 1, 2 ou 3,
à condition que
pour R⁰ = éthyle, l'anion A⁻ = CH₃CH₂OSO₃⁻ doit être exclu et le composé méthylsulfate de N,N,N,N-tétraméthyldiaminométhoxycarbonium est exclu.

6. Sels de thiouronium de formule (1), 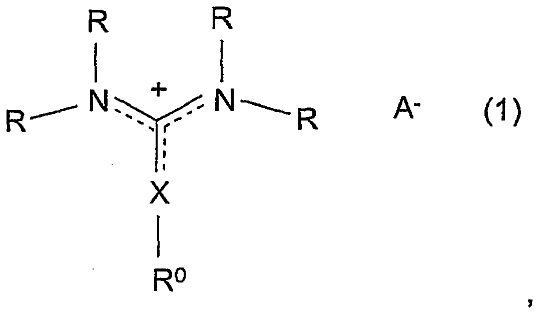 dans laquelle
X désigne S,
où les substituants R et R⁰ revêtent chacun, indépendamment l'un de l'autre, la signification
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, pouvant être substitué par des groupements alkyle ayant 1-6 atomes de C,
et R peut également désigner hydrogène,
où un ou plusieurs substituants R ou R⁰ peuvent être partiellement ou totalement substitués par halogène ou partiellement par CN ou NO₂, mais où l'halogénation en position α dans R⁰ est exclue, et
halogène désigne F, Cl, Br ou I,
où les substituants R et R⁰ peuvent être reliés les uns aux autres par paires par une liaison simple ou double,
et où un atome de carbone ou deux atomes de carbone non adjacents d'un ou plusieurs substituants R ou R⁰ qui ne sont pas directement adjacents à l'hétéroatome peuvent être remplacés par des atomes et/ou des groupes d'atomes choisis dans le groupe constitué par -O-, -C(O)-, -C(0)0-, -S-, -S(O)-, -SO₂-, -SO₃-, -N=, -N=N-, -NH-, -NR'-, -PR'-, -P(O)R'-, -P(O)R'-O-, -O-P(O)R'-O- et -P(R')₂=N-, où R' est alkyle non fluoré, partiellement fluoré ou perfluoré ayant 1-6 atomes de C, cycloalkyle saturé ou partiellement insaturé ayant 3-7 atomes de C, phényle non substitué ou substitué ou hétérocycle non substitué ou substitué,
et
A⁻ est choisi dans le groupe constitué par:
[R¹SO₃]⁻, [R^{F'}SO₃]⁻, [(R^{F}SO₂)₂N]⁻, [(R^{F}SO₂)₃C]⁻, [(FSO₂)₃C]⁻, [R¹CH₂OSO₃]⁻, [R¹C(O)O]⁻, [R^{F'}C(O)O]⁻, [CCl₃C(O)O]⁻, [(CN)₃C]⁻, [(CN)₂CR¹]⁻, [(R¹O(O)C)₂CR¹]⁻, [P(CₙF₂ₙ₊₁₋ₘHₘ)_{y}F_{6-y}]⁻, [P(C₆F₅)_{y}F_{6-y}]⁻,
[R¹₂P(O)O]⁻, [R¹P(O)O₂]²⁻, [(R¹O)₂P(O)O]⁻, [(R¹O)P(O)O₂]²⁻, [(R¹O)(R¹)P(O)O]⁻, [R^{F}₂P(O)O]⁻, [R^{F}P(O)O₂]²⁻, [BF_{z}R^{F}_{4-z}]⁻, [BF_{z}(CN)_{4-z}]⁻, [B(CN)₄]⁻, B(C₆F₅)₄]⁻, [B(OR¹)₄]⁻, [N(CF₃)₂]⁻, [N(CN)₂]⁻, [AlCl₄]⁻ ou [SiF₆]²⁻,
où les substituants R^{F} et R^{F'} revêtent chacun, indépendamment l'un de l'autre, la signification
alkyle à chaîne linéaire ou ramifiée et perfluoré ayant 1-20 atomes de C, où le groupement trifluorométhyle est exclu pour R^{F'},
alcényle à chaîne linéaire ou ramifiée et perfluoré ayant 2-20 atomes de C et une ou plusieurs doubles liaisons,
phényle perfluoré et cycloalkyle saturé, partiellement ou totalement insaturé ayant 3-7 atomes de C, pouvant être substitué par des groupements perfluoroalkyle,
où les substituants R^{F} ou R^{F'} peuvent être reliés les uns aux autres par paires par une liaison simple ou double et
où un atome de carbone ou deux atomes de carbone non adjacents du substituant R^{F} ou R^{F'} qui ne sont pas en position α par rapport à l'hétéroatome peuvent être remplacés par des atomes et/ou des groupes d'atomes choisis dans le groupe constitué par -O-, -C(O)-, -S-, -S(O)-, -SO₂-, -N=, -N=N-, -NR'-, -PR'- et -P(O)R'- ou peuvent posséder un groupement terminal R'-O-SO₂- ou R'-O-C(O)-, où R' désigne alkyle non fluoré, partiellement fluoré ou perfluoré ayant 1-6 atomes de C, cycloalkyle saturé ou partiellement insaturé ayant 3-7 atomes de C, phényle non substitué ou substitué ou hétérocycle non substitué ou substitué
et
où les substituants R¹ revêtent chacun, indépendamment l'un de l'autre, la signification
hydrogène dans le cas où A⁻ = [(CN)₂CR¹]⁻ ou [(R¹O(O)C)₂CR¹]⁻, ou
hydrogène dans le cas où A⁻ = [R¹CH₂OSO₃]⁻ et les substituants R et R⁰ = des groupements alkyle ayant de 1 à 20 atomes de C,
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, pouvant être substitué par des groupements alkyle ayant 1-6 atomes de C,
où les substituants R¹ peuvent être partiellement substitués par CN, NO₂ ou halogène et
halogène désigne F, CI, Br ou I,
où les substituants R¹ peuvent être reliés les uns aux autres par paires par une liaison simple ou double et
où un atome de carbone ou deux atomes de carbone non adjacents du substituant R¹ qui ne sont pas en position α par rapport à l'hétéroatome peuvent être remplacés par des atomes et/ou des groupes d'atomes choisis dans le groupe constitué par -O-, -C(O)-, -C(O)O-, -S-, -S(O)-, -SO₂-, -SO₃-, -N=, -N=N-, -NH-, -NR'-, -PR'- et -P(O)R'-, -P(O)R'O-,-OP(O)R'O-, -PR'₂=N-, -C(O)NH-, -C(O)NR'-, -SO₂NH- ou -SO₂NR'-, où R' désigne alkyle non fluoré, partiellement fluoré ou perfluoré ayant 1-6 atomes de C, cycloalkyle saturé ou partiellement insaturé ayant 3-7 atomes de C, phényle non substitué ou substitué ou hétérocycle non substitué ou substitué
et les variables
n désigne 1 à 20,
m désigne 0, 1, 2 ou 3,
y désigne 1, 2, 3 ou 4,
z désigne 0, 1, 2 ou 3,
à condition que
pour R et R⁰ = des groupements alkyle ayant de 1 à 20 atomes de C, les anions A⁻ = [BF₄]⁻, CF₃COO⁻ ou [B(C₆H₅)₄]⁻ doivent être permis
et les composés
[n-butyl-SC(NH₂)₂]⁺ éthylOSO₃⁻, 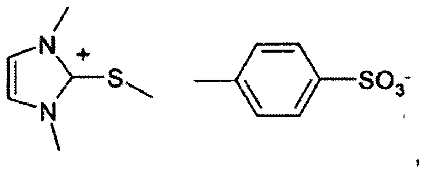 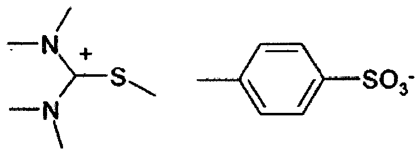 et
le p-toluènesulfonate de 2-((1S,2S,5R)-2-isopropyl-5-méthylcyclo-hexyl)thiouronium
et
[[(CH₃)₂N]₂CSCH₃]⁺CH₃SO₄⁻
sont exclus.

7. Sel selon la revendication 5 ou 6, **caractérisé en ce que** R est hydrogène et/ou alkyle à chaîne linéaire ou ramifiée ayant de 1 à 12 atomes deC.

8. Sel selon la revendication 5 ou 6, **caractérisé en ce que** R est choisi dans le groupe constitué par méthyle, éthyle, n-propyle, isopropyle, n-butyle, tertio-butyle, sec-butyle, phényle et cyclohexyle.

9. Sel selon l'une des revendications 6 à 8, **caractérisé en ce que** R⁰ désigne alkyle à chaîne linéaire ou ramifiée ayant de 1 à 12 atomes de C, pouvant être partiellement ou totalement substitué par F, mais où la fluoration du groupement α-CH₂ de R⁰ est exclue, et X = S.

10. Sel selon l'une des revendications 5 ou 7 à 8, **caractérisé en ce que** R⁰ désigne alkyle à chaîne linéaire ou ramifiée ayant de 1 à 12 atomes de C et X = O.

11. Sel selon l'une des revendications 5 ou 6, **caractérisé en ce que** l'anion est choisi dans le groupe constitué par [CF₃CF₂SO₃]⁻, [CH₃CH₂SO₃]⁻, [(CF₃SO₂)₂N]⁻, [(C₂F₅SO₂)₂N]⁻, [(CF₃SO₂)₃C]⁻, [(C₂F₅SO₂)₃C]⁻, [CH₃CH₂OSO₃]⁻, [(FSO₂)₃C]⁻, [CF₃C(O)O]⁻, [CF₃CF₂C(O)O]⁻, [CH₃CH₂C(O)O]⁻, [CH₃C(O)O]⁻, [P(C₂F₅)₃F₃]⁻, [P(CF₃)₃F₃]⁻, [P(C₂F₄H)(CF₃)₂F₃]⁻, [P(C₂F₃H₂)₃F₃]⁻, [P(C₂F₅)(CF₃)₂F₃]⁻ [P(C₆F₅)₃F₃]⁻ , [P(C₃F₇)₃F₃]⁻, [P(C₄F₉)₃F₃]⁻, [P(C₂F₅)₂F₄]⁻ [(C₂F₅)₂P(O)O]⁻, [(C₂F₅)P(O)O₂]²⁻, [P(C₆F₅)₂F₄]⁻, [(CF₃)₂P(O)O]⁻, [(CH₃)₂P(O)O]⁻, [(C₄F₉)₂P(O)O]⁻, [CF₃P(O)O₂]²⁻, [CH₃P(O)O₂]²⁻, [(CH₃O)₂P(O)O]⁻, [BF₃(CF₃)]⁻, [BF₂(C₂F₅)₂]⁻, [BF₃(C₂F₅)]⁻, [BF₂(CF₃)₂]⁻, [B(C₂F₅)₄]⁻, [BF₃(CN)]⁻, [BF₂(CN)₂]⁻, [B(CN)₄]⁻, [B(CF₃)₄]⁻, [B(OCH₃)₄]⁻, [B(OCH₃)₂(OC₂H₅)]⁻, [B(O₂C₂H₄)₂]⁻, [B(O₂C₂H₂)₂]⁻, [B(O₂CH₄)₂]⁻, [N(CF₃)₂]⁻, [N(CN₂)₂]⁻, [C(CN)₃]⁻, [AlCl₄]⁻ ou [SiF₆]²⁻,
à condition que
pour X = O, les anions CF₃COO⁻ ou [B(C₆H₅)₄]⁻ doivent être permis, pour X = O et R⁰ = éthyle, l'anion A⁻ = CH₃CH₂OSO₃⁻ doit être exclu et pour X = S et R et R⁰ = des groupements alkyle ayant de 1 à 20 atomes de C, les anions A⁻ = [BF₄]⁻, CF₃COO⁻ ou [B(C₆H₅)₄]⁻ doivent être permis
et les composés
[n-butyl-SC(NH₂)₂]⁺ éthylOSO₃⁻, 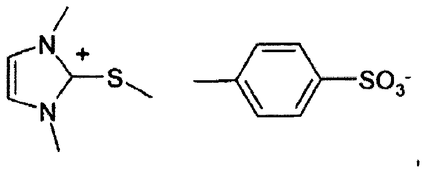 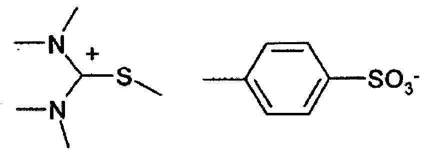 et
le p-toluènesulfonate de 2-((1S,2S,5R)-2-isopropyl-5-méthylcyclo-hexyl)thiouronium sont exclus.

12. Procédé de préparation d'un sel selon l'une des revendications 5 à 11, **caractérisé en ce qu'**une thiourée C(S)(NR₂)₂ ou une urée C(O)(NR₂)₂, où les radicaux R sont tels que définis selon l'une des revendications 5 à 11, est alkylée en utilisant un ester AR⁰, où les radicaux R⁰ sont tels que définis selon l'une des revendications 5 à 11 et A est choisi dans le groupe constitué par [R¹CH₂OSO₃], [R¹SO₃], [R^{F}SO₃], [R^{F}C(O)O] et [CCl₃C(O)O], ou en utilisant un sel d'oxonium (R⁰)₃O⁺ A⁻, où les radicaux R¹ ou R^{F} sont tels que définis selon l'une des revendications 5 à 11 et A⁻ est choisi dans le groupe constitué par [(FSO₂)₃C]⁻ et [BF₄]⁻, en respectant la condition que
pour X = O, les anions CF₃COO⁻ ou [B(C₆H₅)₄]⁻ doivent être permis,
pour X = O et R⁰ = éthyle, l'anion A⁻ = CH₃CH₂OSO₃⁻ doit être exclu et
pour X = S et R et R⁰ = des groupements alkyle ayant de 1 à 20 atomes de C, les anions A⁻ = [BF₄]⁻, CF₃COO⁻ ou [B(C₆H₅)₄]⁻ doivent être permis
et les composés
[n-butyl-SC(NH₂)₂]⁺ éthylOSO₃⁻, 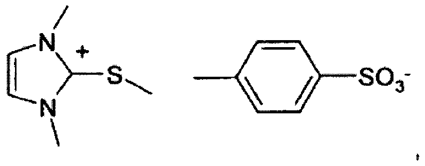 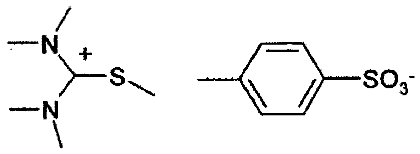 et
le p-toluènesulfonate de 2-((1S,2S,5R)-2-isopropyl-5-méthylcyclohexyl)thiouronium,
le méthylsulfate de N,N,N,N-tétraméthyldiaminométhoxycarbonium, [[(CH₃)₂N]₂CSCH₃]⁺CH₃SO₄⁻
sont exclus.

13. Procédé selon la revendication 12, **caractérisé en ce que** la réaction est effectuée à une température à laquelle au moins l'un des composants est liquide.

14. Procédé de préparation d'un sel de formule (1) selon l'une des revendications 5 à 11, **caractérisé en ce qu'**un sel ayant un cation de formule générale (1) selon l'une des revendications 5 à 11 et un anion A⁻ choisi dans le groupe constitué par [BF₄]⁻, Br⁻, Cl⁻, I⁻ ou [ClO₄]⁻ est réagi avec un sel Kt⁺ A⁻ ou avec un acide AH, où Kt est un métal alcalin ou un métal alcalino-terreux et A est tel que défini selon l'une des revendications 5 à 11.
